# EUROPÄISCHE PATENTANMELDUNG

(11) **EP 4 614 510 A1**
(43) Veröffentlichungstag der Anmeldung: **10.09.2025**
(21) Anmeldenummer: 24162381.8
(22) Anmeldetag: 08.03.2024
(51) Int. Cl.: G16H 30/20, G16H 40/63, G01R 33/54

(54) **COMPUTERIMPLEMENTIERTES VERFAHREN ZU EINEM UNTERSTÜTZEN UND/ODER ASSISTIEREN EINES BENUTZERS MITTELS EINES ASSISTENZSYSTEMS BEI EINEM AUSFÜHREN EINES MESSPROGRAMMS WÄHREND EINER MAGNETRESONANZDATENERFASSUNG**

(71) Anmelder: Siemens Healthineers AG, 91301 Forchheim (DE)
(72) Erfinder: Arroyo Camejo, Silvia Bettina, 90763 Fürth (DE); Franger, Dirk, 91052 Erlangen (DE)
(74) Vertreter: Siemens Healthineers Patent Attorneys

(57) **Zusammenfassung**

Die Erfindung geht aus von einem computerimplementierten Verfahren zu einem Unterstützen und/oder Assistieren eines Benutzers mittels eines Assistenzsystems bei einem Ausführen eines Messprogramms während einer Magnetresonanzdatenerfassung, umfassend die Schritte:
- Auswählen eines Messprogramms zu einem Erfassen von Magnetresonanzdaten,
- Ausführen des ausgewählten Messprogramms und Bereitstellen der Magnetresonanzdaten und/oder von aus den Magnetresonanzdaten rekonstruierten Bilddaten,
- Erfassen weiterer Messinformationen, wobei die weiteren Messinformationen vor einem Ausführen des ausgewählten Messprogramms oder während eines Ausführens des ausgewählten Messprogramms erfasst werden, und Bereitstellen der weiteren Messinformationen,
- Ermitteln zumindest einer Bewertungsinformation mittels zumindest eines Analysemoduls des Assistenzsystems in Abhängigkeit von den Magnetresonanzdaten und/oder Bilddaten und/oder den weiteren Messinformationen, und Bereitstellen der zumindest einen Bewertungsinformation,
- Generieren einer Assistenzinformation, wobei die Assistenzinformation in Abhängigkeit von der zumindest einen Bewertungsinformation mittels des zumindest einen Analysemoduls generiert wird, und Bereitstellen der Assistenzinformation und
- Ausgabe der Assistenzinformation an einen Benutzer.

## Beschreibung

Die vorliegende Erfindung betrifft ein Computerimplementiertes Verfahren zu einem Unterstützen und/oder Assistieren eines Benutzers mittels eines Assistenzsystems bei einem Ausführen eines Messprogramms während einer Magnetresonanzdatenerfassung. Des Weiteren betrifft die vorliegende Erfindung auch ein Assistenzsystem, das zu einer Durchführung des Verfahrens zu einem Unterstützen und/oder Assistieren eines Benutzers bei einem Ausführen eines Messprogramms während einer Magnetresonanzdatenerfassung ausgebildet ist. Weiterhin geht die Erfindung aus von einer Magnetresonanzvorrichtung mit einem Assistenzsystem. Des Weiteren betrifft die vorliegende Erfindung auch ein Computerprogrammprodukt, welches ein Programm umfasst und direkt in einen Speicher einer programmierbaren Steuerungseinheit ladbar ist, mit Programmmitteln, um ein Verfahren zu einem Unterstützen und/oder Assistieren eines Benutzers mittels eines Assistenzsystems bei einem Ausführen eines Messprogramms zur einer Magnetresonanzdatenerfassung auszuführen, wenn das Programm in der Steuerungseinheit ausgeführt wird.

Unabhängig vom grammatikalischen Geschlecht eines bestimmten Begriffes sind Personen mit männlicher, weiblicher oder anderer Geschlechteridentität mit umfasst.

Diagnostische Bildgebungsvorrichtungen, wie z. B. Magnetresonanzvorrichtungen, liefern umfangreiche strukturelle und funktionelle Informationen über einen Patienten, damit ein Arzt eine Diagnose stellen kann. Diese Informationen liegen in Form von Bilddaten, insbesondere Magnetresonanzbilddaten, vor. Die Genauigkeit einer medizinischen Diagnose hängt stark von der Qualität der aufgenommenen medizinischen Bilddaten ab. Dabei hängt die Qualität der medizinischen Bilddaten von verschiedenen Faktoren ab. Ein Faktor kann eine Hardware-Konfiguration der Magnetresonanzvorrichtung sein. Dabei können eine B0-Magnetfeld-Inhomogenität und/oder eine Gradientenfeldstärke und/oder auch eine Qualität und Robustheit von Zubehör wie lokalen Spulen einen starken Einfluss sowohl auf die durchschnittliche Bildqualität als auch auf die Variabilität der Bildqualität der erfasstem Magnetresonanzbilddaten haben.

Ein weiterer Faktor für die Beeinflussung der Bildqualität ist der Patient. Beispielsweise können Bewegungen des Patienten erheblich die Bildqualität beeinflussen und beispielsweise zu Bildartefakten führen. Auch bei der Bildgebung des Abdomens, bei der der Patient definierte Atembewegungen ausführen soll, kann ein Fehlverhalten, insbesondere ein falsch ausgeführte Atmung, zu einer Beeinträchtigung der Bildqualität führen. Ein weiteres Beispiel ist die Bildgebung des Herzens, bei der ein EKG-Signal die Bildgebung triggert und/oder auslöst. Bei Patienten mit einer kardialen Arrhythmie kann diese jedoch zu einer fehlerhaften Auslösen der Erfassung der Bilddaten führen.

Ein zusätzlicher Aspekt für die Beeinflussung der Bildqualität ist das medizinische Bedienpersonal, dass die Magnetresonanzuntersuchung an einem Patienten durchführt und/oder betreut. Für eine optimale Bedienung einer Magnetresonanzvorrichtung, insbesondere für eine optimale Einstellung und/oder Anpassung von Betriebsparametern und/oder Messparametern, ist eine langjährige Erfahrung des medizinischen Bedienpersonals erforderlich. Weniger erfahrenes medizinisches Bedienpersonal dagegen nutzt häufig die Standardeinstellungen, ohne dabei das Optimum der Magnetresonanzvorrichtung auszunutzen.

Zur Unterstützung des medizinischen Bedienpersonals sind Softwarekomponenten bekannt, die in der Lage sind, hardwarebedingte Mängel oder Artefakte in den Bilddaten teilweise zu kompensieren. Dazu zählen beispielsweise Verzerrungskorrekturalgorithmen in der Bildrekonstruktion, die Bildartefakte kompensieren, die durch Inhomogenität des B0-Magnetfeldes entstehen. Zudem sind auch Messprogramme und/oder Messsequenzen bekannt, die besonders robust gegenüber Bewegungen von Patienten während der Magnetresonanzdatenerfassung sind oder eine freie Atmung während der Magnetresonanzdatenerfassung von Untersuchungen des Abdomens und/oder des Herzens ermöglichen. Jedoch ist eine auf den Patienten optimierte Anpassung von Messsequenzen nicht verfügbar.

Der vorliegenden Erfindung liegt insbesondere die Aufgabe zugrunde, einfache und automatisierte Unterstützung eines Benutzers während einer Ausführung eines Messprogramms bereitzustellen. Die Aufgabe wird durch die Merkmale der unabhängigen Ansprüche gelöst. Vorteilhafte Ausgestaltungen sind in den Unteransprüchen beschrieben.

Die Erfindung geht aus von einem computerimplementierten Verfahren zu einem Unterstützen und/oder Assistieren eines Benutzers mittels eines Assistenzsystems bei einem Ausführen eines Messprogramm während einer Magnetresonanzdatenerfassung, umfassend die folgenden Verfahrensschritte:
- Auswählen eines Messprogramms zu einem Erfassen von Magnetresonanzdaten mit einer definierten diagnostischen Fragestellung,
- Ausführen des ausgewählten Messprogramms, wobei das Ausführen des ausgewählten Messprogramms ein Erfassen von Magnetresonanzdaten und/oder ein rekonstruieren von Bilddaten aus den erfassten Magnetresonanzdaten umfasst, und Bereitstellen der Magnetresonanzdaten und/oder den rekonstruierten Bilddaten,
- Erfassen von weiteren Messinformationen, wobei die weiteren Messinformationen vor einem Ausführen des ausgewählten Messprogramms oder während eines Ausführens des ausgewählten Messprogramm erfasst werden, und Bereitstellen der weiteren Messinformationen,
- Ermitteln zumindest einer Bewertungsinformation mittels zumindest eine Analysemoduls des Assistenzsystems in Abhängigkeit von den bereitgestellten Magnetresonanzdaten und/oder den bereitgestellten rekonstruierten Bilddaten und/oder den bereitgestellten weiteren Messinformationen, und Bereitstellen der zumindest einen Bewertungsinformation,
- Generieren einer Assistenzinformation, wobei die Assistenzinformation in Abhängigkeit von der zumindest einen Bewertungsinformation mittels des zumindest einen Analysemoduls generiert wird, und Bereitstellen der Assistenzinformation und
- Ausgabe der Assistenzinformation über eine Benutzerschnittstelle an einen Benutzer.

Für die Magnetresonanzdatenerfassung wird eine Magnetresonanzuntersuchung an einem Patienten, insbesondere an einem zu untersuchenden Bereich eines Patienten, mittels einer Magnetresonanzvorrichtung durchgeführt. Für eine Magnetresonanzuntersuchung, bei der eine definierte klinische und/oder diagnostische Fragestellung geklärt werden soll, wird zunächst anhand der definierten klinischen und/oder diagnostischen Fragestellung ein Messprogramm ausgewählt. Die definierte klinische und/oder diagnostische Fragestellung kann beispielsweise von einer zu untersuchenden Körperregion des Patienten und/oder von einer Erkrankung des Patienten abhängen.

Ein derartiges Messprogramm kann dabei mehrere Messschritte umfassen. Die einzelnen Messschritte können dabei jeweils zumindest eine Magnetresonanzsequenz umfassen. Bei mehreren Magnetresonanzsequenzen werden diese bevorzugt in einer definierten und/oder bestimmten Folge nacheinander ausgeführt und/oder ausgespielt. Die einzelnen Magnetresonanzsequenzen umfassen bevorzugt eine zeitliche Folge von Hochfrequenzpulsen. Beispielsweise kann eine Magnetresonanzsequenz eine T1-gewichtete Sequenz oder eine T2-gewichtete Sequenz oder eine Spin-Echo-Sequenz usw. umfassen. Die einzelnen Magnetresonanzsequenzen unterscheiden sich dabei hinsichtlich ihrer Sequenzparameter.

Die Auswahl eines Messprogramms erfolgt dabei durch einen Benutzer. Dabei kann der Benutzer auch auf voreingestellte Messprogramme zugreifen, die den Messablauf der einzelnen Magnetresonanzsequenzen für eine klinische oder diagnostische Fragestellung festlegen. Zudem kann auch ein erfahrener Benutzer ein Messprogramm aus einzelnen Magnetresonanzsequenzen selbst zusammenstellen und/oder auswählen.

Während eines Ausführens des Messprogramms werden die einzelnen Messschritte, insbesondere die einzelnen Messsequenzen, ausgeführt und dabei Magnetresonanzdaten erfasst. Hierzu befindet sich der Patient, insbesondere der zu untersuchende Bereich des Patienten, innerhalb eines Patientenaufnahmebereichs einer Magnetresonanzvorrichtung. Insbesondere befindet sich hierbei der zu untersuchende Bereich des Patienten in einem Isozentrum der Magnetresonanzvorrichtung.

Während des Ausführens des Messprogramms werden zudem aus den erfassten Magnetresonanzdaten Bilddaten rekonstruiert. Dabei kann eine Rekonstruktion der Bilddaten mittels des Messprogramms erfolgen oder auch mittels einer Rekonstruktionseinheit. Die bereitgestellten Magnetresonanzdaten umfassen bevorzugt Magnetresonanz-Rohdaten und/oder K-Raum-Daten.

Neben den Magnetresonanzdaten können zudem weitere Messinformationen erfasst werden. Diese weiteren Messinformationen können dabei Patientendaten umfassen, die bereits vor der Magnetresonanzuntersuchung bekannt sind und/oder erfasst wurden. Beispielsweise können diese Patientendaten bereits bei einer Registrierung des Patienten erfasst worden sein, wie beispielsweise ein Patientengewicht und/oder eine Größe des Patienten und/oder ein Alter des Patienten. Zudem können derartige Patientendaten auch aus Voruntersuchungen des Patienten bekannt sein, wie beispielsweise eine kardiale Arrhythmie eines Patienten. Zudem können auch die weiteren Messinformationen erst während des Ausführens des Messprogramms erfasst werden, wie beispielsweise physiologische Daten des Patienten, die während des Ausführens des Messprogramms laufend erfasst werden. Derartige physiologische Daten können beispielsweise EKG-Daten des Patienten oder auch Respirations-Daten umfassen. Zudem können die weiteren Messinformationen, die fortlaufend während des Ausführens des Messprogramms erfasst werden, auch Informationen hinsichtlich einer Bewegung des Patienten umfassen.

Das Assistenzsystem umfasst zumindest ein Analysemodul. Bevorzugt umfasst das Assistenzsystem mehrere unterschiedliche Analysemodule. Die einzelnen Analysemodule bewerten und/oder analysieren jeweils für eine definierte Fragestellung die bereitgestellten Magnetresonanzdaten und/oder die bereitgestellten Bilddaten und/oder die bereitgestellten weiteren Messinformationen. Die definierte Fragestellung kann dabei eine Fragestellung hinsichtlich einer Qualität, insbesondere Bildqualität, in den bereitgestellten Magnetresonanzdaten und/oder Bilddaten umfassen. Zudem kann die definierte Fragestellung auch eine klinische und/oder diagnostische Fragestellung umfassen. Zudem kann die definierte Fragestellung auch eine technische Fragestellung umfassen. Zudem kann die definierte Fragestellung auch eine allgemeine untersuchungsrelevante Fragestellung umfassen.

Zur Beantwortung der jeweiligen definierten Fragestellung ermitteln die einzelnen Analysemodule eine Bewertungsinformation in Abhängigkeit von den bereitgestellten Magnetresonanzdaten und/oder den bereitgestellten Bilddaten und/oder den bereitgestellten weiteren Messinformationen. Zur Ermittlung der Bewertungsinformation weisen die einzelnen Analysemodule einen Bewertungsalgorithmus auf. Die Bewertungsinformation kann beispielsweise eine Kategorisierung und/oder Klassifizierung einer ermittelten Antwort in "Gut" oder "Schlecht" umfassen. Zudem kann die Bewertungsinformation auch eine Multi-Class-Klassifizierung umfassen, bei der die Bewertungsinformation im Format (1,0,0,...,0), (0,1,0,...,0), (0,0,1,...,0), ... (0,0,0,...,1) vorliegt. Zudem kann die Bewertungsinformation auch eine Multi-Class-Multi-Label-Klassifizierung umfassen, bei der die Bewertungsinformation im Format (1, 0, 0,...,0), (1,1,0,...,0) (1,1,1,...,0), ... (1,1,1,...,1) vorliegt. In allen Fällen kann die Bewertungsinformation zudem den Wert "-1" annehmen, wenn beispielsweise keine Analyse und/oder Ermittlung einer Bewertungsinformation möglich war und/oder eine Analyse mittels des zumindest einen Analysemoduls abgebrochen wurde. Zudem können die Bewertungsinformationen weitere, dem Fachmann als sinnvoll erscheinende Kategorisierung und/oder Klassifizierung umfassen.

Neben den Analysemodulen kann das Assistenzsystem weitere Module und Einheiten aufweisen. Bevorzugt weist das Assistenzsystem einen Transaktionsmanager auf, wobei der Transaktionsmanager dazu ausgebildet ist, die bereitgestellten Magnetresonanzdaten und/oder die bereitgestellten Bilddaten und die bereitgestellten weiteren Messinformationen den zumindest einem Analysemodul oder den mehreren Analysemodulen bereitzustellen. Zudem kann der Transaktionsmanager auch einen Datenaustausch zwischen einzelnen Analysemodulen oder zwischen Analysemodulen und weiteren Modulen regeln und/oder steuern. Beispielsweise kann der Transaktionsmanager auch die Bewertungsinformation von Analysemodulen für weitere Module bereitstellen. Hierzu können die einzelnen Analysemodule und/oder die weiteren Module jeweils eine entsprechende Schnittstelle aufweisen, die mit dem Transaktionsmanager kommuniziert. Insbesondere kann der Transaktionsmanager auch eine Ausführung der einzelnen Analysemodule und/oder eine Reihenfolge einer Ausführung von einzelnen Analysemodulen steuern.

Zudem kann das Assistenzsystem auch ein Konfigurations-Userinterface umfassen, das bevorzugt dazu ausgebildet sein kann, einem Benutzer bei der Konfiguration und/oder Auswahl des Messprogramms eine Auswahl an Analysemodulen bereitzustellen, wobei aus dieser Auswahl der Benutzer ein Analysemodul oder auch mehrere Analysemodule für das Bereitstellen einer Assistenzinformation auswählen kann. Bevorzugt kann dabei der Benutzer mittels des Konfigurations-Userinterfaces eine Unterstützung, insbesondere eine Auswahl zumindest eines Analysemoduls und/oder zumindest eines weiteren Moduls, an einer bestimmten und/oder definierten Position im Messprogramm konfigurieren. Dabei kann der Benutzer bei manchen Analysemodulen auch die Möglichkeit haben, mittels des Konfigurations-Userinterfaces einen Datentyp und/oder ein Datenformat von Eingangsdaten für das ausgewählte Modul auszuwählen. Eine weitere Möglichkeit mittels des Konfigurations-Userinterfaces ist, dass der Benutzer auch einzelne Analysemodule konfigurieren, insbesondere an zumindest einen Messschritt anpassen, kann. Auch kann der Benutzer dabei eine Verknüpfung von mehreren Analysemodulen konfigurieren und/oder auswählen.

Des Weiteren kann das Konfigurations-Userinterface auch dazu ausgebildet sein, ein Datenformat von Eingangsdaten und/oder Ausgangsdaten von Analysemodulen festzulegen. Zudem kann das Konfigurations-Userinterface auch dazu ausgebildet sein, eine Auswahl von Analysemodulen automatisch festzulegen, wobei der Benutzer aus dieser Auswahl zumindest ein Analysemodul für eine Unterstützung des Messprogramms auswählen kann.

Anhand der Bewertungsinformation wird zudem eine Assistenzinformation generiert. Das Generieren der Assistenzinformation erfolgt dabei durch das Analysemodul anhand der zumindest einen Bewertungsinformation. Die Assistenzinformation ist für eine Ausgabe an einen Benutzer bestimmt. Die Assistenzinformation kann dabei dem Benutzer einen Hinweis geben und/oder darüber informieren, ob während der Magnetresonanzuntersuchung, insbesondere des Ausführens des Messprogramms, Probleme und/oder kritische Situationen und/oder Auffälligkeiten aufgetreten sind, die eine Magnetresonanzdatenerfassung und/oder das Ausführen des Messprogramms beeinträchtigen. Zudem kann die Assistenzinformation auch den Benutzer darüber informieren, dass kritische Situationen und/oder Auffälligkeiten erkannt wurden und diese zu einem möglichen Problem bei der Ausführung des Messprogramms und/oder der Magnetresonanzdatenerfassung führen kann. Zudem kann die Assistenzinformation auch dazu ausgebildet sein, eine Art der Probleme und/oder der kritische Situationen und/oder der Auffälligkeiten zu benennen. Beispielsweise kann die Assistenzinformation den Benutzer auf eine mangelnde Bildqualität in den erfassten Magnetresonanzdaten und/oder den bereitgestellten Bilddaten hinweisen und zudem auch den Benutzer über eine mögliche, jedoch unerwünschte Bewegung des Patienten während des Ausführens des Messprogramms hinweisen, wobei die Bewegung des Patienten die Ursache für unzureichende Bildqualität sein kann.

Bevorzugt ist das zumindest eine Analysemodul dazu ausgebildet, die Analyse der bereitgestellten Magnetresonanzdaten und/oder der bereitgestellten rekonstruierten Bilddaten und/oder der bereitgestellten weiteren Messinformationen fortlaufend während der gesamten Dauer des Ausführens zumindest einen Messschritts auszuführen, so dass bei einem Auftreten eines spontanen Problems und/oder einer kritischen Situation der Benutzer unmittelbar darüber informiert werden kann.

Die Ausgabe der Assistenzinformation erfolgt bevorzugt über eine Benutzerschnittstelle der Magnetresonanzvorrichtung. Vorzugsweise erfolgt hierbei die Ausgabe der Assistenzinformation über eine visuelle Darstellungseinheit, beispielsweise einen Monitor und/oder eine Touch-Display. Der Benutzer ist bevorzugt ein medizinisches Bedienpersonal zur Betreuung der Magnetresonanzuntersuchung. Das medizinische Bedienpersonal kann beispielsweise ein Arzt, insbesondere ein Radiologe, oder ein medizinischer Technologe für Radiologie sein.

Die Erfindung weist den Vorteil auf, dass ein Benutzer während einer Magnetresonanzuntersuchung, insbesondere eines Ausführens des Messprogramms, direkt auf Probleme und/oder kritische Situationen und/oder Auffälligkeiten bei der Magnetresonanzdatenerfassung hingewiesen werden kann. Damit erhält der Benutzer direkt ein Feedback und kann aufgrund der Information, insbesondere der Assistenzinformation, entscheiden, ob er Maßnahmen zur Behebung der Fehler oder Probleme ausführen möchte, beispielsweise einzelne Messschritte des Messprogramms wiederholen oder Aufnahmeparameter ändern möchte. Beispielsweise kann der Benutzer auch eine Atemanhaltedauer für den Patienten anpassen, wenn dieser Probleme hat, das Atmen lange anzuhalten. Des Weiteren kann der Benutzer auch eine alternative Messsequenz für den Messschritt auswählen. Zudem kann der Benutzer auch entscheiden, dass zwar eine Qualität der Bilddaten vorliegt, die mängelbehaftet ist, für eine Beurteilung der klinischen oder diagnostischen Fragestellung jedoch ausreichend ist. Zudem kann der Benutzer aufgrund der Information, insbesondere der Assistenzinformation, entscheiden, ob er Maßnahmen zur genaueren Analyse von in den Bilddaten erkannten Auffälligkeiten ausführen möchte, beispielsweise zusätzliche Messschritte, deren Fokus auf einen Bildbereich mit der Auffälligkeit gerichtet ist.

Durch das direkte Informieren des Benutzers, insbesondere durch die Ausgabe der Assistenzinformation während des Ablaufs des Messprogramms, kann zudem ein Aufwand, insbesondere ein Untersuchungsaufwand, für den Patienten als auch Benutzer reduziert werden. Sind beispielsweise die erfassten Magnetresonanzdaten und/oder die aus den Magnetresonanzdaten rekonstruierten Bilddaten für eine diagnostische Bewertung ungeeignet, kann unmittelbar der Messschritt oder mehrere Messschritte wiederholt und/oder mit Änderungen ausgeführt werden. Eine Wiederholung erst nach Tagen oder Wochen, wenn die Magnetresonanzdaten und/oder Bilddaten analysiert sind und eine Wiederholungstermin für den Patienten gefunden wurde, kann dabei verhindert werden. Insbesondere müssen beispielsweise nur die relevanten, insbesondere die mangelbehafteten und/oder fehlerbehaften, Messschritte wiederholt werden und nicht das ganze Messprogramm.

Zudem kann es auch sein, sofern das Assistenzsystem neben dem zumindest einen Analysemodul auch zumindest ein Empfehlungsmodul umfasst, dass das Empfehlungsmodul anhand der bereitgestellten Bewertungsinformation und/oder der bereitgestellten Assistenzinformation eine Empfehlung und/oder einen Vorschlag ermittelt und für den Benutzer bereitstellt. Diese Empfehlung und/oder der Vorschlag kann beispielsweise einen weiteres Vorgehen für das Messprogramm umfassen. Dies ermöglicht es auch ungeübten Benutzern bei Auftreten von Auffälligkeiten in den erfassten Magnetresonanzdaten und/oder den weiteren Messinformationen das Messprogramm erfolgreich zu beenden und für eine anschließende Diagnose bereitzustellen.

Alternativ oder zusätzlich kann es vorgesehen sein, dass die zumindest eine Assistenzinformation über eine Benutzeroberfläche zur Steuerung und/oder Überwachung des Messprogramms an einen Benutzer ausgegeben wird. Die Benutzeroberfläche zur Steuerung und/oder Überwachung des Messprogramms zeigt dabei bevorzugt die einzelnen Messschritte des Messprogramms an. Dabei können für die einzelnen Messschritte auch die für den Messschritt aktivierten und/oder ausgewählten Analysemodule angezeigt werden. Bevorzugt wird die Assistenzinformation für den jeweiligen Messschritt in der Benutzeroberfläche angezeigt. Insbesondere erfolgt hierbei die Anzeige und/oder Ausgabe der Assistenzinformation an einer definierten Position an der Benutzeroberfläche. Derart kann eine einfache und schnelle Übermittlung der Assistenzinformation an den Benutzer erfolgen. Insbesondere kann sich hierbei der Benutzer auf eine einzige Benutzeroberfläche konzentrieren und erhält alle für das Messprogramm zur Verfügung stehenden Informationen inklusive der Assistenzinformationen.

Alternativ oder zusätzlich kann es vorgesehen sein, dass das Assistenzsystem auch eine eigene Benutzeroberfläche zur Ausgabe von Informationen, insbesondere der Assistenzinformation, umfasst. Eine derartige Benutzeroberfläche kann beispielsweise ein Assistenz-Userinterface umfassen, das neben der Assistenzinformation den Benutzer auch darüber informieren kann, welches Analysemodul gerade ausgeführt wird und für welchen Messschritt dieses ausgewählt wurde.

Alternativ oder zusätzlich kann es vorgesehen sein, dass die zumindest eine weitere Bewertungsinformation durch ein weiteres Analysemodul in Abhängigkeit von der bereitgestellten Bewertungsinformation des zumindest einen Analysemoduls ermittelt wird. Beispielsweise kann dabei die bereitgestellte Bewertungsinformation des ersten Analysemoduls die Eingangsdaten für das weitere Analysemodul umfassen. Zudem können die Eingangsdaten für das weitere Analysemodul sowohl die bereitgestellte Bewertungsinformation als auch die bereitgestellten Magnetresonanzdaten und/oder die bereitgestellten rekonstruierten Bilddaten und/oder die bereitgestellten weiteren Messinformationen umfassen. Insbesondere baut hierbei das weitere Analysemodul auf das erste Analysemodul zur Klärung einer klinischen Fragestellung und/oder einer technischen Fragestellung und/oder einer patientenrelevanten Fragestellung auf. Die Assistenzinformation wird dabei bevorzugt von dem weiteren Analysemodul generiert. Derart kann eine vorteilhafte Unterstützung eines Benutzers auch bei komplexen Fragestellungen und/oder komplexen Situationen, insbesondere komplexen Messsituationen, bereitgestellt werden.

Alternativ oder zusätzlich kann es vorgesehen sein, dass zur Generierung der Assistenzinformation zumindest zwei Bewertungsinformationen von zumindest zwei Analysemodulen eingehen. Bevorzugt ermittelt dabei jedes der zumindest zwei Analysemodule unabhängig von den weiteren Analysemodulen eine Bewertungsinformation. Anschließend wird aus allen zur Verfügung stehenden Bewertungsinformationen die Assistenzinformation generiert. Das Generieren der Assistenzinformation kann dabei beispielsweise mittels einer künstlichen Intelligenz (KI) erfolgen. Dabei kann es sein, dass zur Generierung der Assistenzinformation die KI die einzelnen Bewertungsinformationen unterschiedliche gewichtet werden. Eine Gewichtung der einzelnen Bewertungsinformationen kann dabei abhängig sein von einer Art des Messprogramms, insbesondere der einzelnen Messschritte, und/oder von einer Art des Analysemoduls.

In einer vorteilhaften Weiterbildung des erfindungsgemäßen Verfahrens kann es vorgesehen sein, dass die Assistenzinformation eine Statusinformation des Analysemoduls umfasst. Die Statusinformation des Analysemoduls kann dabei dem Benutzer anzeigen, welcher Art das Analysemodul ist, beispielsweise ob ein Qualitäts-Analysemodul und/oder ein technisches Analysemodul und/oder ein klinisches Analysemodul für die Beurteilung und/oder Analyse des Messschritts ausgewählt wurde. Zudem kann die Statusinformation dem Benutzer anzeigen, ob aktuell eine Analyse mittels des Analysemoduls durchgeführt wird. Zudem kann die Statusinformation dem Benutzer anzeigen, ob die Analyse mittels des Analysemoduls erfolgreich beendet wurde und ein Ergebnis vorliegt. Zudem kann die Statusinformation dem Benutzer auch anzeigen, ob die Analyse mittels des Analysemoduls nicht durchgeführt werden konnte. Bevorzugt wird die Statusinformation des Analysemoduls durch entsprechende Symbole angezeigt, die vorteilhafterweise an einer definierten und/oder festgelegten Position auf der Benutzeroberfläche angeordnet sind. Hierdurch kann der Benutzer einen schnellen Überblick über das Analysemodul erhalten.

Alternativ oder zusätzlich kann es vorgesehen sein, dass die Assistenzinformation eine Probleminformation und/oder eine Auffälligkeitsinformation umfasst. Die Probleminformation umfasst eine Information, die den Benutzer auf ein Problem und/oder einen kritischen Zustand und/oder auf eine Auffälligkeit während des Ausführens des Messprogramms hinweist. Beispielsweise kann ein von dem Analysemodul erkanntes Problem und/oder ein von dem Analysemodul erkannter kritischer Zustand eine mangelhafte Bildqualität und/oder eine Bewegung des Patienten umfassen. Zudem kann ein von dem Analysemodul erkanntes Problem und/oder ein von dem Analysemodul erkannter kritischer Zustand auch eine falsche Atmung des Patienten bei beispielsweise einer Herzbildgebung sein. Zudem kann die Probleminformation auch eine Information umfassen, dass kein Problem und/oder kein kritischer Zustand während des Ausführens des Messprogramms aufgetreten ist und/oder von dem Analysemodul erkannt wurde. Die Auffälligkeitsinformation umfasst bevorzugt eine Information, die eine Auffälligkeit des Patienten beschreibt, also insbesondere einen von einer Norm abweichenden Zustand. Derartige Auffälligkeitsinformationen stellen nicht zwangsläufig ein Qualitätsproblem in den erfassten Daten dar. Vielmehr kann die Auffälligkeitsinformation auch eine neutrale Information umfassen, die auf beispielsweise eine Anomalie und eine Abnormität während der Ausführung zumindest einen Messschritts des ausgewählten Messprogramms hinweist. Zudem kann die Auffälligkeitsinformation auch eine Information umfassen, dass keine Auffälligkeit in den analysierten Daten erkannt wurde. Bei einem Vorhandensein einer Auffälligkeit kann auch eine Anpassung des weiteren Messprogramms sinnvoll sein. Beispielsweise kann bei Erkennen einer Anomalie für diese noch weitere Bilddaten in einem weiteren Messschritt bereitgestellt werden, um beispielsweise einem Radiologen die Möglichkeit zu geben, anschließend eine Diagnose erstellen zu können. Eine Anpassung des Messprogramms, insbesondere der weiteren Schritte des Messprogramms bei Vorliegen einer Auffälligkeit ist jedoch nicht in jedem Fall zwingend erforderlich. Derart können einem Benutzer umfangreiche Informationen zur aktuellen Magnetresonanzdatenerfassung bereitgestellt werden. Insbesondere kann der Benutzer aufgrund der ausgegebenen Probleminformation und/oder Auffälligkeitsinformation über mögliche Alternativen zur Verbesserung der Magnetresonanzdatenerfassung und/oder zur Behebung des aufgetretenen Problems und/oder einer vorhandenen Auffälligkeit entscheiden.

Beispielsweise kann bei einer Kopfuntersuchung die Assistenzinformation eine Information umfassen, dass eine unspezifische Masse im Gehirn des Patienten erkannt wurde. Zur weiteren Analyse oder zur Diagnose der unspezifischen Masse kann der Benutzer weitere Messschritte, die einen Fokus auf die Erfassung des Bereichs des Gehirns mit der Auffälligkeit aufweisen, in das Messprogramm einbauen. Zudem kann, sofern das Assistenzsystem ein Empfehlungsmodul umfasst, dem Benutzer ein entsprechender Vorschlag zur Anpassung des Messprogramms, insbesondere durch ein Hinzufügen weiterer Messschritte, die einen Fokus auf die Erfassung des Bereichs des Gehirns mit der Auffälligkeit aufweisen, unterbreitet werden. Ein weiteres Beispiel wäre, dass eine Analyse der Bilddaten ergibt, dass keine Auffälligkeit vorliegt. Umfassen beispielsweise einzelne Messschritte eine Kontrastmittel-Messung, um Auffälligkeiten im untersuchten Gewebe besser hervorzuheben, können derartige Messschritte vom Benutzer weggelassen werden. Zudem kann, sofern das Assistenzsystem ein Empfehlungsmodul umfasst, dem Benutzer ein entsprechender Vorschlag zur Anpassung des Messprogramms, insbesondere durch ein Entfernen von Kontrastmittel gestützten Messschritten, unterbreitet werden.

Alternativ oder zusätzlich kann es vorgesehen sein, dass die Ausgabe der Probleminformation und/oder Auffälligkeitsinformation an einer Benutzeroberfläche ein aktives Abrufen durch den Benutzer umfasst. Bevorzugt wird die Probleminformation und/oder Auffälligkeitsinformation an der Benutzerschnittstelle, insbesondere an einer Benutzeroberfläche, über die die Assistenzinformation an einen Benutzer ausgegeben wird, bereitgestellt. Beispielsweise kann das aktive Abrufen durch den Benutzer dabei aktives Anklicken eines Buttons auf der Benutzeroberfläche umfassen, wobei das aktive Anklicken ein Anzeigen der Probleminformation und/oder Auffälligkeitsinformation hervorruft und/oder auslöst. Bevorzugt weist hierzu die Benutzeroberfläche, über die die Probleminformation und/oder Auffälligkeitsinformation abrufbar ist, ein mit der Probleminformation und/oder Auffälligkeitsinformation verlinktes Symbol und/oder ein mit der Probleminformation und/oder Auffälligkeitsinformation verlinktes Objekt auf, das von einem Benutzer beispielsweise mittels einer Computermaus anklickbar und/oder auswählbar ist. Hierdurch kann dem Benutzer bei Bedarf die benötigte Information, insbesondere die Probleminformation und/oder Auffälligkeitsinformation, angezeigt werden. Sofern kein Bedarf seitens des Benutzers besteht, die Probleminformation und/oder Auffälligkeitsinformation anzusehen, kann eine übersichtliche Benutzeroberfläche ohne zu viele Zusatzinformationen bereitgestellt werden.

In einer vorteilhaften Weiterbildung des erfindungsgemäßen Verfahrens kann es vorgesehen sein, dass das zumindest eine Analysemodul zumindest ein Qualitäts-Analysemodul und/oder zumindest ein klinisches Analysemodul und/oder zumindest ein technisches Analysemodul und/oder zumindest ein allgemeines Analysemodul umfasst.

Das zumindest eine Qualitäts-Analysemodul ist das ausgebildet, bestimmte Merkmale in Eingangsdaten zu erkennen, wobei die bestimmten Merkmale sich auf bestimmte oder spezifische Datenqualitätsprobleme beziehen. Dabei ist das zumindest eine Qualitäts-Analysemodul dazu ausgebildet, einen Schweregrad des Datenqualitätsproblems zu bewerten. Liegen als Eingangsdaten des zumindest einen Qualitäts-Analysemoduls rekonstruierte Bilddaten vor, kann das zumindest eine Qualitäts-Analysemodul dazu ausgebildet sein, in den rekonstruierten Bilddaten Bewegungsartefakte und/oder Gibbs-Artefakte usw. zu erkennen. Bevorzugt ist das zumindest eine Qualitäts-Analysemodul dazu ausgebildet, die Eingangsdaten kontinuierlich hinsichtlich des bestimmten oder spezifischen Datenqualitätsproblems zu überprüfen und zu analysieren. Ein Vorhandensein eines Datenqualitätsproblems kann beispielsweise eine Bildqualität in den rekonstruierten Bilddaten derart beeinträchtigen, dass eine diagnostische Auswertung der Bilddaten nicht mehr möglich ist oder auch die Gefahr eine Fehldiagnose aufgrund der Bildqualität gegeben ist. Ein Beispiel hierfür können starke Bewegungsartefakte während der Bilddatenerfassung sein. Beispielsweise kann ein Qualitäts-Analysemodul ein trainiertes Qualitäts-Analysemodul umfassen, dass derart trainiert ist, aus in den rekonstruierten Bilddaten vorhandenen Bildartefakten auf eine Bewegung des Patienten zu schlie-βen.

Zudem kann das zumindest eine Qualitäts-Analysemodul auch weitere Messinformationen, beispielsweise physiologische Daten des Patienten, während des Ausführens des Messprogramms überwachen und analysieren. Derartige physiologische Daten können beispielsweise EKG-Daten des Patienten sein, die während des Ausführens des Messprogramms vom Patienten erfasst werden. Zudem können derartige physiologische Daten auch Daten aus einer Überwachung einer Atmung des Patienten und/oder weitere, dem Fachmann als sinnvoll erscheinender physiologischer Daten umfassen. Das zumindest eine Qualitäts-Analysemodul kann dabei beispielsweise eine Wahrscheinlichkeit bestimmen, mit der ein Problem und/oder ein kritischer Zustand in den Bilddaten auftreten kann, wenn keine Gegenmaßnahmen ergriffen werden. Z.B. kann ein Erkennungsalgorithmus, der das EKG-Signal des Patienten während des Ausführens des Messprogramms analysiert, um mögliche Herzrhythmusstörungen zu erkennen, daraus eine Wahrscheinlichkeit für mögliche Bewegungsartefakte bestimmen. In die Bestimmung der Wahrscheinlichkeit kann dabei eine Maß für eine Abweichung des physiologischen Signals von einem erwartenden, normalen physiologischen Signal eingehen. Überschreitet die Wahrscheinlichkeit einen Grenzwert, wird dies von dem zumindest einen Qualitäts-Analysemodul als ein Qualitätsproblem gewertet und/oder erkannt, was sich in der Bewertungsinformation von dem zumindest einen Qualitäts-Analysemodul widerspiegelt.

Das zumindest eine Qualitäts-Analysemodul kann zudem auch nur die Bildqualität in den Bilddaten ermitteln. Dabei kann das zumindest eine Qualitäts-Analysemodul beispielsweise eine der folgenden Bildqualitätseigenschaften berücksichtigen:
- Normalisiertes Quadratisches Mittel (normalized root mean square - NRMS), auch Streuindex genannt, umfasst einen statistischer Fehlerindikator.
- Spitzen-Signal-Rausch-Verhältnis (Peak signal-to-noise ratio - PSNR), umfasst das Verhältnis zwischen der maximal möglichen Leistung eines Signals und der Stärke des störenden Rauschens.
- DCT (discrete cosine transform) subbands similarity (DSS). DSS nutzt wichtige Merkmale der menschlichen visuellen Wahrnehmung aus, indem sie Änderungen in Strukturinformationen in Teilbändern im Bereich der DCT misst und die Qualitätsschätzungen für diese Teilbänder gewichtet.
- Gradient magnitude similarity deviation (GMSD). Bildverläufe reagieren empfindlich auf Bildverzerrungen, während verschiedene lokale Strukturen in einem verzerrten Bild unterschiedlich stark beeinträchtigt werden. Mittels der GMSD wird die pixelweise Gradienten-Magnituden-Ähnlichkeit (GMS) in Kombination mit der Standardabweichung verwendet ein Bildqualitätsindex berechnet.
- Haar wavelet-based Perceptual similarity index (HaarPSI) ist ein Ähnlichkeitsmaß für Bilder, das darauf abzielt, die wahrnehmungsmäßige Ähnlichkeit zweier Bilder in Bezug auf einen menschlichen Betrachter korrekt einzuschätzen.
- Mean deviation similarity index (MDSI) verwendet die Gradientengröße zur Messung struktureller Verzerrungen und Farbigkeitsmerkmale zur Messung von Farbverzerrungen in Bildern. Diese beiden Ähnlichkeitskarten werden kombiniert, um eine Gradienten-Chromatizitäts-Ähnlichkeitskarte zu bilden und daraus die endgültige Qualitätsbewertung zu berechnen.
- Mean structural similarity index measure (MSSIM) misst die Ähnlichkeit zwischen zwei gegebenen Bildern.
- Multi-scale structural similarity index measure (MSSSIM) umfasst eine fortgeschrittenere Form der SSIM, die über mehrere Skalen in einem Prozess mit mehrstufiger Verringerung der Abtastung durchgeführt wird.
- Visual information fidelity (VIF) umfasst einen vollständiger Referenzindex zur Bewertung der Bildqualität, der auf natürlichen Szenenstatistiken und der Vorstellung von Bildinformationen basiert, die vom menschlichen visuellen System extrahiert werden.
- Visual saliency-based index (VSI). Hierbei wird die visuelle Ausprägung bei der Berechnung einer lokalen Qualitätskarte des verzerrten Bildes verwendet. Zudem wird die visuelle Ausprägung auch beim Zusammenfassen des Qualitätsfaktors als Gewichtungsfunktion verwendet, um die Bedeutung einer lokalen Region widerzuspiegeln.
- Deep image structure and texture similarity (DISTS) beschreibt eine Bildqualitätsmethode, die Korrelationen von räumlichen Durchschnittswerte ("Texturähnlichkeit") mit Korrelationen in Merkmalskarten ("Strukturähnlichkeit") kombiniert.
- Learned perceptual image patch similarity (LPIPS) wird verwendet, um die Wahrnehmungsähnlichkeit zwischen zwei Bildern zu beurteilen. LPIPS berechnet im Wesentlichen die Ähnlichkeit zwischen den Aktivierungen zweier Bild-Patches für ein vordefiniertes Netzwerk.
- Perceptual image error metric (PieAPP) misst den Wahrnehmungsfehler eines verzerrten Bildes in Bezug auf eine Referenz und den zugehörigen Datensatz.
- Total variation (TV) identifiziert mehrere leicht unterschiedliche Konzepte, die sich auf die Struktur des Wertebereichs einer Funktion oder eines Maßes beziehen.
- Blind referenceless image spatial quality evaluator (BRISQUE) ist ein Modell, das nur die Bildpixel zur Berechnung von Merkmalen verwendet. Es hat sich als äußerst effizient erwiesen, da zur Berechnung seiner Merkmale keine Transformation erforderlich ist. Es basiert auf dem räumlichen NSS-Modell (Natural Scene Statistics) lokal normalisierter Luminanzkoeffizienten im räumlichen Bereich sowie auf dem Modell für paarweise Produkte dieser Koeffizienten.
- Natural image quality evaluator (NIQE) misst den Abstand zwischen den aus einem Bild berechneten NSS-basierten Merkmalen und den Merkmalen, die aus einer Bilddatenbank erhalten wurden, die zum Trainieren des Modells verwendet wurde. Die Merkmale werden als mehrdimensionale Gaußsche Verteilungen modelliert.

Das zumindest eine klinische Analysemodul ist bevorzugt dazu ausgebildet die Eingangsdaten, insbesondere die rekonstruierten Bilddaten und/oder K-Raum-Daten, auf Auffälligkeiten zu analysieren. Derartige Auffälligkeiten können einen Verdacht auf eine Krankheit, beispielsweise einen Verdacht auf eine Blutung und/oder einen Verdacht auf einen Infarkt, umfassen. Anhand der Eingangsdaten des Analysemoduls, insbesondere der rekonstruierten Bilddaten, kann eine Wahrscheinlichkeit für ein Vorliegen einer Auffälligkeit in den rekonstruierten Bilddaten von dem zumindest einen technischen Analysemodul ermittelt werden. Überschreitet die ermittelte Wahrscheinlichkeit einen Grenzwert, wird dies von dem zumindest einen klinischen Analysemodul als Auffälligkeit und/oder Anomalie gewertet und/oder erkannt, was sich in der Bewertungsinformation von dem zumindest einen klinischen Analysemodul widerspiegelt. Jedoch erfolgt mittels des klinischen Analysemoduls keine Diagnose der Eingangsdaten, insbesondere der rekonstruierten Bilddaten, sondern stellt nur eine Hilfestellung für eine Diagnose durch einen Arzt dar. Insbesondere soll mittels der Analysemodule und der bereitgestellten Bewertungsinformationen und/oder der bereitgestellten Assistenzinformation einem Diagnose erstellendem Arzt eine maximale Unterstützung bereitgestellt werden, indem beispielsweise auch Bilddaten einer erkannten Auffälligkeit und/oder Anomalie bei der Diagnose vorliegen, die bei dem ursprünglich ausgewählten Messprogramm ohne Anpassung aufgrund der Assistenzinformation nicht zwangsläufig vorliegen würden.

Das zumindest eine technische Analysemodul ist bevorzugt dazu ausgebildet, technische und/oder gerätebezogene Informationen, die für das Messprogramm zur Verfügung stehen, zu analysieren. Insbesondere ist das zumindest eine technische Analysemodul zur Analyse von Spulendaten von lokalen Hochfrequenzspulen ausgebildet. Die lokalen Hochfrequenzspulen werden zur Erfassung von Magnetresonanzdaten um den zu untersuchenden Bereich des Patienten positioniert. Für unterschiedliche Körperbereich stehen auch unterschiedliche lokale Hochfrequenzspulen zur Verfügung, beispielsweise eine Kopf-Hochfrequenzspule für eine Kopfuntersuchung oder eine Knie-Hochfrequenzspule für eine Knieuntersuchung. Anhand der Spulendaten kann beispielsweise das zumindest eine technische Analysemodul erkennen, ob für das ausgewählte Messprogramm die dafür erforderliche Hochfrequenzspule mit einer Scannereinheit einer Magnetresonanzvorrichtung verbunden ist. Zudem kann das technische Analysemodul auch zu einer Erkennung und/oder Früherkennung von Hardware-Defekten ausgebildet sein. Beispielsweise das zumindest eine technische Analysemodul dazu ausgebildet sein, aktuelle Betriebsparameter von lokalen Hochfrequenzspulen zu analysieren und daraus auf einen Zustand, insbesondere eine verbleibende Lebensdauer, der lokalen Hochfrequenzspulen oder von einzelnen Komponenten der lokalen Hochfrequenzspule schließen.

Das zumindest eine allgemeine Analysemodul ist bevorzugt dazu ausgebildet, allgemeine Prozesse, die mit dem Ausführen des Messprogramms verbunden sind, zu überwachen und zu analysieren. Dabei kann das zumindest eine allgemeine Analysemodul einen technischen Erkennungsalgorithmus umfassen. Ein derartiger technischer Erkennungsalgorithmus kann beispielsweise zu einer Erkennung und/oder Analyse eines Auslösens einer Bildvorverarbeitung ausgebildet sein. Alternativ oder zusätzlich kann das zumindest eine allgemeine Analysemodul auch einen einstellungsabhängigen Erkennungsalgorithmus umfassen. Ein derartiger einstellungsabhängiger Erkennungsalgorithmus kann beispielsweise Einstellungen des ausgewählten Messprogramms mit vorhandenen Software-Konfigurationen und/oder Software-Lizenzen und/oder mit Scanner-Konfigurationen vergleichen und bei Abweichungen einen Hinweis generieren, dass zur Ausführung des Messprogramms nicht alle Software relevanten und/oder Scanner relevanten Voraussetzungen erfüllt sind und deshalb mit Einschränkungen und/oder Problemen während des Ausführens des Messprogramms zu rechnen ist. Alternativ oder zusätzlich kann das zumindest eine allgemeine Analysemodul auch zu einer Analyse von allgemeinen Prozessen des Messprogramms ausgebildet sein, wie beispielsweise eine Analyse zur Berichterstellung.

Diese Ausgestaltung der Erfindung weist den Vorteil auf, dass für ein breites Spektrum an möglichen Problemen und/oder ein breites Spektrum an kritischen Zuständen bei einer Ausführung des ausgewählten Messprogramms eine Unterstützung, beispielsweise eine Anpassung des ausgewählten Messprogramms aufgrund der bereitgestellten Assistenzinformation nahelegen, durch das Assistenzsystem angeboten wird. Hierdurch kann der Benutzer, insbesondere eine die Magnetresonanzuntersuchung betreuendes medizinisches Bedienpersonal, direkt auf mögliche Komplikationen unterschiedlicher Art während des Ausführens des Messprogramms hingewiesen werden. Dies ermöglicht auch ein frühzeitiges Einleiten von Gegenmaßnahmen durch den Benutzer, so dass trotz vorhandener Komplikationen und/oder Problemen das Messprogramm ausgeführt werden kann.

Alternativ oder zusätzlich kann es vorgesehen sein, dass das zumindest eine Analysemodul eine definierte klinische Fragestellung und/oder eine definierte technische Fragestellung und/oder eine definierte patientenrelevanten Fragestellung in Abhängigkeit von dem ausgewählten Messprogramm analysiert. Bevorzugt ist das zumindest eine Analysemodul auf das ausgewählte Mesprogramm abgestimmt. Das ausgewählte Messprogramm ist anhand einer definierten klinischen und/oder diagnostischen Fragestellung ausgewählt worden. Auch das zumindest eine Analysemodul ist auf die klinische und/oder diagnostische Fragestellung des ausgewählten Messprogramms abgestimmt. Umfasst beispielweise das ausgewählte Messprogramm eine Kopfuntersuchung, kann die definierte klinische Fragestellung des zumindest einen Analysemoduls eine Analyse von Kopfbildern umfassen. Dabei können die Kopfbilder nach Auffälligkeiten und/oder Unregelmäßigkeiten überwacht und/oder analysiert werden. Zudem kann die definierte klinische Fragestellung des zumindest einen Analysemoduls auch einen Zeitpunkt einer Kontrastmittelgabe umfassen. Dabei kann das zumindest eine Analysemodul auch Überwachen und/oder Analysieren, ob ein Kontrastmittel zum richtigen Zeitpunkt verabreicht wurde usw. Bevorzugt umfasst das zumindest eine Analysemodul zur Analyse der definierten klinischen Fragestellung zumindest eine Qualitäts-Analysemodul und/oder ein klinisches Analysemodul.

Zudem kann neben einer definierten klinischen Fragestellung auch eine definierte technische Fragestellung im Zusammenhang mit dem ausgewählten Messprogramm von dem zumindest einem Analysemodul geklärt werden. Bevorzugt umfasst das zumindest eine Analysemodul zur Analyse der definierten technischen Fragestellung zumindest ein technisches Analysemodul. Für beispielsweise Kopfuntersuchungen kann die definierte technische Fragestellung des zumindest einen technischen Analysemoduls sein, "ob die richtige Hochfrequenzspulen verwendet wird" und/oder "ob die Hochfrequenzspule korrekt positioniert und/oder gesteckt ist" und/oder "ob erforderliche Zusatzeinheiten, wie beispielsweise ein Kontrastmittelinjektor, korrekt angeschlossen sind" usw.

Zudem kann neben einer definierten klinischen Fragestellung und/oder einer definierten technischen Fragestellung auch eine definierte patientenrelevante Fragestellung im Zusammenhang mit dem ausgewählten Messprogramm von dem zumindest einem Analysemodul geklärt werden. Bevorzugt umfasst das zumindest eine Analysemodul zur Analyse der definierten patientenrelevanten Fragestellung zumindest eine Qualitäts-Analysemodul und/oder ein klinisches Analysemodul. Ist beispielsweise bereits bekannt, dass der Patient eine koronale Arrhythmie, so wird beispielsweise bei einem ausgewählten Messprogramm, das eine Herzuntersuchung des Patienten umfasst, kein Analysemodul ausgewählt, dass zur Erkennung einer koronalen Arrhythmie ausgebildet ist. Vielmehr wird ein Analysemodul ausgewählt, dass in den rekonstruierten Bilddaten bereits die koronale Arrhythmie des Patienten bei einer Analyse berücksichtigt.

Hierdurch kann eine individuelle Unterstützung des Benutzers bei einer Ausführung des ausgewählten Messprogramms bereitgestellt werden. Insbesondere können für das Messprogramm relevante Fragestellungen überwacht und/oder analysiert werden. Zudem können derart Vorerkrankungen und/oder bereits vorliegende Befunde des Patienten bei der Ausführung des Messprogramms berücksichtigt werden.

In einer vorteilhaften Weiterbildung des erfindungsgemäßen Verfahrens kann es vorgesehen sein, dass das zumindest eine Analysemodul die zumindest eine Bewertungsinformation mittels eines regelbasiertem Algorithmus und/oder eines auf maschinellem Lernen basierten Algorithmus ermittelt. Einem regelbasierter Algorithmus liegen definierte Regeln zugrunde, nach denen dieser Algorithmus die ihm gestellte Aufgabe durchführt. Zur Lösung der Aufgabe kann ein Ergebnis mit zumindest einem Schwellwert verglichen werden und daraus eine Aussage, insbesondere die Bewertungsinformation, abgeleitet werden.

Ein auf einem maschinellen Lernen basierter Algorithmus umfasst bevorzugt ein trainiertes maschinelles Lernverfahren und wurde bereit dazu trainiert, bestimmte Merkmale und/oder ein bestimmtes Muster in den zu analysierenden Eingangsdaten zu erkennen. Im Allgemeinen ahmt ein trainiertes maschinelles Lernverfahren kognitive Funktionen nach, die Menschen mit anderen menschlichen Gedanken assoziieren. Insbesondere durch das Training auf Basis von Trainingsdaten ist das maschinelle Lernverfahren in der Lage, sich an neue Gegebenheiten anzupassen und Muster zu erkennen und zu extrapolieren. Ein anderer Begriff für "trainiertes maschinelles Lernverfahren" ist "trainierte Funktion" oder "trainiertes maschinelles Lernmodell". Generell können Parameter eines maschinellen Lernverfahrens mittels eines Trainings angepasst werden. Insbesondere können ein überwachtes Training, ein teilüberwachtes Training, ein unüberwachtes Training, Reinforcement Learning und/oder aktives Lernen eingesetzt werden. Darüber hinaus kann Repräsentationslernen (ein alternativer Begriff ist "Feature Learning") verwendet werden. Insbesondere die Parameter des maschinelles Lernverfahrens können durch mehrere Trainingsschritte iterativ angepasst werden. Des Weiteren kann innerhalb des Trainings eines neuronalen Netzes der Backpropagation-Algorithmus verwendet werden. Insbesondere kann ein maschinelles Lernverfahren ein neuronales Netz, eine Unterstützungsvektormaschine, einen Entscheidungsbaum und/oder ein Bayes'sches Netzwerk umfassen, und/oder das maschinelles Lernverfahren kann auf k-Means-Clustering, Q-Learning, genetischen Algorithmen und/oder Assoziationsregeln basieren. Insbesondere kann ein neuronales Netz ein tiefes neuronales Netz, ein faltungsneuronales Netz oder ein faltungstiefes neuronales Netz sein. Darüber hinaus kann ein neuronales Netzwerk ein kontradiktorisches Netzwerk, ein tiefes kontradiktorisches Netzwerk und/oder ein generatives kontradiktorisches Netzwerk sein.

Ein künstliches neuronales Netz (KNN, englisch artificial neural network- ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen. Das künstliche neuronale Netz wurde insbesondere im Vorfeld bereits geeignet für die Bestimmung eines Qualitätsmaßes trainiert. Das jeweilige Analysemodul ist dabei auf die definierte spezifische Fragestellung und/oder Aufgabe trainiert.

Zudem kann es auch sein, dass das zumindest eine Analysemodul die zumindest eine Bewertungsinformation mittels eines hybriden Ansatzes, insbesondere einer Kombination aus einem Algorithmus, der ein künstlichen neuronalen Netz umfasst, und einem regelbasiertem Algorithmus. Hierbei hat sich herausgestellt, dass eine derartiger hybrider Ansatz bei Analysemodulen eine besonders hohe Zuverlässigkeit der Bewertungsinformation und/oder der Assistenzinformation umfasst. Bei einem derartigen hybriden Ansatz kann eine erste Teilaufgabe des Analysemoduls durch ein künstliches neuronales Netz, beispielsweise ein Deep Learning Model, gelöst werden und eine zweite Teilaufgabe des Analysemoduls mittels eines regelbasierten Algorithmus.

Auch kann es sein, zur Ermittlung einer Assistenzinformation zwei oder mehr Analysemodule verwendet und/oder benötigt werden. Dabei können die unterschiedlichen Analysemodule auch unterschiedliche Ansätze zur Ermittlung einer Bewertungsinformation aufweisen. Bevorzugt ermittelt dabei jedes der zumindest zwei Analysemodule unabhängig von den weiteren Analysemodulen eine Bewertungsinformation. Anschließend wird aus allen zur Verfügung stehenden Bewertungsinformationen die Assistenzinformation generiert. Das Generieren der Assistenzinformation kann dabei beispielsweise mittels einer künstlichen Intelligenz (KI) erfolgen. Dabei kann es sein, dass zur Generierung der Assistenzinformation die KI die einzelnen Bewertungsinformationen unterschiedliche gewichtet werden. Eine Gewichtung der einzelnen Bewertungsinformationen kann dabei abhängig sein von einer Art des Messprogramms, insbesondere der einzelnen Messschritte, und/oder von einer Art des Analysemoduls.

Vorzugsweise wurde der auf einem maschinellen Lernen basierter Algorithmus dazu trainiert, bestimmte Merkmale und/oder ein bestimmtes Muster in den zu analysierenden Eingangsdaten im Hinblick auf die zu klärende Fragestellung zu erkennen. Für das Training werden bevorzugt Trainingsdatensätze verwendet, deren Eingangsdaten, insbesondere Magnetresonanzdaten, beispielsweise K-Raum-Daten und/oder Magnetresonanz-Rohdaten, und/oder rekonstruierte Bilddaten und/oder weitere Messinformationen, bereits hinsichtlich einer definierten klinischen Fragestellung bewertet wurden. Bevorzugt liegen hierbei Trainingsdatensätze von unterschiedlichen Trainingspatienten vor.

Derart kann eine schnelle und robuste Bewertung der definierten spezifischen Fragestellung und/oder Aufgabe mittels des zumindest einen Analysemoduls während eines Ausführens des Messprogramms für einen Benutzer bereitgestellt werden. Insbesondere kann derart der Benutzer vorteilhaft unterstützt werden und dabei manuelle und/oder subjektive Fehler bei der Bewertung der definierten spezifischen Fragestellung und/oder Aufgabe reduziert und/oder verhindert werden. Zudem kann auch für komplexe Fragestellungen eine schnelle Unterstützung bereitgestellt werden.

In einer vorteilhaften Weiterbildung des erfindungsgemäßen Verfahrens kann es vorgesehen sein, dass anhand der zumindest einen bereitgestellten Bewertungsinformation des zumindest einen Analysemoduls zumindest ein Vorschlag für zumindest einen Messschritt des Messprogramms mittels eines Empfehlungsmoduls des Assistenzsystems ermittelt wird und der zumindest eine Vorschlag für den zumindest einen Messschritt bereitgestellt wird. Das Assistenzsystem kann dabei mehrere Empfehlungsmodule umfassen, wobei die einzelnen Empfehlungsmodule zur Ermittlung zumindest eines Vorschlags für jeweils eine definierte und/oder spezifische Fragestellung ausgebildet sind. Die Ermittlung des zumindest einen Vorschlags ist dabei abhängig von Eingangsdaten des zumindest einen Empfehlungsmoduls, wobei die Eingangsdaten die von dem zumindest einen Analysemodul bereitgestellt Bewertungsinformation umfassen. Der zumindest eine Vorschlag des jeweiligen Empfehlungsmoduls ist dabei von Ausgangsdaten des Empfehlungsmoduls umfasst. Bei Vorliegen von Bewertungsinformationen von unterschiedlichen Analysemodulen können auch unterschiedliche Empfehlungsmodule zur Ermittlung zumindest jeweils eines Vorschlags vorgesehen sein, wobei die unterschiedlichen Empfehlungsmodule nach unterschiedlichen Fragestellungen einen Vorschlag ermitteln. Zudem kann auch ein einziges Empfehlungsmodul einen Vorschlag anhand von Bewertungsinformationen von zwei oder mehr Analysemodulen ermitteln. Bei Messprogrammen, bei denen eine Entscheidung vom Benutzer zum weiteren Messverlauf in Abhängigkeit der erfassten Magnetresonanzdaten während des Ausführens des Messprogramms festgelegt werden muss, kann zudem der Vorschlag eine Empfehlung für den weiteren Messverlauf angeben.

Eine Datenübertragung zwischen den Analysemodulen und den Empfehlungsmodulen erfolgt bevorzugt mittels des Transaktionsmanagers des Assistenzsystems. Beispielsweise kann der Transaktionsmanager die Bewertungsinformation von Analysemodulen für Empfehlungsmodule bereitstellen. Insbesondere ist der Transaktionsmanager dazu ausgebildet, die Bewertungsinformation eines bestimmten und/oder speziellen Analysemoduls einem Empfehlungsmodul bereitzustellen, dass zur Ermittlung zumindest eines Vorschlags anhand der Bewertungsinformation des bestimmten und/oder speziellen Analysemoduls ausgebildet ist. Hierzu können die einzelnen Analysemodule und/oder die einzelnen Empfehlungsmodule jeweils eine entsprechende Schnittstelle aufweisen, die mit dem Transaktionsmanager kommuniziert.

Eine Auswahl und/oder Konfigurierung des zumindest einen Empfehlungsmoduls erfolgt bevorzugt mittels des Konfigurations-Userinterfaces des Assistenzsystems. Dabei kann das zumindest eine Empfehlungsmodul direkt für zumindest einen Messschritt ausgewählt und konfiguriert werden. Zudem kann auch bei einer Auswahl zumindest eines Analysemoduls zumindest ein Empfehlungsmodul dem Benutzer zur Auswahl vorgeschlagen werden. Des Weiteren kann es auch sein, dass der Benutzer zumindest ein Empfehlungsmodul an dem Konfigurations-Userinterface des Assistenzsystems auswählt und dabei einen Vorschlag für zumindest ein Analysemodul zur Auswahl erhält, wobei das zumindest eine Analysemodul dabei die Eingangsdaten für das zumindest eine Empfehlungsmodul liefern kann.

Diese Ausgestaltung der Erfindung weist den Vorteil auf, dass während eines Ausführens des Messprogramms einem Benutzer direkt ein Vorschlag zur Verbesserung und/oder zur Abhilfe und/oder Prävention eines von einem Analysemodul erkannten Problems und/oder einer von einem Analysemodul erkannten Auffälligkeit bereitgestellt werden kann. Somit wird der Benutzer nicht nur auf ein Problem und/oder eine Auffälligkeit aufmerksam gemacht, sondern auch gleich einen Vorschlag zur Behebung des Problems und/oder zur Analyse der Auffälligkeit unterbreitet. Dies ermöglicht es insbesondere unerfahrenen Benutzern auch bei Problempatienten das Messprogramm erfolgreich abzuschließen und für eine Diagnose relevante Bilddaten bereitzustellen.

Alternativ oder zusätzlich kann es vorgesehen sein, dass das zumindest eine Empfehlungsmodul zusammen mit zumindest einem Analysemodul auf eine definierte klinische Fragestellung und/oder eine definierte technische Fragestellung des Messprogramms abgestimmt ist. Der zumindest eine Vorschlag des zumindest einen Empfehlungsmoduls umfasst bevorzugt einen Vorschlag zu einer Problembehebung für den zumindest einen Messschritt, sofern die bereitgestellte Bewertungsinformation ein Problem und/oder einen kritischer Zustand in dem zumindest einen Messschritt erkannt hat. Beispielsweise ist zumindest ein technisches Analysemodul und zumindest ein technisches Empfehlungsmodul zur Beantwortung einer definierten technischen Fragestellung, die mit dem ausgewählten Messprogramm verbunden sein kann, vorgesehen. Ein technisches Empfehlungsmodul ist zu einer Verarbeitung von zumindest einer Bewertungsinformation zumindest eines technischen Analysemoduls ausgebildet. Beispielsweise kann bei einem erkannten Problem und/oder einem erkannten kritischen Zustand einer falsch positionierten lokalen Hochfrequenzspule mittels eines technischen Analysemoduls das technische Empfehlungsmodul einem Benutzer einen Vorschlag für eine korrekte Positionierung der lokalen Hochfrequenzspule unterbreiten. Damit kann einem Benutzer für ein konkretes Problem ein konkreter, auf das Problems abgestimmter Vorschlag zur Behebung des Problems unterbreitet werden.

Zudem kann zumindest ein Qualitäts-Analysemodul und/oder zumindest ein klinisches Analysemodul zusammen mit einem klinischen Empfehlungsmodul zur Beantwortung einer klinischen Fragestellung, die mit dem ausgewählten Messprogramm verbunden sein kann, vorgesehen sein. Ein klinisches Empfehlungsmodul ist zu einer Verarbeitung von zumindest einer Bewertungsinformation zumindest eines klinischen Analysemoduls und/oder zumindest eines Qualitäts-Analysemoduls ausgebildet. Beispielsweise kann bei einem erkannten Problem und/oder einem erkannten kritischen Zustand einer unerwünschten Patientenbewegung während einer Magnetresonanzdatenerfassung das technische Empfehlungsmodul ein Widerholen des Messschritts und/oder eine Anpassung zumindest eines Messparameters und/oder ein Auswählen einer alternativen Messsequenz für diesen Messschritt usw. vorschlagen. Damit kann einem Benutzer für ein konkretes technisches Problem ein konkreter technischer, auf das Problem abgestimmter Vorschlag zur Behebung des Problems unterbreitet werden.

Alternativ oder zusätzlich kann es vorgesehen sein, dass zur Ermittlung des Vorschlags das zumindest eine Empfehlungsmodul zumindest einen auf ein erkanntes Problem und/oder eine erkannte Auffälligkeit angepassten Vorschlag, vorzugsweise auf mehrere auf das erkannte Problem und/oder auf die erkannte Auffälligkeit angepasste Vorschläge, umfasst. Vorzugsweise umfasst ein Empfehlungsmodul mehrere Vorschläge, die zur Behebung eines Problems und/oder einer Auffälligkeit oder auch zur Behebung mehrere Probleme und/oder mehrerer Auffälligkeiten zur Verfügung stehen. Von dem Empfehlungsmodul wird dann derjenige Vorschlag zur Behebung eines erkannten Problems und/oder einer erkannten Auffälligkeit ausgewählt, der anhand der bereitgestellten Bewertungsinformationen die beste Lösung für das erkannte Problem und/oder die erkannte Auffälligkeit anbietet. Beispielsweise kann hierzu das zumindest eine Empfehlungsmodul einen regelbasierten Algorithmus aufweisen, der anhand der Eingangsdaten, insbesondere zumindest einer Bewertungsinformation zumindest eines Analysemoduls, einen Vorschlag und/oder eine Empfehlung für das weitere Vorgehen ermittelt und bereitstellt. Dies ermöglicht es, dass für unterschiedliche erkannte Probleme und/oder Komplikationen und/oder unterschiedliche erkannte Auffälligkeiten auch unterschiedliche Lösungen mittels des zumindest einen Empfehlungsmoduls bereitgestellt und/oder einem Benutzer angeboten werden können.

Alternativ oder zusätzlich kann es vorgesehen sein, dass das zumindest eine Empfehlungsmodul mehrere zur Verfügung stehende Vorschläge umfasst, wobei die mehreren Vorschläge in einer Datenbank gespeichert sind. Die Datenbank kann dabei von dem Empfehlungsmodul umfasst sein. Dies ermöglicht ein schnelles Abrufen der Vorschläge für eine Ausgabe an den Benutzer. Zudem ist es auch denkbar, dass die Datenbank mehreren Empfehlungsmodulen zur Verfügung steht, wobei unterschiedliche Empfehlungsmodule mit einem gleichen Vorschlag in der Datenbank verlinkt und/oder verknüpft sein können. Beispielsweise können unterschiedliche Empfehlungsmodule für unterschiedliche klinische Fragestellungen, bei denen jedoch ein gleiches Problem erkannt wurde, beispielsweise eine unerwünschte Bewegung eines Patienten, ähnliche oder gleiche Vorschläge für den Benutzer bereitstellen.

Alternativ oder zusätzlich kann es vorgesehen sein, dass das zumindest eine Empfehlungsmodul eine erweiterte Assistenzinformation ermittelt, wobei die erweiterte Assistenzinformation den zumindest einen ermittelten Vorschlag und/oder eine Statusinformation des zumindest einen Empfehlungsmoduls umfasst. Die Statusinformation des Empfehlungsmoduls kann dabei dem Benutzer anzeigen, welcher Art das Empfehlungsmodul ist, beispielsweise ob ein Qualitäts-Empfehlungsmodul und/oder ein technisches Empfehlungsmodul und/oder ein klinisches Empfehlungsmodul für die Beurteilung des Messschritts zur Verfügung steht und/oder ausgewählt wurde. Zudem kann die Statusinformation dem Benutzer auch anzeigen, ob aktuell eine Analyse mittels des Empfehlungsmoduls durchgeführt wird. Zudem kann die Statusinformation dem Benutzer auch anzeigen, ob die Analyse mittels des Empfehlungsmoduls erfolgreich beendet wurde und ein Ergebnis, insbesondere ein Vorschlag, vorliegt. Dabei kann auch die Statusinformation dem Benutzer anzeigen, ob weitere Schritte zur Behebung eines Problems und/oder zur Klärung einer Auffälligkeit erforderlich sind. Zudem kann die Statusinformation dem Benutzer anzeigen, ob die Analyse mittels des Empfehlungsmoduls nicht durchgeführt werden konnte. Bevorzugt wird die Statusinformation des Empfehlungsmoduls durch entsprechende Symbole angezeigt, die vorteilhafterweise an einer definierten Position auf der Benutzeroberfläche angeordnet sind. Hierdurch kann der Benutzer einen schnellen Überblick über das Empfehlungsmodul erhalten. Zudem kann neben der Statusinformation dem Benutzer auch direkt einen möglichen Lösungsvorschlag angezeigt werden, der zu einer Behebung eines möglichen Problems und/oder zur Klärung einer Auffälligkeit beitragen kann.

Alternativ oder zusätzlich kann es vorgesehen sein, dass die erweiterte Assistenzinformation über eine Benutzeroberfläche zur Steuerung und/oder Überwachung des Messprogramms an einen Benutzer ausgegeben wird. Die Benutzeroberfläche zur Steuerung und/oder Überwachung des Messprogramms zeigt dabei bevorzugt die einzelnen Messschritte des Messprogramms an. Dabei können für die einzelnen Messschritte auch die für den jeweiligen Messschritt aktivierten und/oder ausgewählten Empfehlungsmodule angezeigt werden. Bevorzugt wird die erweiterte Assistenzinformation für den jeweiligen Messschritt in der Benutzeroberfläche angezeigt. Insbesondere erfolgt hierbei die Anzeige und/oder Ausgabe der erweiterten Assistenzinformation an einer definierten Position an der Benutzeroberfläche. Derart kann eine einfache und schnelle Übermittlung der erweiterten Assistenzinformation an den Benutzer erfolgen. Insbesondere kann sich hierbei der Benutzer auf eine einzige Benutzeroberfläche konzentrieren und erhält alle für das Messprogramm zur Verfügung stehenden Informationen inklusive der erweiterten Assistenzinformation.

Alternativ oder zusätzlich kann es vorgesehen sein, dass das Assistenzsystem auch eine eigene Benutzeroberfläche zur Ausgabe von Informationen, insbesondere der erweiterten Assistenzinformation, umfasst. Eine derartige Benutzeroberfläche kann beispielsweise ein Assistenz-Userinterface umfassen, das neben der Assistenzinformation den Benutzer auch darüber informieren kann, welches Analysemodul und/oder Empfehlungsmodul gerade ausgeführt wird und für welchen Messschritt dieses ausgewählt wurde.

Alternativ oder zusätzlich kann es vorgesehen sein, dass nach einem Ausführen eines Vorschlags eine Feedback-Information von einem Benutzer bezüglich eines Erfolgs des Vorschlags eingebbar ist. Mittels des Feedbacks kann ein Erfolg der vorgeschlagenen Lösungen ermittelt werden. Sofern das zumindest eine Empfehlungsmodul und/oder das Assistenzsystem einen selbstlernenden Algorithmus umfasst, kann dabei eine Reihenfolgen der zur Verfügung stehenden Vorschläge an eine Erfolgsquote angepasst werden, so dass ein Benutzer zunächst den am erfolgversprechendsten Vorschlag zur Verbesserung und/oder Abhilfe eines erkannten Problems und/oder den am erfolgversprechendsten Vorschlag zur Klärung einer Auffälligkeit erhält.

In einer vorteilhaften Weiterbildung des erfindungsgemäßen Verfahrens kann es vorgesehen sein, dass das Assistenzsystem zu einer Ausführung des zumindest einen Vorschlags für den zumindest einen Messschritt ausgebildet ist. Vorzugsweise umfasst das Assistenzsystem eine Steuereinheit, die eine Ausführung des zumindest einen Vorschlags für den zumindest einen Messschritt steuert. Zudem kann auch das zumindest eine Empfehlungsmodul zu einer Ausführung des zumindest einen Vorschlags ausgebildet sein. Auch hier umfasst vorteilhaft das zumindest eine Empfehlungsmodul eine Steuereinheit, die eine Ausführung des zumindest einen Vorschlags für den zumindest einen Messschritt steuert. Dabei steuert die Steuereinheit die Hardware-Komponenten zur Ausführung des zumindest einen Messschritts entsprechend dem zumindest einen Vorschlag. Dabei kann ein derartiger Vorschlag ein Wiederholen des Messschritts und/oder eine Anpassung zumindest eines Messparameters bei einem Wiederholen des Messschritts und/oder ein Durchführen eines alternativer Messschritts mit einer zu dem bisherigen Messschritt alternativen Messsequenz usw. umfassen.

Die Steuereinheit des Assistenzsystems umfasst zumindest ein Rechenmodul und/oder einen Prozessor, wobei die Steuereinheit zum Ausführen zumindest eines von dem Empfehlungsmodul vorgeschlagenen Vorschlags ausgebildet ist. So ist insbesondere die Steuereinheit dazu ausgebildet, computerlesbare Instruktionen auszuführen, um das Vorschlag auszuführen. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen, um den Vorschlag auszuführen. Derart ist die Steuereinheit dazu ausgebildet, zumindest eines von dem Empfehlungsmodul vorgeschlagenen Vorschlags auszuführen.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Diese Ausgestaltung der Erfindung weist den Vorteil auf, dass eine einfache und schnelle Umsetzung des zumindest einen von dem Empfehlungsmodul vorgeschlagenen Vorschlags für einen Benutzer bereitgestellt werden kann. Insbesondere kann derart auch eine korrekte Umsetzung und/oder Ausführung des zumindest einen Vorschlags gewährleistet werden.

Alternativ oder zusätzlich kann es vorgesehen sein, dass das Assistenzsystems zu einer Ausführung des zumindest einen Vorschlags für den zumindest einen Messschritt in einem halbautomatischen Ausführungsmodus oder in einem vollautomatischen Ausführungsmodus ausgebildet ist. In dem halbautomatischen Ausführungsmodus erfolgt das Ausführen erst, nachdem der Benutzer eine Bestätigungseingabe getätigt hat. Diese Bestätigungseingabe ist für das Assistenzsystem ein Auslöser für eine automatische Ausführung des zumindest einen Vorschlags. Lehnt der Benutzer jedoch das automatische Ausführen des Vorschlags durch das Assistenzsystem ab, obliegt es dem Benutzer den Vorschlag manuell auszuführen oder nicht auszuführen. In dem automatischen Ausführungsmodus wird der zumindest eine Vorschlag ohne vorheriges Nachfragen beim Benutzer automatisch ausgeführt. Zudem kann es vorgesehen sein, dass nach einer Ausführung eines von einem Empfehlungsmodul vorgeschlagenen Vorschlags mittels des halbautomatischen oder des automatischen Ausführungsmodus dem Benutzer eine Information über die Ausführung ausgegeben wird. Dabei kann eine einfache und schnelle Umsetzung des zumindest einen von dem Empfehlungsmodul vorgeschlagenen Vorschlags für einen Benutzer bereitgestellt werden kann. Insbesondere kann derart auch eine korrekte Umsetzung und/oder Ausführung des zumindest einen Vorschlags gewährleistet werden.

Alternativ oder zusätzlich kann es vorgesehen sein, dass der Benutzer für jeden Messschritt des Messprogramms den Ausführungsmodus zur Ausführung eines Vorschlags auswählen kann. Bevorzugt erfolgt die Auswahl des Ausführungsmodus für jeden der Messschritte des Messprogramms mittels des Konfigurations-Userinterfaces. Das Assistenzsystem ermöglicht dabei dem Benutzer, für jeden Messschritt eine individuelle Auswahl des Ausführungsmodus anzugeben. Dabei kann der Benutzer für einzelne Messschritte, die von besonderem Interesse für den Benutzer sind, den halbautomatischen Ausführungsmodus wählen und hat damit die Kontrolle über mögliche auszuführende Vorschläge beibehalten.

In einer vorteilhaften Weiterbildung des erfindungsgemäßen Verfahrens kann es vorgesehen sein, dass das Messprogramm mehrere Messschritte umfasst und für zumindest einen der mehreren Messschritte eine Unterstützung mittels des Assistenzsystems für einen Benutzer auswählbar ist. Dabei kann der Benutzer bei besonders kritischen Messschritten eine Unterstützung mittels des Assistenzsystems auswählen. Bevorzugt erfolgt das Auswählen einer Unterstützung für zumindest einen der Messschritte mittels des Konfigurations-Userinterfaces. Besonders vorteilhaft ist hierbei für jeden der mehreren Messschritte des Messprogramms eine Unterstützung mittels des Assistenzsystems für einen Benutzer auswählbar. Das Bereitstellen einer Auswahl von zumindest einen Empfehlungsmodul und/oder zumindest einen Analysemodul für den zumindest einen Messschritt erfolgt dabei bevorzugt mittels eines Konfigurations-Userinterfaces des Assistenzsystems. Insbesondere kann hierbei der Benutzer eine individuelle Unterstützung für jeden der mehreren Messschritte auswählen und einstellen. Beispielsweise können Benutzer mit langjähriger Erfahrung mit Magnetresonanzuntersuchungen nur für besonders kritische Messschritte des Messprogramms eine Unterstützung auswählen. Dagegen können Benutzer mit geringer Erfahrung mit Magnetresonanzuntersuchungen für alle Messschritte eines Messprogramms eine Unterstützung auswählen. Beispielsweise kann ein Benutzer mittels des Konfigurations-Userinterfaces auswählen, für welche Messschritte er eine Unterstützung haben möchte, insbesondere welche Analysemodule und/oder Empfehlungsmodule er für bestimmte Messschritt haben möchte. Zudem kann der Benutzer auch die einzelnen Module, insbesondere die einzelnen Analysemodule und/oder Empfehlungsmodule konfigurieren, ob er nur ein Feedback und/oder eine Information zu den einzelnen Messschritten haben möchte oder auch eine Empfehlung für das weitere Vorgehen des Messprogramms.

Alternativ oder zusätzlich kann es vorgesehen sein, dass für zumindest einen der mehreren Messschritte des Messprogramms zumindest ein Analysemodul und/oder zumindest ein Empfehlungsmodul zugeordnet ist und von einem Benutzer auswählbar ist. Bevorzugt sind von dem Assistenzsystem zumindest ein Analysemodul und/oder zumindest ein Empfehlungsmodul dem zumindest einen Messschritt des Messprogramms zugeordnet, wobei die Zuordnung mittels des Konfigurations-Userinterfaces des Assistenzsystems erfolgt. Das zumindest eine Analysemodul und/oder das zumindest eine Empfehlungsmodul ist dabei bevorzugt auf eine klinische und/oder diagnostische Fragestellung des Messprogramms und/oder des zumindest einen Messschritts abgestimmt. Umfasst beispielsweise das Messprogramm eine Kopfuntersuchung des Patienten, ist das zumindest eine zugeordnete Analysemodul zur Analyse von Kopf-Bilddaten und/oder weiterer Messinformation bezüglich der Kopfuntersuchung ausgebildet. Auch das zumindest eine Empfehlungsmodul ist für eine Ermittlung eines Vorschlags bezüglich einer Kopfuntersuchung ausgebildet. Umfasst dagegen das Messprogramm beispielsweise eine Herzuntersuchung des Patienten, ist das zumindest eine zugeordnete Analysemodul zur Analyse von Herz-Bilddaten und/oder weiterer Messinformation bezüglich der Herzuntersuchung ausgebildet. Auch das zumindest eine Empfehlungsmodul ist für eine Ermittlung eines Vorschlags bezüglich einer Herzuntersuchung ausgebildet. Insbesondere kann hierbei für einen Benutzer eine voreilhafte Unterstützung bei der Ausführung des Messprogramms zur Verfügung gestellt werden. Vorzugsweise kann hierbei der Benutzer bereits bei einer Auswahl von zumindest einem Analysemodul und/oder zumindest einem Empfehlungsmodul vorteilhaft von dem Assistenzsystem unterstützt werden. Zudem kann eine Auswahl von Analysemodulen und/oder Empfehlungsmodulen, die für eine Unterstützung des ausgewählte Messprogramms nicht geeignet ist, vorteilhaft reduziert und/oder verhindert werden.

Alternativ oder zusätzlich kann es vorgesehen sein, dass für zumindest einen der mehreren Messschritte des Messprogramms zumindest ein Analysemodul von einem Benutzer auswählbar ist, wobei die Auswahl des zumindest einen Analysemoduls einen Vorschlag für eine Auswahl zumindest eines Empfehlungsmoduls umfasst. Beispielseise kann eine derartige Verknüpfung zwischen einem Analysemodul und zumindest einem Empfehlungsmodul auch mittels eines Konfigurations-Userinterfaces des Assistenzsystems erfolgen, der bei Auswahl des zumindest einen Analysemoduls die von dem zumindest einen Analysemodul bereitgestellten Ausgangsdaten, insbesondere die zumindest eine Bewertungsinformation, ein für die Verarbeitung der von dem Analysemodul bereitgestellten Ausgangsdaten ausgebildetes Empfehlungsmodul auswählt und/oder zur Auswahl bereitstellt. Bevorzugt ist hierbei die Auswahl des zumindest einen Empfehlungsmoduls mit dem zumindest einen ausgewählten Analysemodul zudem abhängig von einem untersuchungsrelevanten und/oder messprogrammrelevanten Kontext. Hierdurch kann für einen Benutzer eine vorteilhafte Unterstützung zur Verfügung gestellt werden. Insbesondere kann dabei dem Benutzer ein Empfehlungsmodul vorgeschlagen werden, das mit dem ausgewählten Analysemodul kompatibel ist. Des Weiteren kann dabei eine Auswahl eines Empfehlungsmoduls, das für eine Unterstützung des ausgewählte Messprogramms nicht geeignet ist oder inkompatibel zu dem ausgewählten Analysemodul ist, vorteilhaft reduziert und/oder verhindert werden.

Alternativ oder zusätzlich kann es vorgesehen sein, dass für zumindest einen der mehreren Messschritte des Messprogramms zumindest ein Empfehlungsmodul von einem Benutzer auswählbar ist, wobei das zumindest eine Empfehlungsmodul einen Dateneingang voraussetzt, wobei der Dateneingang automatisch mit zumindest einem Analysemodul verknüpft ist. Das zumindest eine Empfehlungsmodul kann zur Ermittlung von Vorschlägen für eine definierte Magnetresonanzuntersuchung und/oder ein definiertes Messprogramm und/oder eine definierte klinische und/oder diagnostische Fragestellung ausgebildet sein. Die für die Ermittlung von Vorschlägen benötigten Eingangsdaten werden durch die automatische Verknüpfung und/oder Auswahl von zumindest einem Analysemodul während eines Ausführens des Messprogramms bereitgestellt. Beispielseise kann eine derartige Verknüpfung zwischen zumindest einem Empfehlungsmodul und zumindest einem Analysemodul auch mittels eines Konfigurations-Userinterfaces des Assistenzsystems erfolgen, der bei Auswahl des zumindest einen Empfehlungsmoduls anhand der erforderlichen Eingangsdaten für das zumindest eine Empfehlungsmodul und anhand der klinischen Fragestellung die mit den erforderlichen Eingangsdaten verknüpften Analysemodule auswählt und/oder aktiviert. Ist das zumindest eine Empfehlungsmodul zur Ermittlung von Vorschlägen für eine komplexe Magnetresonanzuntersuchung ausgebildet, kann es auch sein, dass mehrere Analysemodule mit dem zumindest einem Empfehlungsmodul automatisch verknüpft sind und bei Auswahl des Empfehlungsmoduls automatisch mit ausgewählt werden. Beispielsweise kann ein Empfehlungsmodul, dass für Herzuntersuchungen ausgebildet ist, mit einem Analysemodul, das eine EKG-Überwachung des Patienten analysiert, einem Analysemodul, das eine Atemüberwachung des Patienten analysiert, und einem Analysemodul, das die erfassten Herz-Bilddaten analysiert, verknüpft sein. Für eine vollständige Überwachung und Unterstützung der Herzuntersuchung benötigt das Empfehlungsmodul dabei die Bewertungsinformationen der drei Analysemodule als Eingangsdaten. Bevorzugt ist das Empfehlungsmodul entsprechend der unterschiedlichen Eingangsdaten auch dazu ausgebildet, für unterschiedliche mögliche Probleme und/oder für unterschiedliche Auffälligkeiten Vorschläge zu ermitteln und dem Benutzer zu unterbreiten. Hierdurch kann für einen Benutzer eine vorteilhafte Unterstützung zur Verfügung gestellt werden. Insbesondere kann dabei gewährleistet werden, dass alle erforderlichen Eingangsdaten für eine vollständige Unterstützung und/oder Überwachung des Messprogramms vorliegen.

In einer vorteilhaften Weiterbildung des erfindungsgemäßen Verfahrens kann es vorgesehen sein, dass das Messprogramm mehrere Messschritte aufweist und die mehreren Messschritte des Messprogramms nacheinander ausgeführt werden, wobei für zumindest einen Messschritt eine Unterstützung mittels des Assistenzsystems ausgewählt wurde, wobei ein dem zumindest einen Messschritt, für den die Unterstützung ausgewählt wurde, nachfolgender Messschritt erst gestartet wird, wenn bei dem zumindest einem Messschritt, für den die Unterstützung ausgewählt wurde, die Unterstützung mittels des Assistenzsystems abgeschlossen ist. Hierdurch kann eine einfache Art der Unterstützung realisiert werden. Insbesondere ist für unerfahrene Benutzer eine vorteilhafte Übersichtlichkeit für die einzelnen Messschritte und für die angezeigten Informationen und/oder Vorschläge zur Unterstützung des jeweiligen Messschritts gegeben. Damit kann auch eine mögliche Verwechslung von Maßnahmen für einzelne Messschritte für einen Benutzer reduziert und/oder verhindert werden.

In einer vorteilhaften Weiterbildung des erfindungsgemäßen Verfahrens kann es vorgesehen sein, dass das Messprogramm mehrere Messschritte umfasst und für jeden Messschritt des Messprogramms, für den eine Unterstützung mittels des Assistenzsystems ausgewählt wurde, die Unterstützung mittels des Assistenzsystems in einem Quasi-Echtzeitmodus oder in einem Echtzeitmodus durchgeführt wird. Der Quasi-Echtzeitmodus ist bevorzugt dazu ausgebildet, dass insbesondere vordefinierte Blöcke von teilweise erfassten Daten noch während des Ausführens des Messschritts an das entsprechende Analysemodul zur Analyse übertragen werden. Derart kann bereits eine Datenanalyse durchgeführt und der Anwender unterstützt werden, während der Messschritt noch ausgeführt wird. Dies ermöglicht eine unmittelbarere Rückmeldung von möglichen Problemen und/oder von möglichen Auffälligkeiten an den Benutzer. Zudem kann hierdurch auch das Messprogramm erheblich beschleunigt werden und Messzeit und/oder Untersuchungszeit eingespart werden. Beispielsweise kann ein Analysemodul Segmente und/oder Blöcke eines Atemsignals des Patienten, die in dem aktuell ausgeführten Messschritt erfasst werden, hinsichtlich der Fragestellung, ob der Patient den Befehl zum Atemanhalten erfüllt oder nicht, analysieren. Derart kann stets überwacht werden, insbesondere noch während des aktuellen Messschritts, ob der Patient die Bedingungen für eine Erfassung von Magnetresonanzdaten erfüllt. Mögliche Vorschläge, beispielsweise Warnungen und/oder Workflow-Empfehlungen, eines Empfehlungsmoduls, basierend auf den Bewertungsinformationen des Analysemoduls, können auch während des Messschritts ausgeführt werden.

Der Echtzeitmodus ist bevorzugt dazu ausgebildet, dass erfasste Daten kontinuierlich in Echtzeit an die entsprechenden und/oder ausgewählten Analysemodule übertragen werden. Die Analysemodule ermitteln in Echtzeit Bewertungsinformationen mit einem kontinuierlichen Datenstrom, während der Messschritt ausgeführt wird. Beispielseise ist es vorteilhaft, eine Bewegungsdetektion des Patienten bei bewegungskritischen Messschritten kontinuierlich und in Echtzeit zu überwachen. Hierdurch kann eine Datenanalyse durch die Analysemodule beschleunigt werden. Auch kann dem Benutzer eine Unterstützung angeboten werden, sobald eine kritischer Zustand von einem Analysemodul erkannt wird. Insbesondere kann dem Benutzer jederzeit während eines Ausführens des Messschritts eine Unterstützung angezeigt werden.

Eine Auswahl eines Ausführungsmodus für eine Unterstützung durch das Assistenzsystems, insbesondere ob ein einfacher Ausführungsmodus oder ein Quasi-Echtzeitmodus oder ein Echtzeitmodus gewünscht ist, erfolgt bevorzugt bei der Auswahl und/oder Konfigurierung der einzelnen Module mittels des Konfigurations-Userinterfaces.

In einer vorteilhaften Weiterbildung des erfindungsgemäßen Verfahrens kann es vorgesehen sein, dass ein Austausch und/oder das Bereitstellen der Magnetresonanzdaten und/oder der Bilddaten und/oder der weiteren Messinformationen und/oder der zumindest einen Bewertungsinformation und/oder der zumindest einen Assistenzinformation und/oder zumindest einer erweiterten Assistenzinformation eines Empfehlungsmoduls mittels eines Transaktionsmanagers des Assistenzsystems erfolgt. Dabei steuert der Transaktionsmanager auch eine Datenübertragung zwischen einzelnen Analysemodulen und einzelnen Empfehlungsmodulen, so dass für jedes Analysemodul und/oder Empfehlungsmodul auch die korrekten und/oder relevanten Eingangsdaten für eine Weiterverarbeitung vorliegen. Hierzu kann der Transaktionsmanager auch eine Recheneinheit und/oder eine Steuereinheit aufweisen. Hierdurch kann für einen Benutzer eine vorteilhafte Unterstützung zur Verfügung gestellt werden. Insbesondere kann dabei gewährleistet werden, dass eine Funktionsweise der einzelnen Module für eine Unterstützung des Benutzers während eines Ausführens des Messprogramms gewährleistet werden kann. Insbesondere kann derart für alle für die Unterstützung des Benutzers erforderlichen Module die für ihre Funktionsweise erforderlichen Eingangsdaten bereitgestellt werden.

In einer vorteilhaften Weiterbildung des erfindungsgemäßen Verfahrens kann es vorgesehen sein, dass das Assistenzsystem ein Konfigurations-Userinterface umfasst, wobei das Konfigurations-Userinterface dazu ausgebildet ist, eine Auswahl zumindest eines Analysemoduls und/oder zumindest eines Empfehlungsmoduls für zumindest einen Messschritt zu konfigurieren. Das Konfigurations-Userinterface ist bevorzugt dazu ausgebildet, für ein Messprogramm und/oder einzelnen Messchritten eines Messprogramms einem Benutzer eine Auswahl an zu Verfügung stehenden Modulen, insbesondere Analysemodulen und/oder Empfehlungsmodulen, zur Auswahl und/oder Konfigurierung bereitzustellen. Dabei kann der Benutzer festlegen, mit welchem Analysemodul und/oder Empfehlungsmodul an welcher Position im Messprogramm eine Unterstützung erfolgen soll oder auch für welchen Messschritt eine Unterstützung mit zumindest einem Analysemodul und/oder zumindest einem Empfehlungsmodul erfolgen soll. Zudem kann der Benutzer bei manchen Analysemodulen und/oder Empfehlungsmodulen auch die Möglichkeit haben, mittels des Konfigurations-Userinterfaces einen Datentyp und/oder ein Datenformat von Eingangsdaten für das ausgewählte Modul auszuwählen und/oder das entsprechende Modul an einen Messschritt anzupassen. Zudem kann mittels des Konfigurations-Userinterfaces auch eine Verknüpfung von mehreren Analysemodulen von einem Benutzer konfiguriert und/oder ausgewählt werden. Eine Auswahl zumindest eines Moduls, insbesondere zumindest eines Empfehlungsmoduls und/oder zumindest eines Analysemoduls, und/oder eine Kombination von Modulen kann dabei auch für zumindest einen Messschritt dem Benutzer mittels Konfigurations-Userinterface vorgeschlagen werden. Weiterhin kann der Benutzer auch die einzelnen Module, insbesondere die einzelnen Analysemodule und/oder Empfehlungsmodule konfigurieren, ob er nur ein Feedback und/oder eine Information zu den einzelnen Messschritten haben möchte oder auch eine Empfehlung für das weitere Vorgehen des Messprogramms.

Hierdurch kann für einen Benutzer eine einfache und schnelle Auswahl eines Moduls, insbesondere zumindest eines Analysemoduls und/oder zumindest eines Empfehlungsmoduls erfolgen. Insbesondere ermöglicht diese Ausgestaltung der Erfindung auch ungeübten und/oder unerfahrenen Benutzern eine einfache Verwendung des Analysemoduls und/oder Empfehlungsmoduls während eines Ausführens eines Messprogramms. Zudem kann derart auch sichergestellt werden, dass ein Datensatz, insbesondere ein Bilddatensatz, nach Beenden des Messprogramms zur Verfügung steht, aus dem eine Diagnose erstellt werden kann.

Alternativ oder zusätzlich kann es vorgesehen sein, dass das Konfigurations-Userinterface dazu ausgebildet ist, ein Datenformat von Eingangsdaten der Analysemodule vorzugeben. Die Eingangsdaten der Analysemodule umfassen dabei die bereitgestellten Magnetresonanzdaten und/oder die bereitgestellten rekonstruierten Bilddaten und/oder die bereitgestellten weiteren Messinformationen. Hierdurch können die Eingangsdaten auch für unterschiedliche Analysemodule zur Analyse bereitgestellt werden, ohne dass vorher die Eingangsdaten für das jeweilige Analysemodul aufbereitet werden müssen. Dies ermöglicht auch einen einfachen Austausch von Analysemodulen für eine Analyse der bereitgestellten Eingangsdaten.

Alternativ oder zusätzlich kann es vorgesehen sein, dass die Eingangsdaten die bereitgestellten Magnetresonanzdaten, wobei die bereitgestellten Magnetresonanzdaten K-Raum-Daten und/oder Magnetresonanz-Rohdaten umfassen, und/oder rekonstruierte Bilddaten umfassen, wobei die Magnetresonanzdaten und/oder die Bilddaten ein DICOM-Format und/oder ein ISMRMRD-Format umfassen. DICOM (Digital Imaging and Communications in Medicine) umfasst einen offenen Standard zu einem Austausch und/oder einer Speicherung von medizinischen Bilddaten. Diese medizinischen Bilddaten können beispielsweise digitale Bilder, Zusatzinformationen wie Segmentierungen, Oberflächendefinitionen oder Bildregistrierungen umfassen. Mittels des DICOM-Formats ist dabei sowohl das Format zur Speicherung der medizinischen Bilddaten als auch ein Kommunikationsprotokoll zu deren Austausch standardisiert. Das ISMRMRD-Format ist ein gemeinsames MR-Rohdatenformat, das die Zusammenarbeit, die gemeinsame Nutzung und den Datenaustausch von Daten und Datenverarbeitungswerkzeugen erleichtert. Derart werden vorteilhaft einheitliche Datenformate für die bereitgestellten Magnetresonanzdaten und/oder Bilddaten verwendet, die bereits in der Verarbeitung und/oder Analyse von Magnetresonanzdaten und/oder medizinischen Bilddaten Standard sind und damit eine einfache Implementierung der Analysemodule ermöglicht.

Alternativ oder zusätzlich kann es vorgesehen sein, dass die Eingangsdaten die bereitgestellten weiteren Messinformationen umfassen, wobei die bereitgestellten weiteren Messinformationen ein Datenformat umfassen, das abhängig von einer Datenart der bereitgestellten weiteren Messinformationen ist. Die weiteren Messinformationen umfassen bevorzugt physiologische Daten des Patienten und/oder weitere Patientendaten und/oder technische Daten. Die physiologischen Daten können dabei ein Atemsignal und/oder ein EKG-Signal und/oder weitere physiologische Daten umfassen. Die physiologischen Daten umfassen bevorzugt ein ISMRMRD-Format oder weitere öffentliche Community-Standard Datenformate. Zudem sind auch proprietäre Datenformate denkbar. Die weiteren Patientendaten können dabei ein Patientenalter und/oder eine Größe des Patienten und/oder ein Gewicht des Patienten usw. umfassen, wobei die weiteren Patientendaten Daten umfassen, die für die Ausführung des Messprogramms erforderlich und/oder wichtig sind. Die weiteren Patientendaten umfassen bevorzugt Einheiten, wie Meter, KG, Jahre usw. Die technischen Daten können dabei ein Hardware-Parameter umfassen, wie beispielsweise eine Temperatur und/oder Steckkontakte eines Spulensteckers usw. Die technischen Daten umfassen bevorzugt ein ISMRMRD-Format oder weitere öffentliche Community-Standard Datenformate. Zudem sind auch proprietäre Datenformate denkbar. Zudem können die technischen Daten auch eine Temperatureinheit umfassen. Derart kann eine breite Verwendung der bereitgestellten weiteren Messinformationen gesichert werden. Zudem können auch unterschiedliche Analysemodule auf die weitern Patientendaten und damit auf die genormten Datenformate zugreifen, ohne dass vorher die Eingangsdaten für das jeweilige Analysemodul und/oder das Analysemodul auf die Eingangsdaten aufbereitet und/oder angepasst werden muss.

In einer vorteilhaften Weiterbildung des erfindungsgemäßen Verfahrens kann es vorgesehen sein, dass das zumindest eine Analysemodul und/oder das zumindest eine Empfehlungsmodul Ausgangsdaten generiert, wobei die Ausgangsdaten zumindest teilweise ein definiertes Ausgangsdatenformat umfasst. Eine Vorgabe eines definierten Ausgangsdatenformats erfolgt bevorzugt mittels des Konfigurations-Userinterfaces. Die Ausgangsdaten mit dem definierten und/oder spezifischen Ausgangsdatenformat umfassen bevorzugt die Bewertungsinformation und/oder die Statusinformation der Analysemodule. Zudem können die Ausgangsdaten mit dem definierten und/oder spezifischen Ausgangsdatenformat auch die Statusinformation der Empfehlungsmodule umfassen. Derart kann eine einfache Weiterverarbeitung der bereitgestellten Ausgangsdaten ermöglicht werden. Insbesondere können somit unterschiedliche Empfehlungsmodule auf die von einem Analysemodul bereitgestellten Ausgangsdaten zugreifen, ohne dass vorher eine aufwendige Anpassung der bereitstellten Ausgangsdaten und/oder der Empfehlungsmodule erfolgen muss. Zudem kann auch ein Austausch von Modulen vereinfacht werden, da ein Datenformat sowohl von Eingangsdaten als auch von Ausgangsdaten für die einzelnen Module standardisiert ist.

Alternativ oder zusätzlich kann es vorgesehen sein, dass die Ausgangsdaten der Analysemodule definierte Datenklassen umfassen, wobei eine Einteilung der Ausgangsdaten in einer Datenklasse einem semantischen Test der zu klärenden Fragestellung des Analysemoduls umfasst. Derart kann eine einfache Weitergabe der Daten an weitere Module ermöglicht werden.

Die definierten Datenklassen umfassen eine Klassifikation der Ausgangsdaten der Analysemodule. Für eine Klassifikation der Ausgangsdaten der Analysemodule stehen bevorzugt ein "Traffic-light Classifier", ein "Binary Classifier", ein "Multi-Class Classifier" und ein "Multi-Class Multi-Label Classifier" zur Verfügung. Zudem können in einer alternativen Weiterbildung der Erfindung weitere, dem Fachmann als sinnvoll erscheinende Klassifikationsklassen zur Verfügung stehen. Zur Einteilung der Ausgangsdaten in die einzelnen Datenklassen stellt sich zunächst die Ausgangsfrage, ob die klinische Fragestellung und/oder die Analyse des zumindest einen Analysemoduls als binäre Klassifikation von 1 bis N formuliert werden kann. Kann diese Ausgangsfrage mit "Ja" beantwortet werden, so stehen die beiden Klassen "Traffic-light Classifier" und "Binary Classifier" zur Verfügung. Zur weiteren Unterteilung ist zu die Bedingung analysieren, ob zudem die Ausgabeoptionen des Analysemoduls mit "ja" oder "nein" bzw. mit "gut" oder "schlecht" beantwortet werden können. In einem derartigen Fall werden die Ausgangsdaten der "Traffic-light Classifcation" zugeordnet. Ist die Bedingung dagegen nicht erfüllt, werden die Ausgangsdaten er "Binary Classifcation" zugeordnet.

Ist die Antwort auf die Ausgangsfrage dagegen ein "nein", so stehen die beiden Klassen "Multi-Class Classifier" und "Multi-Class Multi-Label Classifier" zur Verfügung. Bei Mehrklassen-Classifier können die Daten in drei oder mehr Kategorien unterteilt werden. Umfasst jede Instanz und/oder Kategorie genau einen definierten Wert, werden die Ausgangsdaten dem "Multi-Class Classifier" zugeordnet. Sind dagegen für jede Instanz und/oder Kategorie mehrere Aussagen oder Werte möglich, werden die Ausgangsdaten dem "Multi-Class Multi-Label Classifier" zugeordnet.

Als Ausgangsdaten stehen bei einer "Traffic-light-classifcation" oder einer "Binary classification" die Werte "0", "1" und "-1" zur Verfügung. Der Wert "0" zeigt an, dass keine Auffälligkeiten oder Probleme mittels des Analysemoduls erkannt wurden und das Messprogramm wie geplant fortgesetzt werden kann. Insbesondere sind keine Aktionen vom Benutzer zur Behebung einer kritischen Situation erforderlich. Der Wert "1" zeigt an, dass kritische Zustände oder Problem oder Auffälligkeiten mittels des Analysemoduls erkannt wurden. Häufig ist damit auch eine Interaktion seitens des Benutzer zur Behebung des erkannten kritischen Zustandes oder des erkannten Problems oder der erkannte Auffälligkeit erforderlich. Der Wert "-1" zeigt an, dass kein eindeutiges Ergebnis der Analyse vorliegt. Ein positives oder negatives Ergebnis konnte nicht mit hinreichender Sicherheit nachgewiesen werden. Häufig wird der Benutzer auf ein mögliches Problem oder eine Auffälligkeit aufmerksam gemacht und empfohlen, die Daten manuell zu überprüfen und eine eigene Entscheidung zu treffen.

Als Ausgangsdaten stehen bei einer "Multi-Class Classifier" die Werte "(1, 0, 0,..., 0)", "(0, 1, 0,..., 0)" bis "(0, 0, 0,..., 1)" und "-1" zur Verfügung. Die Werte "(1, 0, 0,..., 0)", "(0, 1, 0,..., 0)" bis "(0, 0, 0,..., 1)" zeigen an, dass jeweils ein bestimmter Fall mittels des Analysemoduls erkannt wurde. Der Wert "-1" zeigt an, dass kein eindeutiges Ergebnis der Analyse vorliegt.

Als Ausgangsdaten stehen bei einer "Multi-Class Multi-Label Classifier" die Werte "(1, 0, 0,..., 0)", "(1, 1, 0,..., 0)" bis "(1, 1, 1,..., 1)" und "-1" zur Verfügung. Die Werte "(1, 0, 0,..., 0)", "(1, 1, 0,..., 0)" bis "(1, 1, 1,..., 1)" zeigen an, dass jeweils ein bestimmter Fall mittels des Analysemoduls erkannt wurde. Der Wert "-1" zeigt an, dass kein eindeutiges Ergebnis der Analyse vorliegt.

Des Weiteren geht die Erfindung aus von einem Assistenzsystem, das zu einer Durchführung des Verfahrens zu einem Unterstützen und/oder Assistieren eines Benutzers bei einem Ausführen eines Messprogramms während einer Magnetresonanzdatenerfassung ausgebildet ist, wobei das Analysemodul einen Transaktionsmanager und zumindest ein Analysemodul aufweist. Vorzugsweise ist der Transaktionsmanager zu einem Datenaustausch des zumindest einen Analysemoduls mit weiteren Einheiten und/oder Modulen ausgebildet. Insbesondere ist der Transaktionsmanager zu einem Bereitstellung von Eingangsdaten, insbesondere von Magnetresonanzdaten und/oder Bilddaten und/oder weiteren Messinformationen, für das zumindest eine Analysemodul ausgebildet. Insbesondere ist der Transaktionsmanager auch zu einem Bereitstellen der Ausgangsdaten des zumindest einen Analysemoduls für weitere Einheiten und/oder Module ausgebildet. Dabei können die Ausgangsdaten einer Benutzerschnittstelle zu einer Ausgabe an einen Benutzer zur Verfügung gestellt werden. Zudem können die Ausgangsdaten auch für eine Weiterverarbeitung weitern Modulen des Assistenzsystems zur Verfügung gestellt werden. Die Ausgangsdaten können dabei auch einem Hinweis, insbesondere eine Assistenzinformation, für ein mittels des zumindest einen Analysemoduls erkanntes Problem und/oder für einen mittels des zumindest einen Analysemoduls erkannten kritischen Zustand und/oder für eine mittels des zumindest einen Analysemoduls erkannte Auffälligkeit an den Benutzer umfassen.

Das zumindest eine Analysemodul ist zu einer Analyse von Eingangsdaten, insbesondere der bereitgestellten Magnetresonanzdaten und/oder der bereitgestellten rekonstruierten Bilddaten und/oder der bereitgestellten weiteren Messinformationen, ausgebildet. Dabei ist das zumindest eine Analysemodul dazu ausgebildet, die Eingangsdaten nach Auffälligkeiten und/oder Unregelmäßigkeiten zu analysieren.

Mittels des Assistenzsystems kann ein Benutzer während einer Magnetresonanzuntersuchung, insbesondere eines Ausführens des Messprogramms, direkt auf Auffälligkeiten und/oder Probleme und/oder kritische Zustände bei der Magnetresonanzdatenerfassung aufmerksam gemacht und/oder hingewiesen werden kann. Damit erhält der Benutzer direkt ein Feedback und kann aufgrund der Information, insbesondere der Assistenzinformation, entscheiden, ob er Maßnahmen zur Behebung der Fehler oder Probleme ausführen möchte bzw. das Auftreten von wahrscheinlichen Problemen verhindern möchte, beispielsweise einzelne Messschritte des Messprogramms wiederholen oder Aufnahmeparameter ändern möchte. Beispielsweise kann der Benutzer auch eine Atemanhaltedauer für den Patienten anpassen, wenn dieser Probleme hat, das Atmen lange anzuhalten. Des Weiteren kann der Benutzer auch eine alternative Messsequenz für den Messschritt auswählen. Zudem kann der Benutzer auch entscheiden, dass zwar eine Qualität der Bilddaten vorliegt, die mängelbehaftet ist, für eine Beurteilung der klinischen oder diagnostischen Fragestellung jedoch ausreichend ist. Ferner kann der Benutzer aufgrund der bereitgestellten Informationen des Assistenzsystems auch entscheiden, ob er weitere Maßnahmen zur Klärung einer erkannten Auffälligkeit ergreifen möchte, wie beispielsweise zusätzlich Messschritte einfügen, deren Fokus auf dem Bereich mit der erkannten Auffälligkeit gerichtet ist.

Durch das direkte Informieren des Benutzers, insbesondere durch die Ausgabe der Assistenzinformation während des Ablaufs des Messprogramms, kann zudem ein Aufwand, insbesondere ein Untersuchungsaufwand, für den Patienten als auch Benutzer reduziert werden. Sind beispielsweise die erfassten Magnetresonanzdaten und/oder die aus den Magnetresonanzdaten rekonstruierten Bilddaten für eine diagnostische Bewertung ungeeignet, kann unmittelbar der Messschritt oder mehrere Messschritte wiederholt und/oder mit Änderungen ausgeführt werden. Eine Wiederholung erst nach Tagen oder Wochen, wenn die Magnetresonanzdaten und/oder Bilddaten analysiert sind und eine Wiederholungstermin für den Patienten gefunden wurde, kann dabei verhindert werden. Insbesondere müssen beispielsweise nur die relevanten, insbesondere die mangelbehafteten und/oder fehlerbehaften, Messschritte wiederholt werden und nicht das ganze Messprogramm.

Die Vorteile des erfindungsgemäßen Assistenzsystems entsprechen im Wesentlichen den Vorteilen des Verfahrens zu einem Unterstützen und/oder Assistieren eines Benutzers bei einem Ausführen eines Messprogramms während einer Magnetresonanzdatenerfassung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Assistenzsystems kann es vorgesehen sein, dass das Assistenzsystem zumindest ein Empfehlungsmodul umfasst. Das zumindest ein Empfehlungsmodul ist dazu ausgebildet, anhand von Eingangsdaten einen Vorschlag für den weiteren Ablauf des Messprogramms zu ermitteln. Die Eingangsdaten des zumindest einen Empfehlungsmoduls umfassen bevorzugt die von dem zumindest einen Analysemodul ermittelten Ausgangsdaten, die mittels des Transaktionsmanagers dem zumindest einen Empfehlungsmodul bereitgestellt werden. Bevorzugt wird der von dem zumindest einen Empfehlungsmodul ermittelte Vorschlag für eine Ausgabe an einen Benutzer einer Benutzerschnittstelle bereitgestellt, wobei das Bereitstellen des ermittelten Vorschlags mittels des Transaktionsmanagers erfolgt.

Diese Ausgestaltung der Erfindung weist den Vorteil auf, dass während eines Ausführens des Messprogramms einem Benutzer direkt ein Vorschlag zur Verbesserung und/oder zur Abhilfe eines von einem Analysemodul erkannten Problems und/oder eines von einem Analysemodul erkannten kritischen Zustands und/oder eine von einem Analysemodul erkannte Auffälligkeit bereitgestellt werden kann. Somit wird der Benutzer nicht nur auf ein Problem und/oder einen kritischen Zustand und/oder eine Auffälligkeit aufmerksam gemacht, sondern erhält auch gleich einen Vorschlag zur Behebung des Problems und/oder des kritischen Zustands und/oder zur Klärung der Auffälligkeit. Dies ermöglicht es insbesondere unerfahrenen Benutzern auch bei Problempatienten das Messprogramm erfolgreich abzuschließen und für eine Diagnose relevante Bilddaten bereitzustellen.

In einer vorteilhaften Weiterbildung des erfindungsgemäßen Assistenzsystems kann es vorgesehen sein, dass der Transaktionsmanager eine Kommunikationsschnittstelle zu einem Datenaustausch des zumindest eine Analysemoduls und/oder des zumindest einen Empfehlungsmoduls umfasst. Derart kann eine einfache Kommunikation zwischen den einzelnen Modulen, insbesondere zwischen den Analysemodulen und/oder den Empfehlungsmodulen, des Assistenzsystems ermöglicht werden.

In einer vorteilhaften Weiterbildung des erfindungsgemäßen Assistenzsystems kann es vorgesehen sein, dass das Assistenzsystem ein Konfigurations-Userinterface umfasst, wobei das Konfigurations-Userinterface dazu ausgebildet ist, eine Auswahl an Analysemodulen und/oder an Empfehlungsmodulen für ein ausgewähltes Messprogramm bereitzustellen. Vorzugsweise erfolgt ein Bereitstellen einer Auswahl an Analysemodulen und/oder an Empfehlungsmodulen für zumindest einen Messschritt des ausgewählten Messprogramms. Insbesondere stellt das Konfigurations-Userinterface dabei eine Auswahl an Analysemodulen und/oder Empfehlungsmodulen zur Verfügung, die auf das Messprogramm, insbesondere auf den zumindest einen Messschritt des Messprogramms abgestimmt sind. Dabei kann das Konfigurations-Userinterface dazu ausgebildet sein, dass bei einer Auswahl eines Moduls, insbesondere eines Analysemoduls und/oder eines Empfehlungsmoduls, automatisch dem Benutzer weitere, mit dem ausgewählten Modul, insbesondere mit den von dem ausgewählten Modul bereitgestellten Ausgangsdaten oder erforderlichen Eingangsdaten, kompatible weitere Module empfohlen werden, die zudem in einem untersuchungsrelevanten und/oder messprogrammrelevanten Kontext übereinstimmen. Zudem können derartige weitere Module automatisch von dem Konfigurations-Userinterface mit aktiviert und/oder ausgewählt werden, wenn beispielsweise eine Funktionsweise des ausgewählten Moduls dies erfordert.

Hierdurch kann für einen Benutzer eine einfache und schnelle Auswahl eines Moduls, insbesondere zumindest eines Analysemoduls und/oder zumindest eines Empfehlungsmoduls erfolgen. Insbesondere ermöglicht diese Ausgestaltung der Erfindung auch ungeübten und/oder unerfahrenen Benutzern eine einfache Verwendung des Analysemoduls während eines Ausführens eines Messprogramms. Zudem kann derart auch sichergestellt werden, dass ein Datensatz, insbesondere ein Bilddatensatz, nach Beenden des Messprogramms zur Verfügung steht, aus dem eine Diagnose erstellt werden kann.

In einer vorteilhaften Weiterbildung des erfindungsgemäßen Assistenzsystems kann es vorgesehen sein, dass das Konfigurations-Userinterface zumindest teilweise in eine Benutzeroberfläche zur Steuerung und/oder Überwachung des Messprogramms integriert ist. Dabei kann eine Auswahl zumindest eines Moduls für zumindest einen Messschritt über die Benutzeroberfläche erfolgen, wobei das Konfigurations-Userinterface über die Benutzeroberfläche aufrufbar und/oder anklickbar ist und eine Art der Unterstützung konfigurierbar ist. Zudem können für die einzelnen Messschritte auch die für den Messschritt aktivierten und/oder ausgewählten Module des Assistenzsystems, insbesondere die Analysemodule und/oder die Empfehlungsmodule, angezeigt werden. Derart kann eine einfache und schnelle Übermittlung von Informationen des Assistenzsystems an den Benutzer erfolgen. Insbesondere kann sich hierbei der Benutzer auf eine einzige Benutzeroberfläche konzentrieren und erhält alle für das Messprogramm zur Verfügung stehenden Informationen.

In einer vorteilhaften Weiterbildung des erfindungsgemäßen Assistenzsystems kann es vorgesehen sein, dass das Konfigurations-Userinterface ein Datenformat für Eingangsdaten und/oder Ausgangsdaten des zumindest einen Analysemoduls und/oder des zumindest einen Empfehlungsmoduls vorgibt. Durch ein einheitliches Datenformat können unterschiedliche Module, insbesondere Analysemodule und/oder Empfehlungsmodule auf die bereitgestellten Daten zugreifen, ohne dass vorher die Daten, insbesondere Eingangsdaten, für das jeweilige Modul aufbereitet werden müssen. Dies ermöglicht einen einfachen Austausch von Modulen, insbesondere von Analysemodulen und/oder Empfehlungsmodulen, innerhalb des Assistenzsystems.

In einer vorteilhaften Weiterbildung des erfindungsgemäßen Assistenzsystems kann es vorgesehen sein, dass das Konfigurations-Userinterface zusammen mit dem Transaktionsmanager ein Framework des Assistenzsystems umfassen, wobei die einzelnen Analysemodule und/oder die einzelnen Empfehlungsmodule innerhalb des Frameworks austauschbar sind. Insbesondere sind die einzelnen Analysemodule und/oder die einzelnen Empfehlungsmodule durch die Vorgabe von einheitlichen Datenformaten für die Eingangsdaten und/oder Ausgangsdaten der Module, insbesondere der Analysemodule und/oder der Empfehlungsmodule in dem Framework austauschbar. Derart kann eine besonders einfache Integration von weiteren Modulen, insbesondere Analysemodulen und/oder Empfehlungsmodulen, erfolgen. Insbesondere kann derart auch eine zur Auswahl stehende Anzahl an Modulen variiert und/oder erweitert werden.

In einer vorteilhaften Weiterbildung des erfindungsgemäßen Assistenzsystems kann es vorgesehen sein, dass das Assistenzsystem eine Benutzeroberfläche zur Ausgabe von Informationen, insbesondere der Assistenzinformation und/oder der erweiterten Assistenzinformation, umfasst. Die Benutzeroberfläche kann dabei ein Assistenz-Userinterface umfassen, das neben der Assistenzinformation den Benutzer auch darüber informieren kann, welches Analysemodul gerade ausgeführt wird und für welchen Messschritt dieses ausgewählt wurde. Derart kann eine einfache und schnelle Übermittlung der Assistenzinformation an den Benutzer erfolgen. Alternativ hierzu kann die Benutzeroberfläche zur Ausgabe von Informationen auch in eine Benutzeroberfläche zur Steuerung und/oder Überwachung des Messprogramms integriert sein. Die Benutzeroberfläche zur Steuerung und/oder Überwachung des Messprogramms zeigt dabei bevorzugt die einzelnen Messschritte des Messprogramms an. Dabei können für die einzelnen Messschritte auch die für den Messschritt aktivierten und/oder ausgewählten Analysemodule angezeigt werden. Bevorzugt wird die Assistenzinformation für den jeweiligen Messschritt in der Benutzeroberfläche angezeigt. Insbesondere kann sich hierbei der Benutzer auf eine einzige Benutzeroberfläche konzentrieren und erhält alle für das Messprogramm zur Verfügung stehenden Informationen inklusive der Assistenzinformationen.

Des Weiteren geht die Erfindung aus von einer Magnetresonanzvorrichtung mit einem Assistenzsystem, wobei das Assistenzsystem zu einer Durchführung des Verfahrens zu einem Unterstützen und/oder Assistieren eines Benutzers bei einem Ausführen eines Messprogramms während einer Magnetresonanzdatenerfassung ausgebildet ist.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten und/oder Objekts ausgelegt und/oder ausgebildet ist. Die Magnetresonanzvorrichtung umfasst bevorzugt eine Magneteinheit zur Erfassung der medizinischen und/oder diagnostischen Magnetresonanzdaten. Hierbei umfasst die Magneteinheit einen Grundmagneten, eine Gradientenspuleneinheit und eine Hochfrequenzantenneneinheit. Die Hochfrequenzantenneneinheit ist hierbei fest innerhalb der Magneteinheit angeordnet und/oder installiert. Die Magneteinheit umgibt dabei einen Patientenaufnahmebereich der Magnetresonanzvorrichtung. Der Patientenaufnahmebereich ist bevorzugt zylinderförmig ausgebildet und zu einer Aufnahme des Patienten, insbesondere des zu untersuchenden Bereichs des Patienten, für eine Magnetresonanzuntersuchung ausgebildet.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das Gradienten-System ist zu einer Erzeugung von Magnetfeldgradienten, die für eine Ortskodierung während einer Bildgebung verwendet werden, ausgebildet. Die Hochfrequenzantenneneinheit ist zu einer Aussendung von Hochfrequenzpulsen und/oder Anregungspulsen zur Generierung von Magnetresonanzsignalen ausgebildet.

Für eine Magnetresonanzuntersuchung wird der Patient, insbesondere der zu untersuchende Bereich des Patienten, innerhalb des Patientenaufnahmebereichs der Magnetresonanzvorrichtung positioniert. Innerhalb des Patientenaufnahmebereichs ist bevorzugt das Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Das Assistenzsystem ist bevorzugt von der Magnetresonanzvorrichtung umfasst. Dabei kann das Assistenzsystem als Ganzes oder zumindest teilweise, insbesondere einzelne Module des Assistenzsystems, auf einem separaten Server, insbesondere zur Systemsteuereinheit separaten Rechner, ausgeführt werden. Zudem ist es auch denkbar, dass das Assistenzsystem als Ganzes oder zumindest teilweise, insbesondere einzelne Module des Assistenzsystems, in einer Cloud und/oder einem Edge-Device ausgeführt werden.

Die Vorteile der erfindungsgemäßen Magnetresonanzvorrichtung entsprechen im Wesentlichen den Vorteilen des Verfahrens zu einem Unterstützen und/oder Assistieren eines Benutzers mittels eines Assistenzsystems bei einem Ausführen eines Messprogramms während einer Magnetresonanzdatenerfassung, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Steuerungseinheit ladbar ist, mit Programmmitteln, um ein Verfahren zu einem Unterstützen und/oder Assistieren eines Benutzers mittels eines Assistenzsystems bei einem Ausführen eines Messprogramms während einer Magnetresonanzdatenerfassung auszuführen, wenn das Programm in der Steuerungseinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: ein erstes Ausführungsbeispiel eines erfindungsgemäßes Verfahrens zu einem Unterstützen und/oder Assistieren eines Benutzers mittels eines Assistenzsystems bei einem Ausführen eines Messprogramms während einer Magnetresonanzdatenerfassung,
- Fig. 2: ein erstes Ausführungsbeispiel des Assistenzsystems,
- Fig. 3: ein zweites Ausführungsbeispiel des Verfahrens,
- Fig. 4: ein zweites Ausführungsbeispiel des Assistenzsystems,
- Fig. 5: ein weiteres Ausführungsbeispiel des Assistenzsystems,
- Fig. 6: ein weiteres Ausführungsbeispiel des Assistenzsystems,
- Fig. 7: ein weiteres Ausführungsbeispiel des Assistenzsystems,
- Fig. 8: ein weiteres Ausführungsbeispiel des Assistenzsystems,
- Fig. 9: das Assistenzsystem in einem serieller Ausführungsmodus,
- Fig. 10: das Assistenzsystem in einem Quasi-Echtzeitmodus,
- Fig. 11: das Assistenzsystem in einem Echtzeitmodus,
- Fig. 12: ein Beispiel für Ausgangsdaten eines Analysemoduls und/oder eines Empfehlungsmoduls,
- Fig. 13: eine Benutzeroberfläche zur Auswahl eines Moduls des Assistenzsystems für einen Messschritt,
- Fig. 14: eine Benutzeroberfläche zur Anzeige eine Assistenzinformation und/oder eines Vorschlags während eines Ausführens des Messschritts,
- Fig. 15: eine erfindungsgemäße Magnetresonanzvorrichtung mit einem Assistenzsystem in einer schematischen Darstellung.

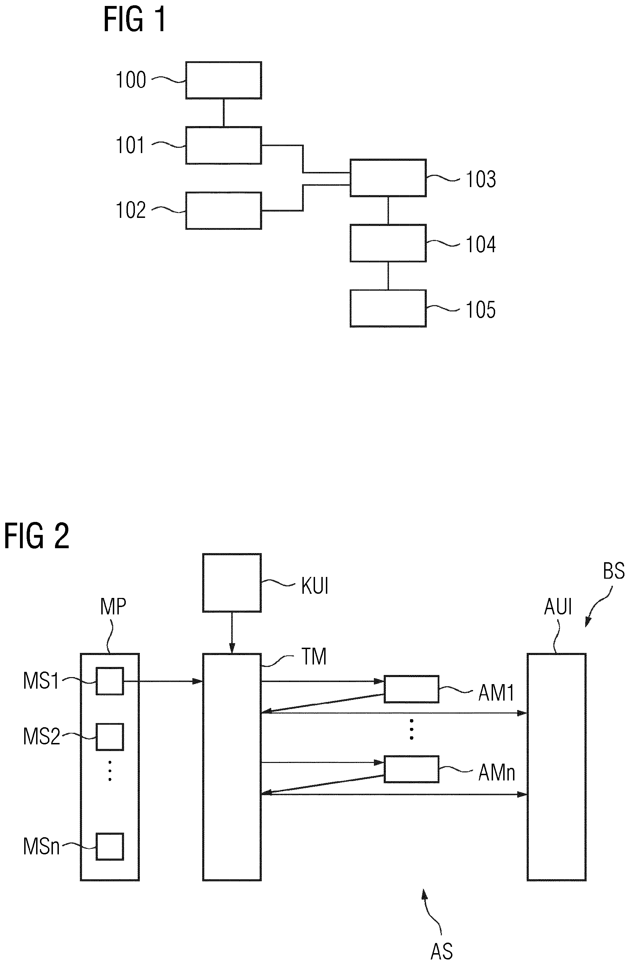

In Fig. 1 ist ein erstes Ausführungsbeispiel eines erfindungsgemäßen computerimplementierten Verfahrens zu einem Unterstützen und/oder Assistieren eines Benutzers mittels eines Assistenzsystems AS bei einem Ausführen eines Messprogramms MP während einer Magnetresonanzdatenerfassung dargestellt. Zur Ausführung des Verfahrens weist das Assistenzsystem AS einen Transaktionsmanager TM und zumindest ein Analysemodul AM auf, wobei zur Ausbildung des Assistenzsystems AS auf Fig. 2 verwiesen wird. Bevorzugt umfasst das Assistenzsystem AS mehrere Analysemodule AM, die zu einer Analyse von unterschiedlichen Daten bezüglich unterschiedlicher Fragestellungen ausgebildet sind.

Der Transaktionsmanager TM ist insbesondere zu einem Datenaustausch des zumindest einen Analysemoduls AM ausgebildet. Dabei ist der Transaktionsmanager TM zu einem Bereitstellen von Eingangsdaten für das zumindest eine Analysemodul AM ausgebildet. Zudem ist der Transaktionsmanager TM auch zu einem Bereitstellen von Ausgangsdaten des zumindest einen Analysemoduls AM, insbesondere für weitere Module des Assistenzsystems AS und/oder weitere Einheiten, wie beispielsweise eine Ausgabeeinheit und/oder eine Darstellungseinheit einer Benutzerschnittstelle BS, ausgebildet.

Das Assistenzsystem AS umfasst zudem ein Konfigurations-Userinterface KUI, das über die Benutzerschnittstelle mit einem Benutzer kommunizieren kann. Über das Konfigurations-Userinterface KUI ist eine Auswahl und/oder eine Konfigurierung zumindest eines Analysemoduls AM durch einen Benutzer vorgesehen. Der Transaktionsmanager TM bildet zusammen mit dem Konfigurations-Userinterface KUI dabei einen Rahmen und/oder ein Framework des Assistenzsystems AS, innerhalb dessen die einzelnen Module, insbesondere Analysemodule AM, agieren. Mit anderen Worten legt das Framework, insbesondere das Konfigurations-Userinterface KUI, dabei auch ein Format von Eingangsdaten und/oder Ausgangsdaten der einzelnen Module des Assistenzsystems AS fest und legt somit auch ein Datenformat für eine Kommunikation der einzelnen Module fest.

Zur Steuerung des Verfahrens zu einem Unterstützen und/oder Assistieren eines Benutzers mittels des Assistenzsystems AS bei einem Ausführen eines Messprogramms MP während einer Magnetresonanzdatenerfassung weist das Assistenzsystem AS eine Steuereinheit und/oder Recheneinheit mit einer entsprechenden Software und/oder entsprechenden Programmen auf, um das Verfahren zu einem Unterstützen und/oder Assistieren eines Benutzers mittels des Assistenzsystems AS bei einem Ausführen eines Messprogramms MP während einer Magnetresonanzdatenerfassung zu steuern.

In einem ersten Verfahrensschritt 100 des erfindungsgemäßen Verfahrens erfolgt ein Auswählen eines Messprogramms MP zu einem Erfassen von Magnetresonanzdaten mit einer definierten diagnostischen Fragestellung. Zusammen mit dem Messprogramm MP kann der Benutzer auch eine Unterstützung mittels des Assistenzsystems AS und damit zumindest ein Analysemodul AM des Assistenzsystems AM, das zu einer Analyse von Daten während eines Ausführens des Messprogramms MP ausgebildet ist, auswählen. Dabei kann durch Auswahl des Messprogramms MP bereits auf die klinische und/oder diagnostische Fragestellung des Messprogramms MP abgestimmte und/oder zugeordnete Auswahl an Analysemodulen AM dem Benutzer zur Auswahl angeboten werden. Das Messprogramm MP umfasst dabei mehrere Messschritte MS1, MS2, ..., MSn, wobei für zumindest einen Messschritt MSi eine Unterstützung mittels des Assistenzsystems AS und damit zumindest ein Analysemodul AM des Assistenzsystems AS auswählbar ist. Zudem kann auch für jeden der mehreren Messschritte MS1, MS2, ..., MSn des Messprogramms MP eine Unterstützung mittels des Assistenzsystems AS und damit zumindest ein Analysemodul AM des Assistenzsystems AS auswählbar sein. Dabei können auch unterschiedliche Analysemodule AM1, AM2, ..., AMn für unterschiedliche Messschritte MS1, MS2, ..., MSn des Messprogramms MP zur Verfügung stehen und/oder dem Benutzer zu Auswahl angeboten werden. Das Auswählen des Messprogramms MP und auch das Auswählen des zumindest einen Analysemoduls AM erfolgt durch einen Benutzer über das Konfigurations-Userinterface KUI an der Benutzerschnittstelle BS. Die Benutzerschnittstelle BS kann dabei von einer Magnetresonanzvorrichtung umfasst sein.

Anschließend erfolgt in einem zweiten Verfahrensschritt 101 ein Ausführen des ausgewählten Messprogramms MP, wobei das Ausführen des Messprogramms MP ein Erfassen von Magnetresonanzdaten umfasst. Während des Erfassens der Magnetresonanzdaten werden in diesem zweiten Verfahrensschritt 101 aus den Magnetresonanzdaten auch direkt Bilddaten rekonstruiert. Das Rekonstruieren der Bilddaten erfolgt über eine Rekonstruktionseinheit. Die Rekonstruktionseinheit kann dabei vom Messprogramm MP umfasst sein oder eine separate Einheit zum Messprogramm MP bilden. Zudem werden in diesem zweiten Verfahrensschritt 101 die Magnetresonanzdaten und die rekonstruierten Bilddaten bereitgestellt. Dabei werden die Magnetresonanzdaten in Form von K-Raum-Daten und/oder Magnetresonanz-Rohdaten bereitgestellt. Das Bereitstellen der Magnetresonanzdaten und/oder der rekonstruierten Bilddaten erfolgt dabei mittels des Transaktionsmanagers RM des Assistenzsystems AS. Die bereitgestellten Magnetresonanzdaten und/oder bereitgestellten die rekonstruierten Bilddaten werden als Eingangsdaten für die Analysemodule AM1, AM2, ..., AMn bereitgestellt, wobei ein Datenformat der bereitgestellten Magnetresonanzdaten und/oder der bereitgestellten rekonstruierten Bilddaten ein DICOM-Format und/oder ein ISMRMRD-Format umfassen.

In einem weiteren dritten Verfahrensschritt 102 werden zudem weitere Messinformationen erfasst und bereitgestellt. Die weiteren Messinformationen können dabei zusätzliche Daten, die während des Ausführens des Messprogramms MP und damit zeitgleich mit dem Ausführen des Messprogramms MP vom Patienten und/oder einer die Magnetresonanzmessung ausführenden Hardware erfasst werden. Diese weiteren Messinformationen können beispielsweise physiologische Daten des Patienten, insbesondere ein Atemsignal und/oder ein EKG-Signal und/oder weitere physiologische Daten umfassen. Zudem können die weiteren Messinformationen auch Daten und/oder Parameter einer zu überwachende Hardwarekonfiguration von beispielsweise einer lokalen Hochfrequenzspule usw. umfassen. Zudem können die weiteren Messinformationen auch weitere Patientendaten, die bereits bei einer Registrierung des Patienten für eine Magnetresonanzuntersuchung und damit vor dem Ausführen des Messprogramms MP erfasst werden, umfassen. Derartige weitere Patientendaten können beispielweise ein Alter und/oder ein Gewicht und/oder eine Größe des Patienten usw. umfassen.

In einem weiteren vierten Verfahrensschritt 103 erfolgt ein Ermitteln zumindest einer Bewertungsinformation mittels des zumindest einen Analysemoduls AM des Assistenzsystems AS in Abhängigkeit von Eingangsdaten, die dem zumindest einem Analysemodul AS mittels des Transaktionsmanagers TM bereitgestellt werden. Die Eingangsdaten des zumindest einen Analysemoduls AM umfassen dabei die bereitgestellten Magnetresonanzdaten und/oder die bereitgestellten rekonstruierten Bilddaten und/oder die bereitgestellten weiteren Messinformation. Insbesondere erfolgt das Ermitteln der zumindest einen Bewertungsinformation in Abhängigkeit von den Magnetresonanzdaten und/oder den rekonstruierten Bilddaten und/oder der weiteren Messinformation. Dabei ist mittels des Konfigurations-Userinterfaces KUI ein Datenformat der Eingangsdaten des zumindest einen Analysemoduls AM vorgegeben. Die bereitgestellten Magnetresonanzdaten und/oder die rekonstruierten Bilddaten umfassen ein DICOM-Format und/oder ein ISMRMRD-Format.

Die bereitgestellten weiteren Messinformationen umfassen ein Datenformat, das abhängig von einer Datenart der bereitgestellten weiteren Messinformationen ist. Umfassen die weiteren Messinformationen physiologischen Daten, dann umfassen die weiteren Messinformationen bevorzugt ein ISMRMRD-Format oder weitere öffentliche Community-Standard Datenformate und/oder auch proprietäre Datenformate. Umfassen die weiteren Messinformationen weitere Patientendaten, wie beispielsweise ein Patientenalter und/oder eine Größe des Patienten und/oder ein Gewicht des Patienten usw., dann umfassen die weiteren Messinformationen Daten, die bevorzugt in Einheiten wie Meter, KG, Jahre usw. angegeben werden.

Das zumindest eine Analysemodul AM umfasst dabei das zusammen mit dem Messprogramm MP ausgewählte Analysemodul AM. Zur Analyse der bereitgestellten Magnetresonanzdaten und/oder der bereitgestellten rekonstruierten Bilddaten und/oder der bereitgestellten weiteren Messinformationen und zur Ermittlung der zumindest einen Bewertungsinformation weist das zumindest eine Analysemodul AM einen regelbasierten Algorithmus und/oder einen auf einem maschinellen Lernen basierten Algorithmus auf. Einem regelbasierter Algorithmus liegen definierte Regeln zugrunde, nach denen dieser Algorithmus die ihm gestellte Aufgabe durchführt. Zur Lösung der Aufgabe kann ein Ergebnis mit zumindest einem Schwellwert verglichen werden und daraus eine Aussage, insbesondere die Bewertungsinformation, abgeleitet werden.

Ein auf einem maschinellen Lernen basierter Algorithmus umfasst bevorzugt ein trainiertes maschinelles Lernverfahren und wurde bereit dazu trainiert, bestimmte Merkmale und/oder ein bestimmtes Muster in den zu analysierenden Eingangsdaten zu erkennen. Im Allgemeinen ahmt ein trainiertes maschinelles Lernverfahren kognitive Funktionen nach, die Menschen mit anderen menschlichen Gedanken assoziieren. Insbesondere durch das Training auf Basis von Trainingsdaten ist das maschinelle Lernverfahren in der Lage, sich an neue Gegebenheiten anzupassen und Muster zu erkennen und zu extrapolieren.

Vorzugsweise wurde der auf einem maschinellen Lernen basierter Algorithmus dazu trainiert, bestimmte Merkmale und/oder ein bestimmtes Muster in den zu analysierenden Eingangsdaten im Hinblick auf die zu klärende Fragestellung zu erkennen.

Für das Training werden bevorzugt Trainingsdatensätze verwendet, deren Eingangsdaten, insbesondere Magnetresonanzdaten, beispielsweise K-Raum-Daten und/oder Magnetresonanz-Rohdaten, und/oder rekonstruierte Bilddaten und/oder weitere Messinformationen, bereits hinsichtlich einer definierten klinischen Fragestellung bewertet wurden. Bevorzugt liegen hierbei Trainingsdatensätze von unterschiedlichen Trainingspatienten vor.

Die Analyse der Eingangsdaten mittels eines Analysemoduls AM erfolgt bevorzugt kontinuierlich während des gesamten Messschritts MSi oder der gesamten Messschritte MS1, MS2, ..., MSn, für das oder für die das Analysemodul AM ausgewählt wurde. Zudem erfolgt auch das Bereitstellen der Bewertungsinformation bevorzugt kontinuierlich während des gesamten Messschritts MSi oder der gesamten Messschritte MS1, MS2, ..., MSn, für das oder für die das Analysemodul AM ausgewählt wurde.

Das zumindest eine Analysemodul AM kann dabei zumindest ein Qualitäts-Analysemodul und/oder zumindest ein klinisches Analysemodul und/oder zumindest ein technisches Analysemodul und/oder zumindest ein allgemeines Analysemodul umfassen.

Das zumindest eine Qualitäts-Analysemodul ist das ausgebildet, bestimmte Merkmale in Eingangsdaten zu erkennen, wobei die bestimmten Merkmale sich auf bestimmte oder spezifische Datenqualitätsprobleme beziehen. Bevorzugt ist das zumindest eine Qualitäts-Analysemodul QAM dazu ausgebildet, die Eingangsdaten kontinuierlich hinsichtlich des bestimmten oder spezifischen Datenqualitätsproblems zu überprüfen und zu analysieren. Ein Vorhandensein eines Datenqualitätsproblems kann beispielsweise eine Bildqualität in den rekonstruierten Bilddaten derart beeinträchtigen, dass eine diagnostische Auswertung der Bilddaten nicht mehr möglich ist oder auch die Gefahr eine Fehldiagnose aufgrund der mangelnden Bildqualität gegeben ist. Zudem kann das zumindest eine Qualitäts-Analysemodul auch weitere Messinformationen, beispielsweise physiologische Daten des Patienten, während des Ausführens des Messprogramms MP überwachen und analysieren. Derartige physiologische Daten können beispielsweise EKG-Daten des Patienten sein, die während des Ausführens des Messprogramms MP vom Patienten erfasst werden. Zudem können derartige physiologische Daten auch Daten aus einer Überwachung einer Atmung des Patienten und/oder weitere, dem Fachmann als sinnvoll erscheinender physiologischer Daten umfassen. Das zumindest eine Qualitäts-Analysemodul kann dabei beispielsweise eine Wahrscheinlichkeit bestimmen, mit der ein Problem und/oder ein kritischer Zustand und/oder eine Auffälligkeit in den Bilddaten auftreten kann, wenn keine Gegenmaßnahmen ergriffen werden. Das Ergebnis der Analyse des zumindest einen Qualitäts-Analysemoduls umfasst die zumindest eine Bewertungsinformation. Dabei kann die Bewertungsinformation auch ein Maß für die Wahrscheinlichkeit eines auftretenden Problems und/oder einer Auffälligkeit umfassen.

Das zumindest eine klinische Analysemodul ist bevorzugt dazu ausgebildet die Eingangsdaten, insbesondere die rekonstruierten Bilddaten, auf Auffälligkeiten zu analysieren. Derartige Auffälligkeiten umfassen bevorzugt eine Abweichung von einer Norm. Dabei kann die Auffälligkeitsinformation eine neutrale Information umfassen, die auf beispielsweise eine Anomalie und eine Abnormität während der Ausführung zumindest einen Messschritts des ausgewählten Messprogramms hinweist. Derartige Auffälligkeiten können zudem auch einen Verdacht auf eine Krankheit, beispielsweise einen Verdacht auf eine Blutung und/oder einen Verdacht auf einen Infarkt, umfassen. Anhand der Eingangsdaten des Analysemoduls AM, insbesondere der rekonstruierten Bilddaten, kann eine Wahrscheinlichkeit für ein Vorliegen einer Auffälligkeit in den rekonstruierten Bilddaten von dem zumindest einen technischen Analysemodul ermittelt werden. Überschreitet die ermittelte Wahrscheinlichkeit einen Grenzwert, wird dies von dem zumindest einen klinischen Analysemodul als Auffälligkeit und/oder Anomalie gewertet und/oder erkannt, was sich in der Bewertungsinformation von dem zumindest einen klinischen Analysemodul AM widerspiegelt. Jedoch erfolgt mittels des klinischen Analysemoduls keine Diagnose der Eingangsdaten, insbesondere der rekonstruierten Bilddaten, sondern stellt nur eine Hilfestellung für eine Diagnose durch einen Arzt dar. Insbesondere soll mittels der Analysemodule und der bereitgestellten Bewertungsinformationen und/oder der bereitgestellten Assistenzinformation einem Diagnose erstellendem Arzt eine maximale Unterstützung bereitgestellt werden.

Mittels des zumindest einen Qualitäts-Analysemoduls und/oder des zumindest einen klinischen Analysemoduls wird dabei eine definierte klinische Fragestellung und/oder eine definierte qualitätsbezogene Fragestellung und/oder eine patientenrelevante Fragestellung in Abhängigkeit des ausgewählten Messprogramms analysiert.

Das zumindest eine technische Analysemodul ist bevorzugt dazu ausgebildet, technische und/oder gerätebezogene Informationen, die für das Messprogramm MP zur Verfügung stehen, zu analysieren. Dabei wird mittels des zumindest einen technischen Analysemoduls eine definierte technische Fragestellung in Abhängigkeit des ausgewählten Messprogramms MP analysiert. Beispielsweise ist das zumindest eine technische Analysemodul zur Analyse von Spulendaten von lokalen Hochfrequenzspulen ausgebildet. Die lokalen Hochfrequenzspulen werden zur Erfassung von Magnetresonanzdaten um den zu untersuchenden Bereich des Patienten positioniert. Für unterschiedliche Körperbereiche stehen auch unterschiedliche lokale Hochfrequenzspulen zur Verfügung, beispielsweise eine Kopf-Hochfrequenzspule für eine Kopfuntersuchung oder eine Knie-Hochfrequenzspule für eine Knieuntersuchung. Anhand der Spulendaten kann beispielsweise das zumindest eine technische Analysemodul erkennen, ob für das ausgewählte Messprogramm MP die dafür erforderliche Hochfrequenzspule mit einer Scannereinheit einer Magnetresonanzvorrichtung 10 verbunden ist. Zudem kann das technische Analysemodul auch zu einer Erkennung und/oder Früherkennung von Hardware-Defekten ausgebildet sein. Beispielsweise kann das zumindest eine technische Analysemodul dazu ausgebildet sein, aktuelle Betriebsparameter von lokalen Hochfrequenzspulen zu analysieren und daraus auf einen Zustand, insbesondere eine verbleibende Lebensdauer, der lokalen Hochfrequenzspulen oder von einzelnen Komponenten der lokalen Hochfrequenzspule schließen.

Das zumindest eine allgemeine Analysemodul ist bevorzugt dazu ausgebildet, allgemeine Prozesse, die mit dem Ausführen des Messprogramms MP verbunden sind zu überwachen und zu analysieren. Dabei kann das zumindest eine allgemeine Analysemodul einen technischen Erkennungsalgorithmus umfassen. Ein derartiger technischer Erkennungsalgorithmus kann beispielsweise zu einer Erkennung und/oder Analyse eines Auslösens einer Bildvorverarbeitung ausgebildet sein. Alternativ oder zusätzlich kann das zumindest eine allgemeine Analysemodul auch einen einstellungsabhängigen Erkennungsalgorithmus umfassen. Ein derartiger einstellungsabhängiger Erkennungsalgorithmus kann beispielsweise Einstellungen des ausgewählten Messprogramms MP mit vorhandenen Software-Konfigurationen und/oder Software-Lizenzen und/oder mit Hardware-Konfigurationen vergleichen und bei Abweichungen einen Hinweis generieren, dass zur Ausführung des Messprogramms MP nicht alle Software relevanten und/oder Scanner relevanten Voraussetzungen erfüllt sind und deshalb mit Einschränkungen und/oder Problemen während des Ausführens des Messprogramms MP zu rechnen ist. Alternativ oder zusätzlich kann das zumindest eine allgemeine Analysemodul auch zu einer Analyse von allgemeinen Prozessen des Messprogramms MP ausgebildet sein, wie beispielsweise eine Analyse zur Berichterstellung.

Die von dem zumindest einen Analysemodul AM ermittelte Bewertungsinformation wird zudem in diesem vierten Verfahrensschritt 103 bereitgestellt. Das Bereitstellen der zumindest einen Bewertungsinformation erfolgt hierbei mittels des Transaktionsmanagers TM.

In einem weiteren fünften Verfahrensschritt 104 erfolgt ein Generieren einer Assistenzinformation. Die Assistenzinformation wird dabei in Abhängigkeit von der zumindest einen Bewertungsinformation mittels des zumindest einen Analysemoduls AM generiert. Bei Verwendung von mehreren Analysemodulen AM1, AM2, ..., AMn bei der Ausführung eines Messschritts MSi kann dabei jedes der Analysemodule AM1, AM2, ..., AMn eine Assistenzinformation generieren. Zudem kann es auch sein, dass bei Verwendung von mehreren Analysemodulen AM1, AM2, ..., AMn bei der Ausführung eines Messschritts MSi nur eines der Analysemodule AMi eine Assistenzinformation generiert, insbesondere wenn das die Assistenzinformation generierende Analysemodul AMi auf die Bewertungsinformationen der weiteren Analysemodule AM1, AM2, ..., AMn als Eingangsdaten zugreift.

Die Assistenzinformation umfasst dabei eine Probleminformation und/oder eine Auffälligkeitsinformation. Die Probleminformation soll einen Benutzer auf ein mögliches Problem, das mit dem Ausführen des Messprogramms MP und/oder der Magnetresonanzuntersuchung verbunden ist, hinweisen soll. Die Auffälligkeitsinformation umfasst bevorzugt eine Information, die auf eine Auffälligkeit des Patienten beschreibt, also insbesondere einen von einer Norm abweichenden Zustand. Derartige Auffälligkeitsinformationen stellen nicht zwangsläufig ein Qualitätsproblem in den erfassten Daten dar. Vielmehr kann die Auffälligkeitsinformation auch eine neutrale Information umfassen, die auf beispielsweise eine Anomalie und eine Abnormität während der Ausführung zumindest einen Messschritts des ausgewählten Messprogramms hinweist.

Zudem umfasst die Assistenzinformation auch eine Statusinformation, die einen Status des zumindest einen Analysemoduls AM wiedergibt. Die Statusinformation umfasst dabei eine Information, welcher Art das Analysemodul AM ist, beispielsweise ob ein Qualitäts-Analysemodul und/oder ein technisches Analysemodul und/oder ein klinisches Analysemodul für die Beurteilung und/oder Analyse des Messschritts MSi ausgewählt wurde. Zudem kann die Statusinformation dem Benutzer anzeigen, ob aktuelle eine Analyse mittels des Analysemoduls AM durchgeführt wird. Zudem kann die Statusinformation dem Benutzer anzeigen, ob die Analyse mittels des Analysemoduls AM erfolgreich beendet wurde und ein Ergebnis vorliegt. Zudem kann die Statusinformation dem Benutzer anzeigen, ob die Analyse mittels des Analysemoduls AM nicht durchgeführt werden konnte.

Das zumindest eine Analysemodul AM generiert dabei Ausgangsdaten, die klassifiziert werden. Die Ausgangsdaten des zumindest einen Analysemoduls umfassen dabei die Bewertungsinformation und/oder die Statusinformation des zumindest einen Analysemoduls AM. Dabei stehen unterschiedliche Datenklassen für eine Klassifikation zur Verfügung. Für eine Klassifikation der Ausgangsdaten der Analysemodule AM stehen bevorzugt ein "Traffic-light Classifier", ein "Binary Classifier", ein "Multi-Class Classifier" und ein "Multi-Class Multi-Label Classifier" zur Verfügung. Eine Einteilung der Ausgangsdaten in eine zur Verfügung stehende Klassifikation umfasst dabei einen semantischen Test der zu klärenden Fragestellung des betreffenden Analysemoduls AM.

Die generierte Assistenzinformation wird zudem in diesem fünften Verfahrensschritt 104 bereitgestellt. Das Bereitstellen der Assistenzinformation erfolgt hierbei mittels des Transaktionsmanagers TM.

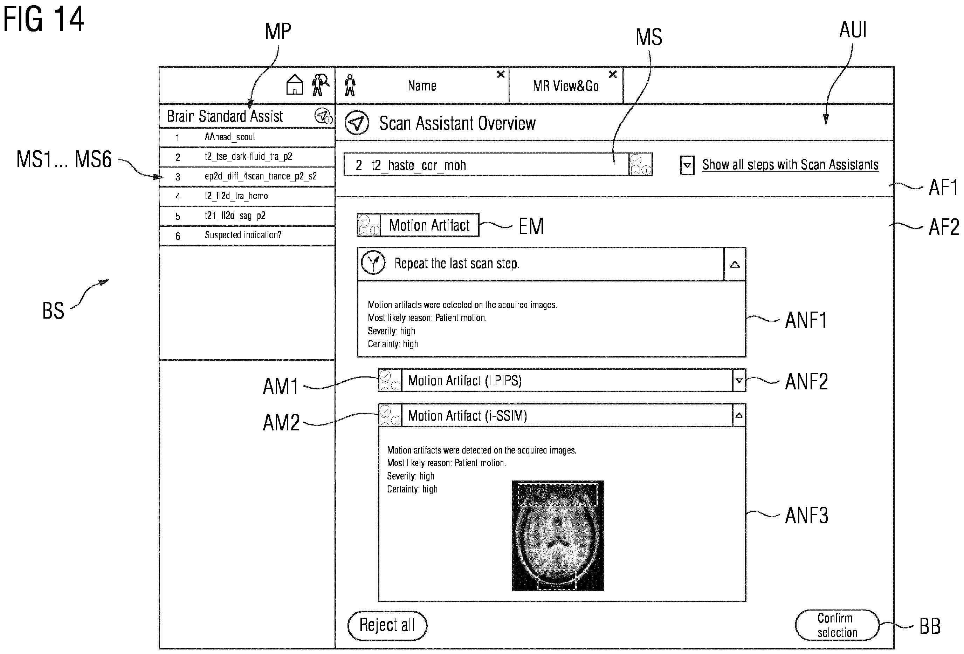

In einem weiteren, sechsten Verfahrensschritt 105 erfolgt eine Ausgabe der Assistenzinformation. Die Ausgabe der Assistenzinformation erfolgt dabei ein Assistenz-Userinterface AUI, das über die Benutzerschnittstelle BS an einen Benutzer ausgegeben wird. Insbesondere erfolgt an der Benutzerschnittstelle BS eine visuelle Ausgabe mittels einer Darstellungseinheit, insbesondere eines Displays und/oder eines Monitors, an den Benutzer. Dabei erfolgt die Ausgabe der Assistenzinformation an den Benutzer über eine Benutzeroberfläche BO zur Steuerung und/oder Überwachung des Messprogramms MP, wobei das Assistenz-Userinterface AUI in die Benutzeroberfläche BO integriert ist (siehe auch Fig. 14).

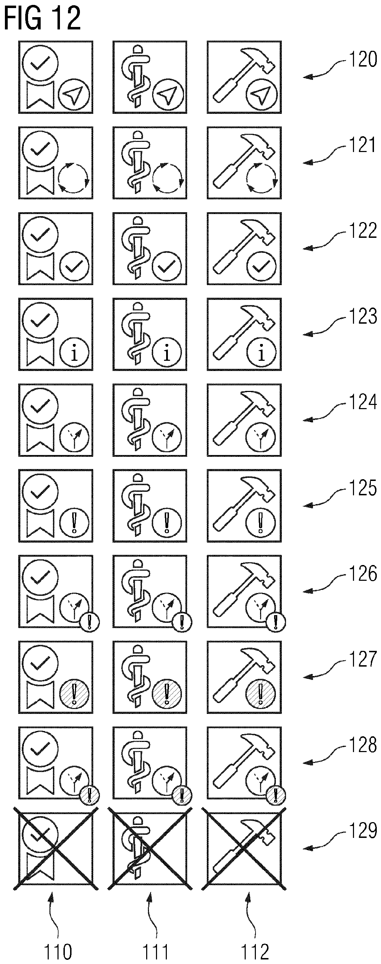

Die Ausgabe der Statusinformation des Analysemoduls AM wird dabei durch entsprechende Symbole angezeigt, die an einer definierten Position auf der Benutzeroberfläche BO angeordnet sind. Beispiele für Ausgabesymbole der Statusinformation sind in Fig. 12 dargestellt.

Die Probleminformation und/oder die Auffälligkeitsinformation wird im vorliegenden Ausführungsbeispiel an der Benutzeroberfläche BO nur bei einem aktiven Abrufen durch den Benutzer angezeigt. Dabei kann durch ein Anklicken eines dafür vorgesehenen Symbols und/oder Buttons auf der Benutzeroberfläche BO die Anzeige der Probleminformation und/oder der Auffälligkeitsinformation erfolgen. Durch die Anzeige der Statusinformation ist der Benutzer bereits informiert, dass eine Probleminformation und/oder eine Auffälligkeitsinformation vorliegt.

Das Bereitstellen der Ausgangsdaten und das Bereitstellen der Assistenzinformation des zumindest einen Analysemoduls AM und auch die Ausgabe der Assistenzinformation erfolgt bevorzugt kontinuierlich während des gesamten Messschritts MSi oder der gesamten Messschritte MS1, MS2, ..., MSn, für das oder für die das Analysemodul AM ausgewählt wurde, so dass ein Benutzer eine ständige Unterstützung erfährt.

In Fig. 2 ist ein erstes Beispiel des Assistenzsystems AM dargestellt. Das Assistenzsystem AM ist hierbei zu einem Ausführen des Verfahrens zu einem Unterstützen und/oder Assistieren eines Benutzers bei einem Ausführen eines Messprogramms MP während einer Magnetresonanzdatenerfassung ausgebildet, wie dies in Fig. 1 beschrieben ist. Das Assistenzsystem AS umfasst den Transaktionsmanager TM, das Konfigurations-Interface KUI und zumindest ein Analysemodul AM1, ..., AMn. Dabei kann im vorliegenden Ausführungsbeispiel das Assistenzsystem AS 1 bis N Analysemodule AM1, ..., AMn umfassen.

Das Messprogramm MP umfasst mehrere Messschritte MS1, MS2, ... MSn. Eine Information der für die einzelnen Messschritte MS1, MS2, ... MSn ausgewählten Analysemodule AM1, ..., AMn werden dem Transaktionsmanager TM mittels des Konfigurations-Userinterfaces bereitgestellt KUI. Während des Ausführens des ersten Messschritts MS1 werden fortlaufend Magnetresonanzdaten und/oder weitere Messinformationen den Analysemodulen AM1, ..., AMn über den Transaktionsmanager TM bereitgestellt. Jedes einzelne Analysemodul AM1, ..., AMn analysiert und/oder klärt dabei eine definierte Fragestellung betreffend den ersten Messschritt MS1 und ermittelt dabei eine Bewertungsinformation und eine Assistenzinformation. Die Assistenzinformation wird mittels des Transaktionsmanagers TM dem Assistenz-Userinterface AUI zur Ausgabe an der Benutzerschnittstelle BS bereitgestellt. An der Benutzerschnittstelle BS erfolgt die Ausgabe der Assistenzinformation.

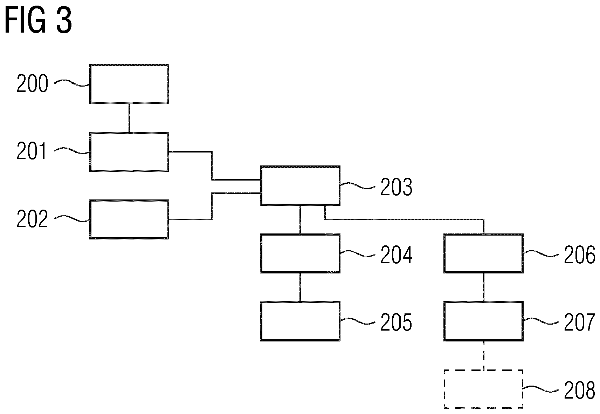

In Fig. 3 ist ein alternatives Ausführungsbeispiel des Verfahrens zu einem Unterstützen und/oder Assistieren eines Benutzers mittels eines Assistenzsystems AS bei einem Ausführen eines Messprogramms MP zur einer Magnetresonanzdatenerfassung dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 1, wobei bezüglich gleichbleibender Bauteile, Merkmale und Funktionen auf die Beschreibung des Ausführungsbeispiels in Fig. 1 verwiesen wird.

In einem ersten Verfahrensschritt 200 erfolgt ein Auswählen eines Messprogramms MP zu einem Erfassen von Magnetresonanzdaten mit einer definierten diagnostischen Fragestellung. Zusammen mit dem Messprogramm MP kann der Benutzer auch eine Unterstützung mittels des Assistenzsystems AS auswählen. Dabei können durch Auswahl des Messprogramms MP eine bereits auf die klinische und/oder diagnostische Fragestellung des Messprogramms MP abgestimmte und/oder zugeordnete Auswahl an Analysemodulen AM und/oder Empfehlungsmodulen EM und/oder eine Kombination von auf die klinische und/oder diagnostische Fragestellung des Messprogramms MP abgestimmte Analysemodule AM und Empfehlungsmodule EM dem Benutzer zur Auswahl angeboten werden. Das Messprogramm MP umfasst dabei mehrere Messschritte MS1, MS2, ..., MSn, wobei für zumindest einen Messschritt MSi eine Unterstützung mittels des Assistenzsystems AS und damit zumindest ein Analysemodul AM und/oder zumindest ein Empfehlungsmodul EM auswählbar ist. Zudem kann auch für jeden der mehreren Messschritte MS1, MS2, ..., MSn des Messprogramms MP eine Unterstützung mittels des Assistenzsystems AS und damit zumindest ein Analysemodul AM und/oder zumindest ein Empfehlungsmodul EM auswählbar sein. Die Auswahl erfolgt mittels des Konfigurations-Interfaces KUI an der Benutzerschnittstelle BS.

Neben der Auswahl der einzelnen Analysemodule AMi und/oder Empfehlungsmodule EMi für die einzelnen Messschritt MSi können diese auch an den jeweiligen Messschritt MSi mittels des Konfigurations-Userinterfaces KUI angepasst werden. Auch kann ein Benutzer mittels des Konfigurations-Userinterfaces KUI auswählen, in welchem Umfang er eine Unterstützung mittels des Assistenzsystems AS beanspruchen möchte, beispielsweise eine automatische Unterstützung, bei der Empfehlungen automatisch von dem Assistenzsystem AS umgesetzt werden, oder eine halbautomatische Unterstützung, bei der Empfehlung erst nach Zustimmung durch den Benutzer umgesetzt werden, oder nur eine Ausgabe von Informationen oder Empfehlungen.

Zudem kann es diesem ersten Verfahrensschritt 200 auch sein, dass bei Auswahl eines Analysemoduls AM für zumindest einen Messschritt MSi des Messprogramms MP bereits Empfehlungsmodule EMi zur Auswahl vorgeschlagen werden. Die vorgeschlagenen Empfehlungsmodule EMi sind dabei bevorzugt auf das ausgewählte Analysemodul AM abgestimmt und können einen Vorschlag zur Abhilfe eines mittels des ausgewählten Analysemoduls AM identifizierten Problems und/oder kritischen Zustands und/oder Auffälligkeit bereitstellen. Die Empfehlungsmodule EMi werden dabei durch das Assistenzsystem AS, insbesondere mittels des Konfigurations-Interfaces KUI, automatisch dem Benutzer bei der Auswahl des Analysemoduls AM vorgeschlagen.

Zudem kann es diesem ersten Verfahrensschritt 200 auch sein, dass bei Auswahl eines Empfehlungsmoduls EM für zumindest einen Messschritt MSi des Messprogramms MP, zumindest ein Analysemodul AM mit dem Empfehlungsmodul EM verknüpft ist. Bei Auswahl des zumindest einen Empfehlungsmoduls EM wird das zumindest eine Analysemodul AM automatisch mit ausgewählt ist. Beispielsweise setzt das ausgewählte Empfehlungsmodul EM einen Dateneingang voraus, der durch die mit dem Empfehlungsmodul EM verknüpften Analysemodule AM bereitgestellt wird. Die Auswahl der Analysemodule AMi erfolgt dabei bevorzugt automatisch mittels des Assistenzsystems AS, insbesondere mittels des Konfigurations-Interfaces KUI.

Dabei können auch unterschiedliche Analysemodule AMi und unterschiedliche Empfehlungsmodule EMi für unterschiedliche Messschritte MS1, MS2, ..., MSn des Messprogramms MP zur Verfügung stehen und/oder dem Benutzer zu Auswahl angeboten werden. Das Auswählen des Messprogramms MP und auch das Auswählen des zumindest einen Analysemoduls AM und/oder Empfehlungsmoduls EM erfolgt durch einen Benutzer mittels des Konfigurations-Interfaces KUI an der Benutzerschnittstelle BS.

In einem zweiten Verfahrensschritt 201 erfolgt ein Ausführen des ausgewählten Messprogramms MP, wobei das Ausführen des Messprogramms MP ein Erfassen von Magnetresonanzdaten umfasst. Neben dem Erfassen von Magnetresonanzdaten werden auch aus den Magnetresonanzdaten Bilddaten rekonstruiert. Die Magnetresonanzdaten und die rekonstruierten Bilddaten werden von dem Transaktionsmanager TM bereitgestellt. Dieser zweite Verfahrensschritt 201 ist analog zu dem Verfahrensschritt 101 zu der Beschreibung zu Fig. 1 ausgebildet, auf die hiermit verwiesen wird.

In einem dritten Verfahrensschritt 202 erfolgt ein Erfassen weiterer Messinformationen, wobei die weiteren Messinformationen vor einem Ausführen des ausgewählten Messprogramms MP oder während eines Ausführens des ausgewählten Messprogramms MP erfasst werden. Dieser dritte Verfahrensschritt 202 ist analog zu dem Verfahrensschritt 102 zu der Beschreibung zu Fig. 1 ausgebildet, auf die hiermit verwiesen wird.

In einem vierten Verfahrensschritt 203 erfolgt ein Ermitteln zumindest einer Bewertungsinformation mittels zumindest eines Analysemoduls AM des Assistenzsystems AS in Abhängigkeit von den bereitgestellten Magnetresonanzdaten und/oder den bereitgestellten Bilddaten und/oder den bereitgestellten weiteren Messinformationen. Dieser vierte Verfahrensschritt 203 ist analog zu dem Verfahrensschritt 103 zu der Beschreibung zu Fig. 1 ausgebildet, auf die hiermit verwiesen wird.

In einem fünften Verfahrensschritt 204 erfolgt ein Generieren einer Assistenzinformation, wobei die Assistenzinformation in Abhängigkeit von der zumindest einen Bewertungsinformation mittels des zumindest einen Analysemoduls AM generiert wird. Dieser fünfte Verfahrensschritt 204 ist analog zu dem Verfahrensschritt 104 zu der Beschreibung zu Fig. 1 ausgebildet, auf die hiermit verwiesen wird.

In einem sechsten Verfahrensschritt 205 erfolgt eine Ausgabe der Assistenzinformation. Dieser sechste Verfahrensschritt 205 ist analog zu dem Verfahrensschritt 105 zu der Beschreibung zu Fig. 1 ausgebildet, auf die hiermit verwiesen wird.

Zeitgleich mit dem fünften und sechsten Verfahrensschritt 204, 205 erfolgt in einem siebten Verfahrensschritt 206 ein Ermitteln zumindest eines Vorschlags für zumindest einen Messschritt MSi des Messprogramms MP mittels des zumindest einen Empfehlungsmoduls EM. Dabei erfolgt das Ermitteln des zumindest einen Vorschlags anhand zumindest einer bereitgestellten Bewertungsinformation des zumindest einen Analysemoduls AM. Zur Ermittlung des zumindest einen Vorschlags greift das zumindest einen Empfehlungsmodul EM auf an eine bestimmte Problemstellung und/oder an eine bestimmte diagnostische Fragestellung angepasste Vorschläge zurück. Dabei kann das zumindest eine Empfehlungsmodul EM mehrere an die bestimmte Problemstellung und/oder an die bestimmte diagnostische Fragestellung angepasste Vorschläge umfassen, die jeweils für sich zu einer Behebung und/oder Lösung und/oder Vermeidung des Problems und/oder zur Klärung einer Auffälligkeit beitragen können. Diese mehreren Vorschläge sind bevorzugt in einer Datenbank gespeichert. Auch kann es sein, dass die bereitgestellte Bewertungsinformation auch eine Information bereithält, die eine Auswahl eines Vorschlags aus den mehreren Vorschlägen durch das zumindest eine Empfehlungsmodul EM begünstigt.

Des Weiteren wird von dem Empfehlungsmodul EM in diesen siebten Verfahrensschritt 207 eine Statusinformation generiert, die von Ausgangsdaten des Empfehlungsmoduls EM umfasst sind. Die Ausgangsdaten des Empfehlungsmoduls EM, insbesondere die Statusinformation des Empfehlungsmoduls, wird dabei klassifiziert, unterschiedliche Ausgangsdatenklassen zur Verfügung stehen. Für eine Klassifikation der Ausgangsdaten der Analysemodule stehen bevorzugt ein "Traffic-light Classifier", ein "Binary Classifier", ein "Multi-Class Classifier" und ein "Multi-Class Multi-Label Classifier" zur Verfügung (siehe Fig. 12).

Zudem wird in diesem siebten Verfahrensschritt 206 von dem zumindest einem Empfehlungsmodul EM eine erweiterte Assistenzinformation ermittelt, wobei die erweiterte Assistenzinformation den zumindest einen ermittelten Vorschlag und/oder die Statusinformation des Empfehlungsmoduls AM umfasst. Die erweiterte Assistenzinformation wird zudem für eine Ausgabe an den Benutzer bereitgestellt. Das Bereitstellen erfolgt dabei mittels des Transaktionsmanagers TM an das Assistenz-Userinterface AUI, wobei das Assistenz-Userinterface AUI die erweiterte Assistenzinformation der Benutzerschnittstelle BS zur Ausgabe an den Benutzer zur Verfügung stellt.

In einem achten Verfahrensschritt 207 erfolgt eine Ausgabe der von dem zumindest einem Empfehlungsmodul EM ermittelten erweiterten Assistenzinformation. Die erweiterte Assistenzinformation umfasst dabei den Vorschlag und die Statusinformation. Die erweiterte Assistenzinformation wird dem Benutzer über das Assistenz-Userinterface AUI an der Benutzerschnittstelle BS mitgeteilt. Dabei wird die erweiterte Assistenzinformation über eine Benutzeroberfläche BO zu Steuerung und/oder Überwachung des Mesprogramms MP an den Benutzer ausgegeben.

Zudem kann das Assistenzsystem AS in einem weiteren, optionalen Verfahrensschritt 208 zu einer Ausführung des zumindest einen Vorschlags für den zumindest einen Messschritt MSi ausgebildet sein. Dabei kann das Assistenzsystems AS zu einer Ausführung des zumindest einen Vorschlags für den zumindest einen Messschritt MSi in einem halbautomatischen Ausführungsmodus oder in einem vollautomatischen Ausführungsmodus ausgebildet sein. Bevorzugt erfolgt in dem ersten Verfahrensschritt 200 bereits eine Auswahl durch den Benutzer, welchen Ausführungsmodus bei der Ausführung der einzelnen Messschritte MS1, MS2, ..., MSn haben möchte. Dabei kann der Benutzer auch unterschiedliche Ausführungsmodi für unterschiedliche Messschritte MS1, MS2, ..., MSn des Messprogramms MP auswählen. Die Auswahl erfolgt über die Benutzerschnittstelle BS.

In dem halbautomatische Ausführungsmodus wird der Benutzer nochmals aufgefordert, eine Bestätigungseingabe vor einer Ausführung des Vorschlags einzugeben. Hierzu wird vom Assistenzsystem AS eine entsprechende Aufforderung zur Bestätigungseingabe generiert und über die Benutzeroberfläche BO an den Benutzer ausgegeben. Sobald die Bestätigungseingabe vorliegt, wird anschließend automatisch von dem Assistenzsystem AS der Vorschlag ausgeführt. Wird von dem Benutzer keine Bestätigungseingabe eingegeben, muss der Benutzer die einzelnen Schritte selbst vornehmen oder auf diese verzichten. In dem automatischen Ausführungsmodus führt das Assistenzsystem AS den Vorschlag automatisch aus und der Benutzer wird lediglich über den Vorschlag und dessen Ausführung informiert.

Zudem kann es in diesem weiteren, optionalen Verfahrensschritt 208 auch vorgesehen sein, dass der Benutzer aufgefordert wird, nach Ausführen des Vorschlags einen Feedback-Information einzugeben, die eine Aussage über einen möglichen Erfolg des ausgeführten Vorschlags umfasst. Hierzu wird vom Assistenzsystem AS eine entsprechende Aufforderung zur Eingabe der Feedback-Information generiert und über die Benutzeroberfläche BO an den Benutzer ausgegeben.

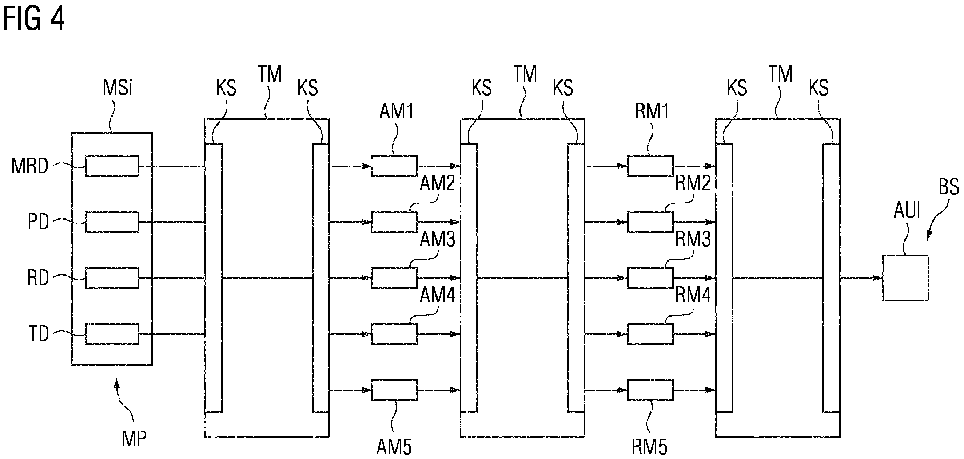

In Fig. 4 ist ein alternatives Ausführungsbeispiel des Assistenzsystems AS dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 2, wobei bezüglich gleichbleibender Bauteile, Merkmale und Funktionen auf die Beschreibung des Ausführungsbeispiels in Fig. 2 verwiesen wird.

Das Assistenzsystem AS in Fig. 4 umfasst neben dem Transaktionsmanager TM und Analysemodulen AM1, AM2, AM3, AM4, AM5 auch Empfehlungsmodule EM1, EM2, EM3, EM4, EM5. Zudem umfasst das Assistenzsystem AS das in Fig. 4 nicht näher dargestellte Konfigurations-Userinterface KUI, wobei hinsichtlich einer Funktionsweise des Konfigurations-Userinterfaces KUI auf die Beschreibung zu Fig. 2 verwiesen wird. Dem Assistenzsystem AS werden zur Ausführung des erfindungsgemäßen Verfahrens von der Magnetresonanzvorrichtung Eingangsdaten zur Analyse mittels der Analysemodule AM1, AM2, AM3, AM4, AM5 bereitgestellt. Das Bereitstellen der Eingangsdaten erfolgt mittels des Transaktionsmanagers TM. Als Eingangsdaten stehen hierbei Magnetresonanzdaten MRD, insbesondere Rohdaten und/oder K-Raumdaten, und/oder aus den Magnetresonanzdaten rekonstruierte Bilddaten zur Verfügung. Des Weiteren stehen als Eingangsdaten auch physiologische Daten PD des Patienten, wie insbesondere EKG-Daten und/oder Atemdaten, zur Verfügung. Des Weiteren stehen als Eingangsdaten weitere Patientendaten RD, die bereits bei der Registrierung des Patienten erfasst werden, zur Verfügung. Des Weiteren stehen als Eingangsdaten Hardware-Parameter TD, beispielsweise Spulenparameter und/oder Temperaturparameter usw., zur Verfügung.

Für die Analyse der Eingangsdaten während des Ausführens des Messprogramms MP stehen im vorliegenden Ausführungsbeispiel fünf Analysemodule AM1, AM2, AM3, AM4, AM5 zur Verfügung. Dabei sind die beiden Analysemodule AM1 und AM2 zur Analyse der Magnetresonanzdaten MRD und der rekonstruierten Bilddaten ausgebildet. Das Analysemodul AM3 ist zur Analyse der physiologischen Daten PD ausgebildet. Das Analysemodul AM4 ist zur Analyse der weiteren Patientendaten RD ausgebildet. Das Analysemodul AM5 ist zur Analyse der Hardware-Parameter TD ausgebildet. Jedes der Analysemodule AM1, AM2, AM3, AM4, AM5 analysiert die Eingangsdaten im Hinblick auf die klinische und/oder diagnostischen Fragestellung des Messprogramms MP. Aus den analysierten Eingangsdaten können die einzelnen Analysemodule AM1, AM2, AM3, AM4, AM5 kritische Zustände und/oder Probleme und/oder Auffälligkeiten erkennen, die aktuell bei der Ausführung des Messprogramms MP, insbesondere des aktuellen ausgeführten Messschritts MSi auftreten und/oder auftreten können. Diese Information ist in der Bewertungsinformation enthalten, wobei von jedem der Analysemodule AM1, AM2, AM3, AM4, AM5 eine Bewertungsinformation ermittelt wird. Die Bewertungsinformationen werden als Ausgangsdaten der Analysemodule AM1, AM2, AM3, AM4, AM5 bereitgestellt von dem Transaktionsmanager TM.

Die Ausgangsdaten der Analysemodule AM1, AM2, AM3, AM4, AM5 werden als Eingangsdaten für Empfehlungsmodule EM1, EM2, EM3, EM4, EM5 von dem Transaktionsmanager TM bereitgestellt. Im vorliegenden Ausführungsbeispiel stehen fünf Empfehlungsmodule EM1, EM2, EM3, EM4, EM5 für die Ermittlung von Vorschlägen zur Verfügung. Dabei können die unterschiedliche Empfehlungsmodule EM1, EM2, EM3, EM4, EM5 einen gleichen Vorschlag umfassen, wobei der Vorschlag zur Behebung von unterschiedlichen Problemen und/oder unterschiedlichen kritischen Zuständen und/oder zur Klärung auf Auffälligkeiten, beispielsweise in den Bilddaten, ausgelegt ist. Sofern die einzelnen Analysemodule AM1, AM2, AM3, AM4, AM5 einen kritischen Zustand ermittelt haben, wird von den jeweiligen Empfehlungsmodulen EM1, EM2, EM3, EM4, EM5 ein Lösungsvorschlag ermittelt. Dabei können einige Empfehlungsmodule EM1, EM2, EM3, EM4, EM5 auch mehrere Vorschläge und/oder Lösungsvorschläge zur Auswahl haben.

Das Empfehlungsmodul EM1 ermittelt in Abhängigkeit von der Bewertungsinformation des Analysemoduls AM1 einen Vorschlag, wobei der Vorschlag ein Wiederholen des aktuellen Messchritts MSi umfasst. Umfasst die Bewertungsinformation keinen kritischen Zustand und/oder keine Auffälligkeit, umfasst der Vorschlag und/oder die Empfehlung des Empfehlungsmoduls EM1 ein Fortführen des aktuellen Messprogramms MP. Das Empfehlungsmodul EM2 ermittelt in Abhängigkeit von den Analysemodulen AM1, AM3 und AM4 einen Vorschlag, wobei ein Wiederholen des aktuellen Messschritts MSi, ein Ändern von Parametereinstellung für den aktuellen Messschritt MSi und ein Hinzufügen von zumindest einen weiteren Messschritt MS als mögliche Vorschläge zur Verfügung stehen. Umfassen die eingehenden Bewertungsinformationen keinen kritischen Zustand und/oder keine Auffälligkeit, umfasst der Vorschlag und/oder die Empfehlung des Empfehlungsmoduls EM2 ein Fortführen des aktuellen Messprogramms MP. Das Empfehlungsmodul EM3 ermittelt in Abhängigkeit von dem Analysemodulen AM2 einen Vorschlag, wobei ein Nachjustieren von Parametern als möglicher Vorschlag zur Verfügung steht. Umfasst die Bewertungsinformation keinen kritischen Zustand und/oder keine Auffälligkeit, umfasst der Vorschlag und/oder die Empfehlung des Empfehlungsmoduls EM3 ein Fortführen des aktuellen Messprogramms MP. Das Empfehlungsmodul EM4 ermittelt in Abhängigkeit von dem Analysemodule AM3 einen Vorschlag, wobei ein Hinzufügen von zumindest einen weiteren Messschritt und ein Anpassen eines EKG-Triggersignals als mögliche Vorschläge zur Verfügung stehen.

Umfasst die Bewertungsinformation keinen kritischen Zustand und/oder keine Auffälligkeit, umfasst der Vorschlag und/oder die Empfehlung des Empfehlungsmoduls EM4 ein Fortführen des aktuellen Messprogramms MP. Das Empfehlungsmodul EM5 ermittelt in Abhängigkeit von den Analysemodulen AM5 einen Vorschlag, wobei eine Kalibration einer Hardware-Einheit und eine Informieren eines technischen Services und ein Informieren des Benutzers als mögliche Vorschläge zur Verfügung stehen. Umfasst die Bewertungsinformation keinen kritischen Zustand und/oder Auffälligkeit, umfasst der Vorschlag und/oder die Empfehlung des Empfehlungsmoduls EM5 ein Fortführen des aktuellen Messprogramms MP.

Das jeweilige Empfehlungsmodul EM1, EM2, EM3, EM4, EM5 ermittelt dabei den Vorschlag, der am besten mit der Bewertungsinformation oder den mehreren Bewertungsinformationen übereinstimmt. Zudem können die Empfehlungsmodule EM1, EM2, EM3, EM4, EM5 auch einen gemeinsamen Vorschlag oder mehrere gemeinsame Vorschläge ermitteln. Der ermittelte Vorschlag und/oder die ermittelten Vorschläge werden mittels des Transaktionsmanagers TM dem Assistenz-Userinterface AUI bereitgestellt und an der Benutzerschnittstelle BS an den Benutzer ausgegeben.

Eine Datenübertragung zwischen der Magnetresonanzvorrichtung und/oder den Analysemodulen AM1, AM2, AM3, AM4, AM5 und/oder den Empfehlungsmodulen EM1, EM2, EM3, EM4, EM5 und/oder der Benutzerschnittstelle BS erfolgt mittels des Transaktionsmanagers TM. Der Transaktionsmanager TM weist hierzu eine Kommunikationsschnittstelle KS zu einem Datenaustausch zwischen der Magnetresonanzvorrichtung und/oder den Analysemodulen AM1, AM2, AM3, AM4, AM5 und/oder den Empfehlungsmodulen EM1, EM2, EM3, EM4, EM5 und/oder der Benutzerschnittstelle BS auf.

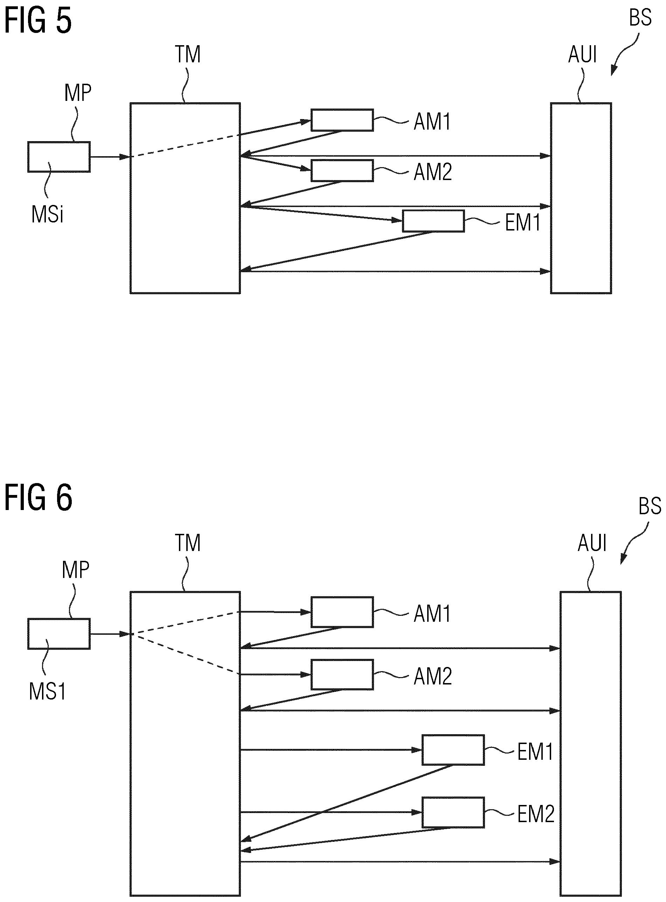

In den Fig. 5 bis 11 sind unterschiedliche Ausführungsbeispiele des Assistenzsystems AS dargestellt, wobei die unterschiedlichen Ausführungsbeispiele eine unterschiedliche Anzahl an Analysemodulen AM1, AM2, AM3 und/oder Empfehlungsmodulen EM1, EM2 umfassen. Ein Datenaustausch zwischen den einzelnen Analysemodulen AM1, AM2 und AM3, den einzelnen Empfehlungsmodulen EM1, EM2 und den weiteren Einheiten erfolgt jeweils mittels des Transaktionsmanagers TM. Eine Auswahl und/oder Konfigurierung der einzelnen Analysemodule AM1, AM2, AM3 und/oder Empfehlungsmodule EM1, EM2 erfolgt mittels des Konfigurations-Userinterfaces KUI, das in den Fig. 5 bis 11 nicht näher dargestellt ist.

In Fig. 5 ist für einen Messschritt MSi eine Unterstützung mittels des Assistenzsystems AS ausgewählt. Dabei wurden zwei Analysemodule AM1, AM2 und ein Empfehlungsmodul EM1 ausgewählt. Das Analysemodul AM1 ermittelt dabei eine Bewertungsinformation, die als Eingangsdaten des zweiten Analysemoduls AM2 dient. Die von dem zweiten Analysemodul AM2 ermittelte Bewertungsinformation dient als Eingangsdaten für des Empfehlungsmoduls EM1. Mittels der ausgewählten Analysemodule AM1, AM2 und des Empfehlungsmoduls EM1 kann eine schrittweise Artefakt-Detektion und Artefakt-Klassifikation durchgeführt werden.

In dem Messschritt MSi werden Magnetresonanzdaten erfasst und daraus Bilddaten mit zumindest ein Bild rekonstruiert. Diese Bilddaten werden mittels des Transaktionsmanagers TM dem ersten Analysemodul AM1 bereitgestellt. Das erste Analysemodul AM1 ermittelt anhand der bereitgestellten Bilddaten, insbesondere des zumindest einen Bilds, ob ein Bildqualitätsproblem in den Bilddaten vorliegt und generiert daraus eine Bewertungsinformation. Die Bewertungsinformation kann beispielsweise den Wert "0" annehmen, wenn kein Bildqualitätsproblem erkannt wurde. Alternativ kann die Bewertungsinformation auch den Wert "1" annehmen, wenn ein Bildqualitätsproblem in den Bilddaten von dem ersten Analysemodul AM1 erkannt wurde.

Die Bewertungsinformation wird von dem Transaktionsmanager TM dem zweiten Analysemodul AM2 bereitgestellt. Wenn die Bewertungsinformation des ersten Analysemoduls AM1 den Wert "0" umfasst, erfolgt mittels des zweiten Analysemoduls AM2 keine weitere Analyse. Umfasst die Bewertungsinformation des ersten Analysemoduls AM1 den Wert "1", wird mittels des zweiten Analysemoduls AM2 eine weitere Analyse der Bilddaten durchgeführt. Dabei wird mittels des zweiten Analysemoduls AM2 bestimmt, welcher Art die Bildqualitätsprobleme sein können und/oder was die Ursache für die Bildqualitätsprobleme in den bereitgestellten Bilddaten sein kann. Beispielsweise können für 70% der Bildqualitätsprobleme Bewegungsartefakte und für 10% der Bildqualitätsprobleme Gibbs Ringing als Ursache ermittelt werden. Das Ergebnis wird von dem zweiten Analysemodul AM2 als Bewertungsinformation zur Verfügung gestellt. Zudem wird die Information, dass Bildqualitätsprobleme erkannt wurden und/oder was die Ursache für die Bildqualitätsprobleme von dem ersten Analysemodul AM1 und/oder zweiten Analysemodul AM2 auch als Assistenzinformation für eine Ausgabe an den Benutzer bereitgestellt. Diese Assistenzinformation wird vom Transaktionsmanager TM dem Assistenz-Userinterface AUI bereitgestellt, wobei das Assistenz-Userinterface AUI die Assistenzinformation der Benutzerschnittstelle BS zur Ausgabe an den Benutzer zur Verfügung stellt.

Die Bewertungsinformation des zweiten Analysemoduls AM2 wird von dem Transaktionsmanager TM dem Empfehlungsmodul EM1 bereitgestellt. Das Empfehlungsmodul EM1 ermittelt anschließend, welches weitere Vorgehen am wahrscheinlichsten zur Lösung des Bildqualitätsproblems führt. Beispielsweise kann das Empfehlungsmodul EM1 einen regelbasierten Algorithmus umfassen, der anhand der Bewertungsinformation des zweiten Analysemoduls AM2 eine wahrscheinlichste Lösung ermittelt. Anhand dieser wahrscheinlichsten Lösung wird von dem Empfehlungsmodul EM1 ein Vorschlag und/oder eine Empfehlung an den Benutzer generiert. Ein Beispiel für einen Vorschlag und/oder eine Empfehlung kann sein: "Der letzte Messschritt sollte wiederholt werden." Dieser Vorschlag und/oder diese Empfehlung wird von dem Empfehlungsmodul EM1 als erweiterte Assistenzinformation bereitgestellt.

Die erweiterte Assistenzinformation wird anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt. Beispielsweise kann dem Benutzer folgende Information angezeigt werden: "Ein Bewegungsartefakt wurde detektiert. Möchten Sie den letzten Messschritt wiederholen?" Der Benutzer hat nun die Möglichkeit, die Empfehlung an der Benutzerschnittstelle BS manuell zu bestätigen oder abzulehnen. Bei einer Bestätigung der Empfehlung wird diese automatisch vom Assistenzsystem AS umgesetzt. Bei einer Ablehnung wird manuell mit dem Messprogramm MP fortgefahren.

In Fig. 6 ist für einen Messschritt MS1 eine Unterstützung mittels des Assistenzsystems AS ausgewählt. Dabei wurden zwei Analysemodule AM1, AM2 und zwei Empfehlungsmodule EM1, EM2 ausgewählt. Die die beiden Analysemodule AM1, AM2 ermitteln dabei unabhängig voneinander eine Bewertungsinformation, die als Eingangsdaten für die Empfehlungsmodule EM1, EM2 dienen.

In dem Messschritt MS1 werden Magnetresonanzdaten erfasst und daraus Bilddaten mit zumindest einem Bild rekonstruiert. Diese Bilddaten werden mittels des Transaktionsmanagers TM dem ersten Analysemodul AM1 und dem zweiten Analysemodul AM2 bereitgestellt. Das erste Analysemodul AM1 analysiert dabei die bereitgestellten Bilddaten hinsichtlich Bewegungsartefakten. Das zweite Analysemodul AM2 analysiert die bereitgestellten Bilddaten hinsichtlich einem Rauschverhalten der Bilddaten. Beide Analysemodule AM1, AM2 ermitteln dabei jeweils unabhängig voneinander eine Bewertungsinformation und stellen zudem eine Assistenzinformation zur Verfügung. Diese Assistenzinformation wird vom Transaktionsmanager TM dem Assistenz-Userinterface AUI bereitgestellt, wobei das Assistenz-Userinterface AUI die Assistenzinformation der Benutzerschnittstelle BS zur Ausgabe an den Benutzer zur Verfügung stellt.

Die Bewertungsinformationen werden von dem Transaktionsmanager TM den Empfehlungsmodulen EM1, EM2 bereitgestellt. Dabei wird die Bewertungsinformation des ersten Analysemoduls AM1 von dem Transaktionsmanager TM dem ersten Empfehlungsmoduls EM1 bereitgestellt. Das erste Empfehlungsmodul EM1 ermittelt hierbei, mit welchen Maßnahmen die von dem ersten Analysemodul AM1 erkannten Bewegungsartefakte in den Bilddaten reduzieren und/oder verhindern könnten. Beispielsweise kann eine Maßnahme sein, den Messschritt zur Erfassung der Bilddaten zu wiederholen. Hierzu wird von dem ersten Empfehlungsmodul EM1 ein entsprechender Vorschlag und/oder Empfehlung ermittelt und als erweiterte Assistenzinformation für eine Ausgabe an einen Benutzer bereitgestellt.

Die Bewertungsinformation des zweiten Analysemoduls AM2 wird von dem Transaktionsmanager TM dem zweiten Empfehlungsmodul EM2 bereitgestellt. Das zweite Empfehlungsmodul EM2 ermittelt hierbei, mit welchen Maßnahmen das von dem zweiten Analysemodul AM2 erkannte Bildrauschen unterdrückt und ein Signal-zu Rausch-Verhältnis verbessert werden kann. Beispielsweise kann eine Maßnahme sein, zumindest eine Mittelung (Average) oder auch mehrere Averages einzustellen. Je nach Art und/oder Intensität des erkannten Bildrauschens kann diese Maßnahme nur für die weiteren Messschritte MSi oder auch bei einem Wiederholen des ersten Messschritts MS1 empfohlen werden. Hierzu wird von dem ersten Empfehlungsmodul EM2 ein entsprechender Vorschlag und/oder Empfehlung ermittelt und als erweiterte Assistenzinformation für eine Ausgabe an einen Benutzer bereitgestellt.

Die erweiterte Assistenzinformation wird anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt. Der Benutzer hat nun die Möglichkeit, die Empfehlung an der Benutzerschnittstelle BS manuell zu bestätigen oder abzulehnen. Bei einer Bestätigung der Empfehlung wird diese automatisch vom Assistenzsystem AS umgesetzt. Bei einer Ablehnung wird manuell mit dem Messprogramm MP fortgefahren.

Das Verfahren aus Fig. 6 kann beispielsweise bei der Kopfbildgebung zur parallelen Überprüfung von Bildqualitätsproblemen unterschiedlicher Typen angewendet werden.

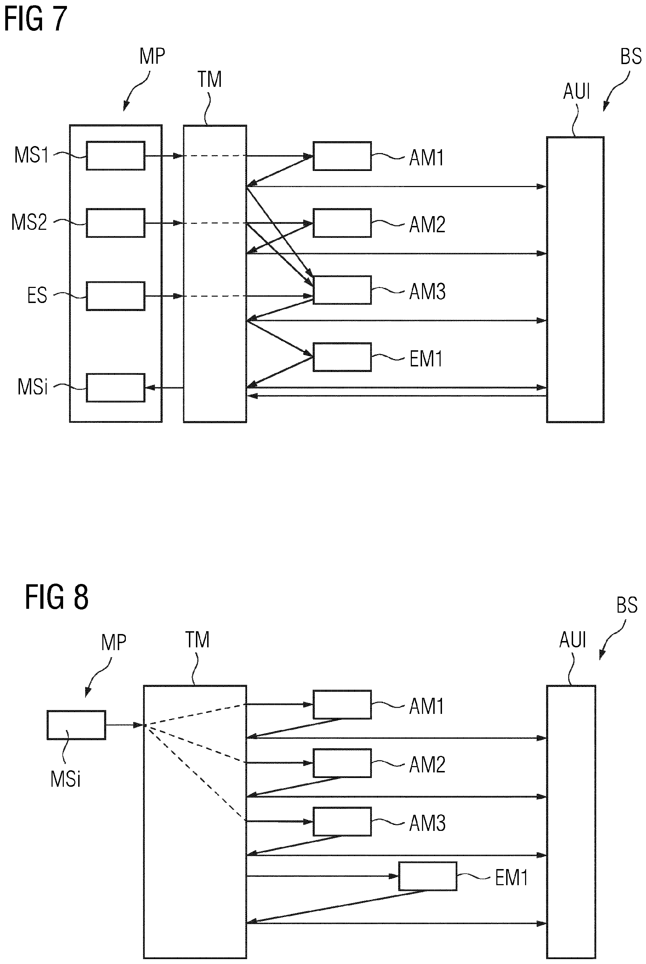

In Fig. 7 ist für mehrere Messschritt MS1, MS2 eine Unterstützung mittels des Assistenzsystems AS ausgewählt. Dabei wurden für den ersten Messschritt MS1 ein erstes Analysemodul AM1, für den zweiten Messschritt MS2 ein zweites Analysemodul AM2 ausgewählt. Ein dritter Schritt umfasst einen Entscheidungsschritt ES, in dem über den weiteren Verlauf des Messprogramms MP entschieden wird. Für diesen Entscheidungsschritt ES wurde ein drittes Analysemodul AM3 ausgewählt. Dabei kann es auch sein, dass nur für den Entscheidungsschritt ES das dritte Analysemodul AM3 von einem Benutzer ausgewählt wurde, und von dem Konfigurations-Userinterface KUI automatisch die beiden Analysemodule AM1, AM2 für den ersten und den zweiten Messschritt MS1, MS2 mit ausgewählt wurden. Für den Entscheidungsschritt ES wurde zudem ein Empfehlungsmodul EM1 ausgewählt. Dabei kann es auch sein, dass nur für den Entscheidungsschritt ES nur das Empfehlungsmodul EM1 von einem Benutzer ausgewählt wurde und von dem Konfigurations-Userinterface KUI automatisch die drei Analysemodule AM1, AM2, AM3 für die entsprechenden Messschritte MS1, MS2 mit ausgewählt wurden.

In Entscheidungsschritt ES wird entschieden, welche weiteren Messschritte MSi nach dem Entscheidungsschritt ES ausgeführt werden. Diese Entscheidung beruht auf den Ergebnissen aus den ersten beiden Messschritten MS1, MS2. Dabei kann der Benutzer auswählen, ob er nur eine Empfehlung angezeigt bekommen möchte, welche zusätzlichen Messschritte MSi zur Klärung von Auffälligkeiten oder bei Eintritt eines bestimmten Ereignisses sinnvoll wären, die Entscheidung und vor allem die Auswahl der weiteren Messschritte MSi jedoch beim Benutzer liegen. Alternativ kann der Benutzer auch eine automatisierte Unterstützung für diesen Entscheidungsschritt ES auswählen, bei dem Benutzer ein Messpfad bei Eintritt eines bestimmten Ereignisses empfohlen wird, wobei der Messpfad bereits vom Assistenzsystem vorbereitet und konfiguriert ist und bereit zu einer Ausführung ist.

Das Verfahren aus Fig. 7 kann beispielsweise bei der Kopfbildgebung angewendet werden. Hierbei kann eine parallele Überprüfung des Patienten auf Auffälligkeiten durchgeführt werden, wobei die erkannten oder detektierten Auffälligkeiten einer Entscheidung über den weiteren Verlauf des Messprogramms MP zugrunde gelegt werden.

In dem ersten Messschritt MS1 werden Magnetresonanzdaten erfasst und daraus erste Bilddaten mit zumindest ein Bild rekonstruiert. Diese ersten Bilddaten werden dem ersten Analysemodul AM1 mittels des Transaktionsmanagers TM bereitgestellt, wobei das erste Analysemodul AM1 die ersten Bilddaten hinsichtlich einer Mittellinienverlagerung des Gehirns analysiert. Das erste Analysemodul AM1 ermittelt dabei eine Bewertungsinformation und stellt zudem eine Assistenzinformation zur Verfügung, die mittels des Transaktionsmanagers TM dem Assistenz-Userinterface AUI zur Ausgabe an der Benutzerschnittstelle BS zur Verfügung gestellt wird. Die Assistenzinformation kann beispielsweise bei einer erkannten Mittellinienverlagerung die Information: "Mittellinienverlagerung des Gehirns detektiert." umfassen.

In dem zweiten Messschritt MS2 werden Magnetresonanzdaten erfasst und daraus zweite Bilddaten mit zumindest einem Bild rekonstruiert. Diese zweiten Bilddaten werden dem zweiten Analysemodul AM2 mittels des Transaktionsmanagers TM bereitgestellt, wobei das zweite Analysemodul AM2 die zweiten Bilddaten hinsichtlich einer Hyperintensität der weißen Gehirnsubstanz analysiert. Das zweite Analysemodul AM2 ermittelt dabei eine Bewertungsinformation und stellt zudem eine Assistenzinformation zur Verfügung, die mittels des Transaktionsmanagers TM dem Assistenz-Userinterface AUI zur Ausgabe an der Benutzerschnittstelle BS zur Verfügung gestellt wird. Die Assistenzinformation kann beispielsweise die Information: "Hyperintensität der weißen Gehirnsubstanz detektiert." umfassen.

In dem Entscheidungsschritt ES wird dem dritten Analysemodule AM3 mittels des Transaktionsmanagers TM mitgeteilt, dass evtl. eine Workflow-Anpassung und/oder eine Anpassung des Messprogramms an dieser Stelle im Messprogramm MP erfolgen soll. Hierzu analysiert das dritte Analysemodul AM3 sowohl die ersten Bilddaten als auch die zweiten Bilddaten, die mittels des Transaktionsmanagers TM bereitgestellt werden. Beispielsweise kann das dritte Analysemodul AM3 die ersten und zweiten Bilddaten hinsichtlich einer räumlich korrelierten Auffälligkeit analysieren und daraus eine Notwendigkeit einer MR-Perfusionsmessung zu ermitteln. Das dritte Analysemodul AM3 ermittelt dabei eine Bewertungsinformation und stellt zudem eine Assistenzinformation zur Verfügung, die mittels des Transaktionsmanagers TM dem Assistenz-Userinterface AUI zur Ausgabe an der Benutzerschnittstelle BS zur Verfügung gestellt wird. Die Assistenzinformation kann beispielsweise die Information: "Perfusionsmessung erforderlich" umfassen.

Die Bewertungsinformation des dritten Analysemoduls AM3 wird vom Transaktionsmanager TM dem Empfehlungsmodul EM1 bereitgestellt. Das Empfehlungsmodul EM1 ermittelt auf Basis der Bewertungsinformation des dritten Analysemoduls AM3 eine Empfehlung und/oder einen Vorschlag und stellt diesen als erweiterte Assistenzinformation für einen Benutzer bereit. Die erweiterte Assistenzinformation wird anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt.

Hat der Benutzer bei der Auswahl des Messprogramms MP für den Entscheidungsschritt ES nur eine Anzeige einer Empfehlung ausgewählt, wird dem Benutzer nun durch Anzeige der erweiterten Assistenzinformation über die Benutzerschnittstelle BS empfohlen, eine entsprechende Anpassung des Messprogramms MP, insbesondere der weiteren Messschritte MSi vorzunehmen. Im vorliegenden Ausführungsbeispiel ist die Empfehlung eine Perfusionsmessung als einen der nächsten Messschritte MSi in das Messprogramm aufzunehmen. Die Auswahl der einzelnen Messschritte MSi kann der Benutzer manuell durchführen oder auch einer automatischen Anpassung der Messschritte MSi durch das Assistenzsystem AS zustimmen.

Hat der Benutzer bei der Auswahl des Messprogramms MP für den Entscheidungsschritt ES die automatisierte Unterstützung ausgewählt, erhält er als Empfehlung und/oder als erweiterte Assistenzinformation über die Benutzerschnittstelle BS einen Vorschlag für einen Messpfad zur Fortführung des Messprogramms MP. Das Empfehlungsmodul EM1 ermittelt dabei einen Messpfad von mehreren zur Verfügung stehenden Messpfaden, die am besten zur Klärung der Auffälligkeiten und/oder der Ereignisse, die in den Analysemodulen AM1, AM2, AM3 detektiert und/oder analysiert wurden, führt. Im vorliegenden Ausführungsbeispiel enthält die Empfehlung einen Messpfad mit einem Perfusionsprotokoll und/oder einer Perfusionsmessung. Dieser Messpfad ist bereits vorkonfiguriert und steht zur Ausführung bereit und muss nur noch vom Benutzer über die Benutzerschnittstelle BS akzeptiert werden. Zudem steht dem Benutzer auch die Möglichkeit offen, den Vorschlag über die Benutzerschnittstelle BS abzulehnen und manuell mit dem Messprogramm MP fortzufahren.

In Fig. 8 ist für einen Messschritt MSi eine Unterstützung mittels des Assistenzsystems AS ausgewählt. Dabei wurden drei Analysemodule AM1, AM2, AM3 und ein Empfehlungsmodul EM1 ausgewählt. Die Analysemodule AM1, AM2, AM3 ermitteln dabei unabhängig voneinander eine Bewertungsinformation, die als Eingangsdaten für das Empfehlungsmodul EM1 dienen. Dieses Ausführungsbeispiel steht exemplarisch für mehrere Analysemodule AMi, welche unterschiedliche Daten analysieren, welche durch denselben Messschritt MSi erzeugt wurden. Ein Anwendungsbeispiel hierfür ist eine Herzbildgebungs-Qualitätssicherung, bei der drei verschiedene Analysemodule AM1, AM2, AM3 drei unterschiedliche Eingangsdaten, beispielsweise Bilddaten, EKG-Daten und eine Atem-Kurve des Patienten, analysieren. Jedes Analysemodul AM1, AM2, AM2 kann dabei signal-spezifische Probleme detektieren. Die Ergebnisse, insbesondere eine Bewertungsinformation, der Analysemodule AM1, AM2, AM3 werden in einem nachfolgenden Empfehlungsmodul EM1 konsolidiert, um eine finale Empfehlung an den Benutzer zu ermitteln.

In dem Messchritt MSi werden Magnetresonanzdaten und weitere Messinformationen erfasst, und die erfassten Magnetresonanzdaten und/oder daraus rekonstruierte Bilddaten sowie die weiteren Messinformationen mittels des Transaktionsmanagers TM den einzelnen Analysemodulen AM1, AM2, AM3 für eine Analyse zur Verfügung gestellt. Die bereitgestellten Messinformationen umfassen dabei EKG-Informationen und Atem-Informationen.

Das ersten Analysemodul AM1 analysiert dabei die bereitgestellten Bilddaten, die Bilddaten des Herzens des Patienten umfassen, hinsichtlich Qualitätsproblemen, insbesondere Bewegungsartefakte in den Bilddaten. Eine daraus ermittelte Bewertungsinformation kann beispielsweise einen Wert "0" oder "1" annehmen und eine entsprechende Assistenzinformation wird generiert. Dabei umfasst die Bewertungsinformation "0" beispielsweise die Assistenzinformation "keine Bewegungsartefakte erkannt" und "1" die Assistenzinformation "Bewegungsartefakte erkannt".

Das zweite Analysemodul AM2 analysiert die EKG-Informationen. Dabei können die EKG-Informationen sowohl EKG-Signale des Patienten als auch Daten einer Platzierung der EKG-Elektroden oder auch Daten einer EKG-Elektroden-Konnektivität umfassen. Das zweite Analysemodul AM2 kann dabei sowohl eine Arrhythmie in den EKG-Signalen erkennen als auch eine Platzierung und eine Konnektivität der Elektroden überprüfen. Daraus ermittelte Bewertungsinformationen können beispielsweise einen Wert "0" oder "1" annehmen und eine entsprechende Assistenzinformation wird generiert. Dabei umfasst die Bewertungsinformation "0" beispielsweise eine Assistenzinformation "keine Arrhythmie erkannt" und/oder "Elektroden korrekt positioniert" und/oder "Elektroden korrekt verbunden" und "1" die Assistenzinformation "Arrhythmie erkannt" und/oder "Elektroden falsch positioniert" und/oder "Elektroden falsch verbunden".

Das dritte Analysemodul AM3 analysiert die Atem-Informationen hinsichtlich eines Befolgens des Patienten von vorgegebenen Atemkommandos. Eine daraus ermittelte Bewertungsinformation kann beispielsweise einen Wert "0" oder "1" annehmen und eine entsprechende Assistenzinformation wird generiert. Dabei umfasst die Bewertungsinformation "0" beispielsweise die Assistenzinformation "Patient hat Atemkommandos korrekt befolgt" und "1" die Assistenzinformation "Patient hat Atemkommandos nicht befolgt".

Die einzelnen Assistenzinformationen werden mittels des Transaktionsmanagers TM dem Assistenz-Userinterface AUI zur Ausgabe an der Benutzerschnittstelle BS zur Verfügung gestellt.

Das Empfehlungsmodul EM1 erhält mittels des Transaktionsmanagers TM die bereitgestellten Bewertungsinformationen der einzelnen Analysemodule AM1, AM2, AM3. Das Empfehlungsmoduls EM1 analysiert dabei die Ergebnisse der drei Analysemodule AM1, AM2, AM3, ob insgesamt ein Qualitätsproblem vorliegt, und falls ein Qualitätsproblem vorliegt, was die wahrscheinlichste Ursache für das Problem ist und wie dieses am besten mitigiert werden kann. Wird beispielsweise mittels des zweiten Analysemoduls AM2 eine Arrhythmie erkannt, kann von dem Empfehlungsmodul EM1 der Vorschlag und/oder die Empfehlung ermittelt werden, den Messschritt MSi mit einem Protokoll, dass weniger sensitiv auf Arrhythmie reagiert, zu wiederholen, wie beispielsweise ein Compressed-Sensing-CINE-Protokoll. Diese Empfehlung und/oder der Vorschlag wird als erweiterte Assistenzinformation bereitgestellt. Die erweiterte Assistenzinformation wird anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt. Eine Beispielhafter Empfehlungstext wäre: "Im letzten Messchritt ,CINE-LAX` wurde eine Bewegung festgestellt. Die Ursache ist wahrscheinlich eine Arrhythmie des Patienten. Möchten Sie die Messung mit dem robusteren ,CS CINE LAX` Protokoll wiederholen?" Der Benutzer kann dann die Empfehlung bestätigen oder ablehnen und manuell fortfahren.

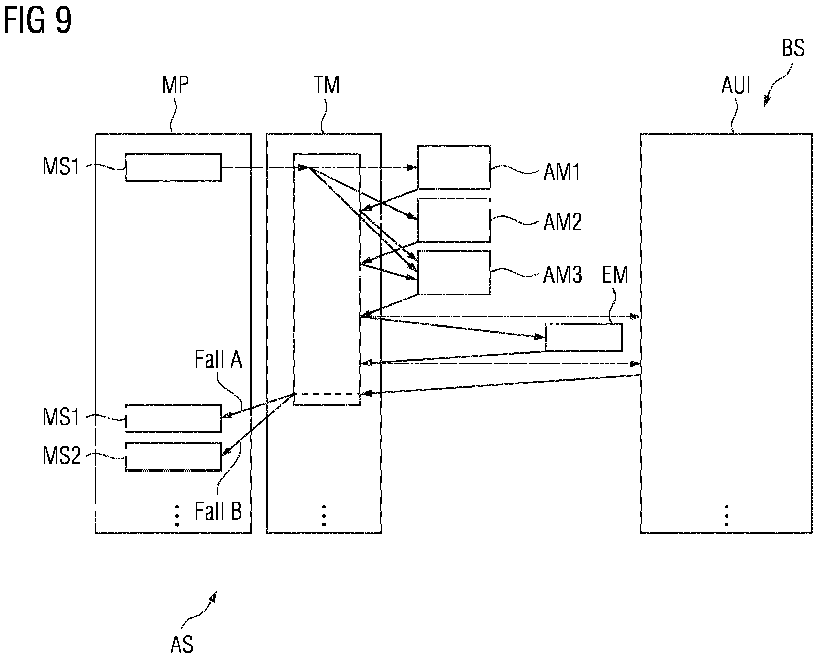

In Fig. 9 ist das Assistenzsystem AS in einem seriellen Ausführungsmodus dargestellt. Für einen ersten Messschritt MS1 sind mehrere Analysemodule AM1, AM2, AM3 und ein Empfehlungsmodul EM 1 ausgewählt. Ein Datenaustausch zwischen den einzelnen Analysemodulen AM1, AM2, AM3 und dem Empfehlungsmodul EM1 erfolgt mittels des Transaktionsmanagers TM. In diesem Ausführungsmodus wird der nächste Messschritt MS2 erst gestartet, wenn der erste Messschritt MS1 und damit auch eine Unterstützung mittels des Assistenzsystems abgeschlossen ist. Im vorliegenden Ausführungsbeispiel ist damit der erst Messschritt MS1 erst abgeschlossen, bis der von dem Empfehlungsmodul EM1 empfohlene Vorschlag ausgeführt wird oder vom Benutzer an der Benutzerschnittstelle BS abgelehnt wird.

In dem ersten Messschritt MS1 werden Magnetresonanzdaten erfasst und daraus Bilddaten mit zumindest einem Bild rekonstruiert. Diese Bilddaten werden mittels des Transaktionsmanagers TM dem drei Analysemodulen AM1, AM2, AM3 bereitgestellt. Das erste Analysemodul AM1 quantifiziert die Stärke der Bildunschärfe auf einem rekonstruierten Bild mittels eines Algorithmus A. Eine beispielhafte Bewertungsinformation wäre ein Skalar mit dem Wert "0,3" und stellt zudem eine Assistenzinformation bereit. Das zweite Analysemodul AM2 quantifiziert die Stärke der Bildunschärfe auf einem rekonstruierten Bild mittels eines Algorithmus B. Eine beispielhafte Bewertungsinformation wäre ein Skalar mit dem Wert "0,8" und stellt zudem eine Assistenzinformation bereit.

Die Bewertungsinformationen der beiden Analysemodule AM1, AM2 werden mittels des Transaktionsmanagers TM dem dritten Analysemodul AM3 bereitgestellt, das hierbei als sekundäres Analysemodul AM3 operiert und eine konsolidierende Bewertungsinformation erstellt. Ein Beispiel für eine konsolidierende Bewertungsinformation des dritten Analysemoduls AM3 wäre der Wert "0,5", die das dritte Analysemodul AM3 auf Basis der beiden Bewertungsinformationen der beiden ersten Analysemodulen AM1 und AM2 bestimmt. Als Assistenzinformation wird eine mittlere Qualitätsklasse bestimmt mit dem Output "Bildschärfe - Schlecht". Die einzelnen Assistenzinformationen werden mittels des Transaktionsmanagers TM dem Konfigurations-Userinterface KUI zur Ausgabe an der Benutzerschnittstelle BS zur Verfügung gestellt.

Das Empfehlungsmodul EM1 erhält mittels des Transaktionsmanagers TM die bereitgestellten Bewertungsinformation des dritten Analysemoduls AM3 und ermittelt daraus einen Vorschlag und/oder eine Empfehlung für den Benutzer. Dabei kann das Empfehlungsmodul EM1 einen regelbasierten Algorithmus umfassen. Eine beispielhafte Empfehlung im vorliegenden Ausführungsbeispiel wäre "Überprüfen Sie die Bildschärfe und ziehen Sie eine Erhöhung der Bildauflösung in Erwägung. Bildauflösung um 20% erhöhen?" Diese Empfehlung und/oder dieser Vorschlag kann dabei für den nachfolgenden Messschritt MS2 oder auch für eine Wiederholung des ersten Messchritts MS2 empfohlen werden. Diese Empfehlung und/oder dieser Vorschlag wird von dem Empfehlungsmodul EM1 als erweiterte Assistenzinformation bereitgestellt und anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt. Der Benutzer kann die Empfehlung annehmen (Fall A), wird die Empfehlung vom Assistenzsystem AS entsprechend umgesetzt. Falls der Benutzer die Empfehlung ablehnt (Fall B) erfolgt eine manuelle Fortführung des Messprogramms MP.

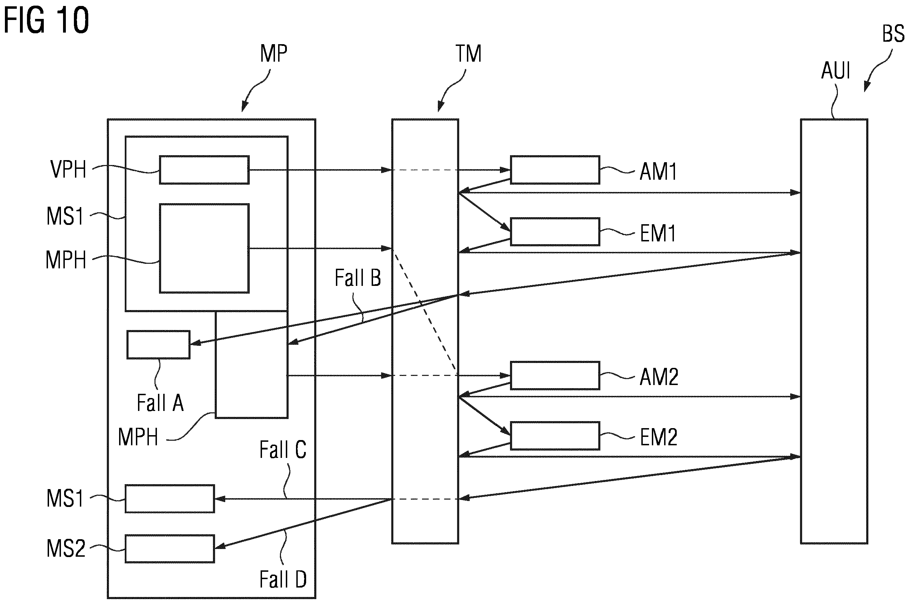

In Fig. 10 ist das Assistenzsystem AS in einem Quasi-Echtzeitmodus dargestellt. Hier umfasst ein erster Messschritt MS1 eine Vorbereitungsphase VPH und eine Messphase MPH. Eine beispielhafte Anwendung dieser Konfiguration ist die Überwachung von Physiosignalen des Patienten, die einen signifikanten Einfluss auf die diagnostische Qualität der akquirierten Magnetresonanzdaten haben können. Eine Analyse dieser Physiosignale kann in Teilsegmenten erfolgen, während der MR-Messchritt MS1 kontinuierlich weiterläuft. Die Vorbereitungsphase VPH ist beispielhaft zur Bestimmung einer Atemanhaltedauer des Patienten ausgebildet. Diese Daten werden an ein erstes Analysemodul AM1 weitergeleitet und dort analysiert. Während das erste Analysemodul AM1 die Atemdaten analysiert, startet bereit die Messphase MPH des ersten Messschritts MS1. Ein Datenaustausch zwischen den einzelnen Analysemodulen AM1, AM2 und den Empfehlungsmodulen EM1, EM2 erfolgt mittels des Transaktionsmanagers TM.

Das erste Analysemodul AM1 analysiert während der Vorbereitungsphase VPH die Atemkurve des Patienten, ob dieser die eingespielten Atemkommandos (beispielsweise "einatmen - ausatmen - einatmen und den Atem anhalten" ausführen kann. Eine daraus ermittelte Bewertungsinformation kann beispielsweise einen Wert "0" oder "1" annehmen und eine entsprechende Assistenzinformation wird generiert. Dabei umfasst die Bewertungsinformation "0" beispielsweise die Assistenzinformation "Der Patient hat die Vorbereitungsphase VPH erfolgreich befolgt" und "1" die Assistenzinformation "Der Patient hat die Vorbereitungsphase VPH nicht erfolgreich befolgt". Die Assistenzinformation wird mittels des Transaktionsmanagers TM dem Assistenz-Userinterface AUI zur Ausgabe an der Benutzerschnittstelle BS zur Verfügung gestellt.

Das erste Empfehlungsmodul EM1 ermittelt anhand der bereitgestellten Bewertungsinformation des ersten Analysemoduls AM1 eine Empfehlung und/oder einen Vorschlag. In dem Fall, dass der Patient den Atemkommandos folgen kann, wäre der Vorschlag und/oder die Empfehlung, mit dem Messprogramm MP einfach fortzufahren ohne Änderungen vorzunehmen. In dem Fall, dass der Patient den Atemkommandos nicht folgen kann, wäre eine mögliche Empfehlung des Empfehlungsmoduls EM1, den Messschritt MS1 abzubrechen und zu überprüfen, ob der Patient hört und/oder versteht etc. und danach die Messung zu wiederholen. Die Empfehlung und/oder der Vorschlag wird als erweiterte Assistenzinformation mittels des Transaktionsmanagers TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt. Dabei kann die Empfehlung auch schrittweise angezeigt werden jeweils eine Bestätigungseingabe oder eine Abbrucheingabe vom Benutzern eingefordert werden.

Lehnt der Benutzer die Empfehlung ab (Fall A) wird das Messprogramm MP manuell durch den Benutzer fortgesetzt (hier nicht näher dargestellt). Nimmt der Benutzer den Vorschlag an (Fall B), erfolgt zudem eine Analyse durch ein zweites Analysemodul AM2. Das zweite Analysemodul AM2 analysiert dabei die Atemanhaltephase, insbesondere die Atemkurve und die Atemanhaltezeit, des Patienten während der Messphase MPH. Hat der Patient die Atemanhaltephase erfolgreich durchgeführt, wird die Bewertungsinformation "0" ermittelt und bereitgestellt. Hat dagegen der Patient die Atemanhaltephase nicht erfolgreich durchgeführt, wird die Bewertungsinformation "1" ermittelt und bereitgestellt. Zudem kann die Bewertungsinformation auch eine Information umfassen, wie lange der Patient es schafft, den Atem anzuhalten.

Ein zweites Empfehlungsmodul EM2 ermittelt basierend auf den Bewertungsinformationen des Analysemoduls AM2 eine Empfehlung und/oder einen Vorschlag. In dem Fall, dass der Patient den Atemkommandos folgen kann, würde das zweite Empfehlungsmodul EM2 keinen neunen Vorschlag und/oder Empfehlung ermitteln. In dem Fall, dass der Patient den Atemkommandos nicht folgen kann, wäre eine mögliche Empfehlung des zweiten Empfehlungsmoduls EM2, die Atemanhaltedauer für den Patienten zu reduzieren, beispielsweise um 4 s zu reduzieren. Danach sollte der Messschritt MS1 neu gestartet werden. Diese Empfehlung und/oder dieser Vorschlag wird von dem zweiten Empfehlungsmodul EM2 als erweiterte Assistenzinformation bereitgestellt und anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt. Der Benutzer kann die Empfehlung annehmen (Fall C) und Messschritt MS1 wiederholen oder ablehnen (Fall D) und mit Messchritt MS2 weitermachen.

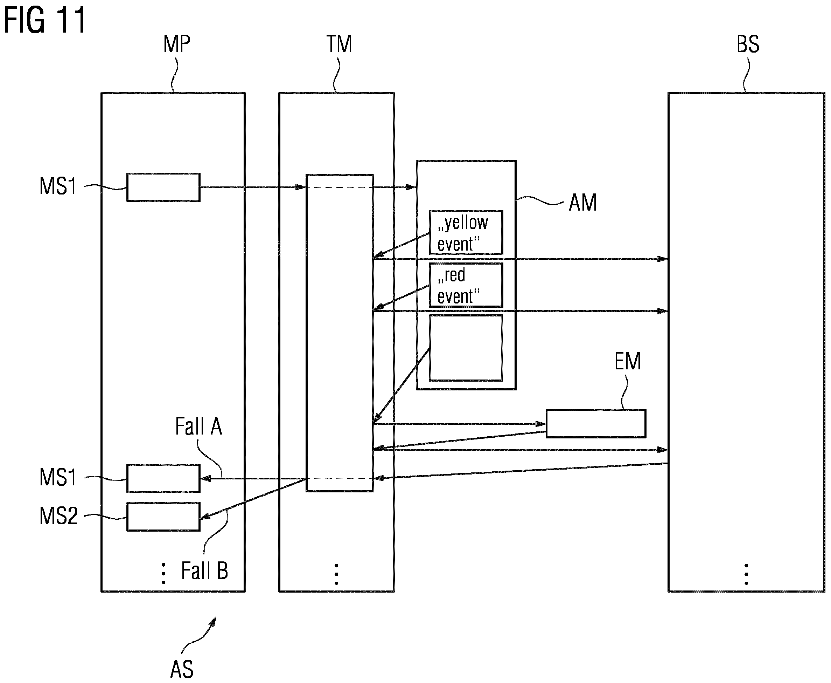

In Fig. 11 ist das Assistenzsystem AS in einem Echtzeitmodus dargestellt, bei dem eine Analyse von Messdaten durchgeführt wird, während die Messung läuft. Für bestimmte Qualitätsanalysen kann es von Vorteil sein ein Feedback so schnell wie möglich dem Benutzer zur Verfügung zu stellen. Das Analysemodul AM1 analysiert permanent die eingehenden Daten und ermittelt fortlaufend eine aktuelle Bewertungsinformation und Assistenzinformation, um den Benutzer fortlaufend ein Feedback zur aktuellen Messung zu geben. Ein Datenaustausch zwischen dem Analysemodul AM1 und dem Empfehlungsmodul EM1 erfolgt mittels des Transaktionsmanagers TM.

Das Analysemodul AM1 führt dabei in Echtzeit, d.h. unmittelbar, während die Daten erzeugt werden und die Messung, insbesondere der erste Messschritt MS1, weiterläuft, eine Analyse der Physiodaten aus. Hierbei können beispielsweise in Echtzeit Bewegungsartefakte in den Bilddaten detektiert und/oder erkannt werden. Dabei können über die Messung hinweg verschiedene und möglichweise transiente Kritikalitätslevel erreicht werden. Dabei ermittelt das Analysemodul AM1 fortlaufend beispielsweise die Messqualität und damit fortlaufend eine Bewertungsinformation und eine Assistenzinformation. Beispielsweise kann die Bewertungsinformation direkt nach dem Start der Messung "Gut" sein und mit einem grünen Anzeigesymbol als Assistenzinformation korreliert sein. Sobald das Analysemodul AM1 eine Änderung erfasst, beispielsweise eine Bewegung, wird eine geänderte Bewertungsinformation generiert, beispielsweise "Unsicher" und diese mit einem gelben Anzeigesymbol als Assistenzinformation korreliert sein. Die Änderung der Messqualität kann auch zu einer geänderten Bewertungsinformation mit dem Wert "Schlecht" führen und diese mit einem roten Anzeigesymbol als Assistenzinformation korreliert sein. Sobald die Messqualität wieder besser wird, wird auch die entsprechende Bewertungsinformation in "Unsicher" oder "Gut" geändert und auch die Assistenzinformation umfasst ein gelbes Anzeigesymbol oder ein grünes Anzeigesymbol. Die Bewertungsinformation wird fortlaufend in Echtzeit mittels des Transaktionsmanagers TM dem Empfehlungsmodul EM1 zur Verfügung gestellt. Die Assistenzinformation wird mittels des Transaktionsmanagers TM dem Assistenz-Userinterface AUI zur Ausgabe an der Benutzerschnittstelle BS zur Verfügung gestellt.

Am Ende der Analyse gibt das Analysemodul AM1 ein finales Resultat aus. Dabei kann es ausschlaggebend sein, wie stark die Messqualität Schwankungen unterliegt. Ist die meiste Zeit die Messqualität "Gut" und nur kurzzeitig "Unsicher" oder "Schlecht", kann das finale Resultat eine Bewertungsinformation mit dem Wert "0" und "Gut" umfassen. Ist dagegen die Messqualität die meiste Zeit "Schlecht" und bleibt auf diesem Level, wird von dem Analysemodul AM1 als finales Resultat eine Bewertungsinformation mit dem Wert "1" und "Schlecht" umfassen.

Das Empfehlungsmodul EM1 ermittelt basierend auf der Bewertungsinformation des Analysemoduls AM1 eine Empfehlung an den Benutzer. Weist dabei beispielsweise die Bewertungsinformation den Wert "0" auf, würde nur ein grünes Anzeigesymbol für die Assistenzinformation angezeigt werden, da kein Handlungsbedarf zur Korrektur vorhanden ist. Weist dagegen die Bewertungsinformation den Wert "1" auf, wird von dem Empfehlungsmodul einen Abbruch und ein Wiederholen des Messschritts MS1 vorgeschlagen. Eine entsprechende erweiterte Assistenzinformation könnte lauten: "Die Messung sollte wegen Bewegungsartefakten abgebrochen werden. Möchten Sie die Messung neu starten?". Diese Empfehlung und/oder dieser Vorschlag wird von dem Empfehlungsmodul EM als erweiterte Assistenzinformation bereitgestellt und anschließend von dem Transaktionsmanager TM dem Assistenz-Userinterface AUI bereitgestellt, wobei das Assistenz-Userinterface AUI die erweiterte Assistenzinformation der Benutzerschnittstelle BS zur Ausgabe an den Benutzer zur Verfügung stellt und dem Benutzer über die Benutzerschnittstelle BS anzeigt.

Der Benutzer kann die Empfehlung des Empfehlungsmoduls EM1 akzeptieren (Fall A) und der erste Messschritt MS1 wird wiederholt oder den Vorschlag ablehnen (Fall B) und manuell mit dem zweiten Messschritt MS2 weiter fortfahren.

In Fig. 12 ist die Klassifikation der Ausgangsdaten der Analysemodule AM und der Empfangsmodule EM tabellarisch dargestellt. Hierbei sind in der ersten Spalte 110 die zur Verfügung stehenden Symbole für Qualitäts-Module, in der zweiten Spalte 111 die zur Verfügung stehenden Symbole für klinischen Module und in der dritten Spalte 112 die zur Verfügung stehenden Symbole für technische Module dargestellt.

Eine erste Zeile 120 zeigt an, dass ein Modul, insbesondere ein Analysemodul AM und/oder ein Empfehlungsmodul EM, ausgewählt wurde. In einer zweiten Zeile 121 sind die Symbole dargestellt, wenn das entsprechende Modul, insbesondere ein Analysemodul AM und/oder ein Empfehlungsmodul EM, gerade eine Analyse durchführt. Die Symbole in den Zeilen 120 und 121 stehen dabei für Ausgangsdaten aller Klassen zur Verfügung.

In Zeile 122, 123 und 124 sind die Symbole dargestellt, wenn das entsprechende Modul, insbesondere ein Analysemodul AM und/oder ein Empfehlungsmodul EM, die Analyse erfolgreich beendet hat und kein Problem und/oder keine Auffälligkeit erkannt wurde. Zeile 122 ist hierbei für Analysemodule AM und/oder Empfehlungsmodule EM, die Ausgansdaten der Klasse "Traffic-light Classifier" bereitstellen. Für diese Klasse liegt kein Problem und/oder kein kritischer Zustand und/oder keine Auffälligkeit vor und es ist keine weitere Aktion durch den Benutzer erforderlich. Zeile 123 ist für ein Analysemodul AM und Zeile 124 für eine Empfehlungsmodul EM, die Ausgangsdaten der Klassen "Multi-Class Classifier" und "Multi-Class Multi-Label Classifier" bereitstellen. Die Analyse wurde erfolgreich beendet mit einer ausreichenden Sicherheit und es ist keine Aktion erforderlich, jedoch kann eine Empfehlung und/oder ein Vorschlag vorliegen.

In Zeilen 125 und 126 sind die Symbole dargestellt, wenn das entsprechende Modul, insbesondere ein Analysemodul AM und/oder ein Empfehlungsmodul EM, die Analyse erfolgreich beendet hat, das Ergebnis jedoch unsicher ist. Zeile 125 ist für ein Analysemodul AM sowohl der Klasse "Traffic-light Classifier" als auch der Klassen "Multi-Class Classifier" und "Multi-Class Multi-Label Classifier". Eine Aktion durch den Benutzer, beispielsweise ein Ausführen eines Vorschlags, wird von den Analysemodulen AM empfohlen. Zeile 126 ist für ein Empfehlungsmodul EM sowohl der Klasse "Traffic-light Classifier" als auch der Klassen "Multi-Class Classifier" und "Multi-Class Multi-Label Classifier". Die Empfehlungsmodule EM stellen einen Vorschlag für den Benutzer bereit.

In Zeile 127 und 128 sind die Symbole dargestellt, wenn das entsprechende Modul, insbesondere ein Analysemodul AM und/oder ein Empfehlungsmodul EM, die Analyse erfolgreich beendet hat und ein Problem und/oder kritischer Zustand und/oder eine Auffälligkeit erkannt wurde. Zeile 127 ist hierbei für Analysemodule AM der Klasse "Traffic-light Classifier". Eine Aktion durch den Benutzer, beispielsweise ein Ausführen eines Vorschlags, wird von den Analysemodulen AM empfohlen. Zeile 128 ist für ein Empfehlungsmodul EM der Klasse "Traffic-light Classifier". Das Empfehlungsmodule EM stellt einen Vorschlag für den Benutzer bereit.

In Zeile 129 sind die Symbole dargestellt, wenn das entsprechende Modul, insbesondere ein Analysemodul AM und/oder ein Empfehlungsmodul EM, die Analyse nicht beenden konnte. Es liegt also kein Ergebnis vor. Die Symbole sind für Module, insbesondere Analysemodule AM und/oder Empfehlungsmodule EM, sowohl der Klasse "Traffic-light Classifier" als auch der Klassen "Multi-Class Classifier" und "Multi-Class Multi-Label Classifier" vorgesehen.

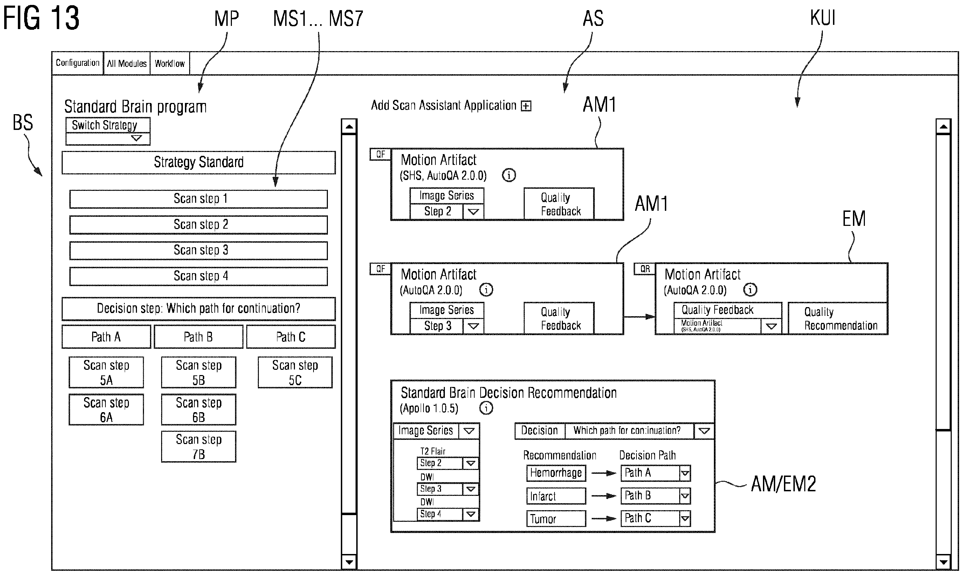

In den Fig. 13 und 14 ist jeweils eine Benutzeroberfläche BO zur Kommunikation des Assistenzsystems AS mit einem Benutzer dargestellt.

In Fig. 13 ist die Benutzeroberfläche BO in einem Auswahlmodus des Konfigurations-Userinterfaces KUI dargestellt. In diesem Auswahlmodus kann der Benutzer neben der Auswahl eines Messprogramms MP für einzelnen Messschritte MS1 bis MS7 eine Unterstützung mittels des Assistenzsystems AS auswählen. Dabei kann der Benutzer eine Art des Analysemoduls AM auswählen. Zudem kann der Benutzer auch ein damit verbundenes Empfehlungsmodul EM auswählen. Im vorliegendem Ausführungsbeispiel umfasst das Messprogramm MP eine Kopfuntersuchung. Die einzelnen Messschritte MS1 bis MS7 des Messprogramms sind nacheinander fortlaufend auf der linken Seite der Benutzeroberfläche BO dargestellt. Die Auswahl der einzelnen Module des Assistenzsystems AS erfolgt über die rechte Hälfte der Benutzeroberfläche BO. Dabei ist für den Messschritt MS2 ein erstes Analysemodul AM1 zur Erkennung von Bewegungsartefakten ausgewählt. Für Messschritt 4 ist zudem nochmals das erste Analysemodul AM1 zur Erkennung von Bewegungsartefakten ausgewählt, diesmal aber zusammen mit einem ersten Empfehlungsmodul EM, das zu einem Ermitteln eines Vorschlags ausgebildet ist.

Zudem kann auch eine Entscheidungsunterstützung mittels des Assistenzsystems AS von dem Benutzer ausgewählt werden. Dabei kann festgelegt werden, welcher Messpfad ausgewählt werden soll, wenn ein bestimmtes Ereignis eintritt. Im vorliegenden Ausführungsbeispiel umfasst das Ereignis einen Verdacht auf eine bestimmte Erkrankung des Patienten. Wird mittels des Assistenzsystems AS eine Entscheidungsunterstützung ausgewählt, kann von einem Benutzer festgelegt werden, welcher Messpfad bei Eintritt eines bestimmten Ereignisses ausgewählt werden soll. Das Assistenzsystem AS unterstütz den Benutzer dabei, den Eintritt des bestimmten Ereignisses zu erkennen und/oder zu ermitteln. Im vorliegenden Ausführungsbeispiel stehen hierbei ein zweites Analysemodul AM2 und ein zweites Empfehlungsmodul EM2 zur Verfügung, wobei der Benutzer aus auswählen kann, welcher Messpfad als Vorschlag ausgegeben wird, wenn ein bestimmtes Ereignis eintritt. Wird von dem zweiten Analysemodul AM2 ein Verdacht auf eine Hirnblutung ermittelt und/oder bestimmt, wird von einem zweiten Empfehlungsmodul EM2 der Messpfad 1 als Vorschlag ausgewählt und dem Benutzer angezeigt. Wird dagegen von dem zweiten Analysemodul AM2 ein Verdacht auf einen Infarkt ermittelt und/oder bestimmt, wird von dem zweiten Empfehlungsmodul EM2 der Messpfad 2 als Vorschlag ausgewählt und dem Benutzer angezeigt. Wird zudem von dem zweiten Analysemodul AM2 ein Verdacht auf einen Tumor ermittelt und/oder bestimmt, wird von dem zweiten Empfehlungsmodul EM2 der Messpfad 3 als Vorschlag ausgewählt und dem Benutzer angezeigt.

In Fig. 14 ist die Benutzeroberfläche BO in einem Empfehlungsmodus des Assistenz-Userinterfaces AUI für ein Messprogramm MP dargestellt. Das Messprogramm umfasst mehrere Messschritte MS1 bis MS6, wobei die einzelnen Messschritte MS1 bis MS6 des Messprogramms nacheinander fortlaufend auf der linken Seite der Benutzeroberfläche BO dargestellt sind. Eine Ausgabe einer Assistenzinformation und/oder eines Vorschlags und/oder einer Statusinformation des ausgewählten Empfehlungsmoduls EM erfolgt auf der rechten Hälfte der Benutzeroberfläche BO.

In einem ersten Anzeigefeld AF1 wird der Messschritt MS angezeigt, für den eine Unterstützung mittels des Assistenzsystems AS vorliegt. Darunter befindet sich ein zweites Anzeigefeld AF2, das zunächst durch ein Symbol einen Status des ausgewählten Empfehlungsmoduls EM anzeigt. Daneben befindet sich ein Name des ausgewählten Empfehlungsmoduls EM. Darunter ist ein erstes Ansichtsfenster ANF1 angeordnet, das eine Statusinformation des Empfehlungsmoduls EM aufweist und zudem den Vorschlag dem Empfehlungsmoduls EM kurz wiedergibt. Bei einem Vergrößern des ersten Ansichtsfensters ANF1, das der Benutzer manuell anklicken muss, kann der Benutzer zusätzliche Informationen des Empfehlungsmoduls EM erhalten. Darunter befinden sich eingerückt zwei weitere Ansichtsfenster ANF2, ANF3, die jeweils ein Analysemodul AM1, AM2 kennzeichnen und dabei auch eine Statusinformation des jeweiligen Analysemoduls AM1, AM2 umfassen. Auch diese beiden Ansichtsfenster ANF2, ANF3 können durch manuelles Anklicken vergrößert werden, so dass zusätzlich eine Assistenzinformation, insbesondere eine von dem jeweiligen Analysemodul AM1, AM2 ermittelte Probleminformation und/oder Auffälligkeitsinformation für den Benutzer sichtbar wird. Unten rechts im zweiten Ansichtsfeld AF2 befindet sich ein Bestätigungsbutton BB, den der Benutzer betätigen und/oder anklicken kann, wenn er möchte, dass das Assistenzsystem AS den von dem Empfehlungsmodul EM vorgeschlagenen Vorschlag automatisch ausführt.

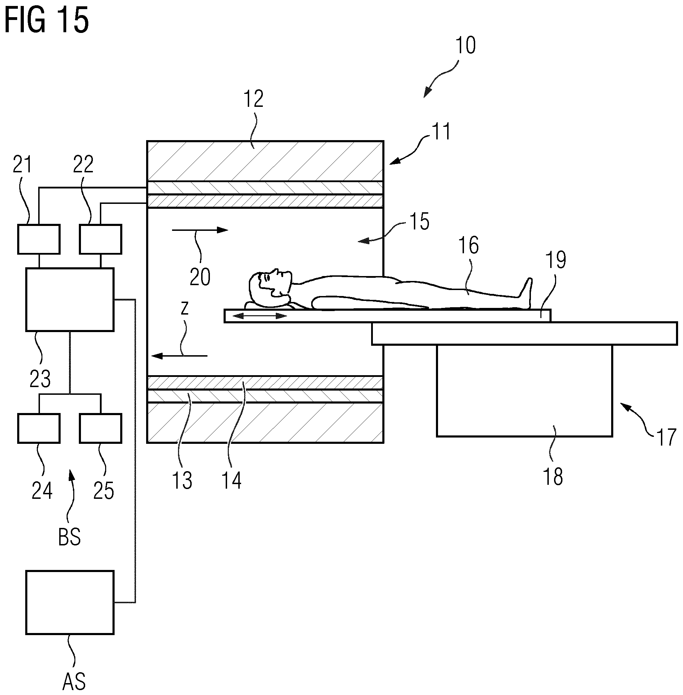

In Fig. 15 ist eine Magnetresonanzvorrichtung 10 schematisch dargestellt. Die Magnetresonanzvorrichtung 10 umfasst eine Magneteinheit 11 mit einem Grundmagneten 12, einer Gradientenspuleneinheit 13 und einer Hochfrequenzantenneneinheit 14. Zudem weist die Magnetresonanzvorrichtung 10 einen Patientenaufnahmebereich 15 auf zu einer Aufnahme eines Patienten 16 für eine Magnetresonanzuntersuchung. Der Patientenaufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 15 jederzeit denkbar.

Für eine Positionierung des Patienten 16, insbesondere eines zu untersuchenden Bereichs des Patienten 16, innerhalb des Patientenaufnahmebereichs 15 weist die Magnetresonanzvorrichtung 11 eine Patientenlagerungsvorrichtung 17 auf. Die Patientenlagerungsvorrichtung 17 weist eine Basiseinheit 18 und einen bezüglich der Basiseinheit 18 bewegbaren Patiententisch 19 auf. Der Patiententisch 19 ist für eine Positionierung Patienten 16, insbesondere des zu untersuchenden Bereichs des Patienten 16, bewegbar innerhalb des Patientenaufnahmebereichs 15 ausgebildet. Insbesondere ist hierbei der Patiententisch 19 in Richtung einer Längserstreckung des Patientenaufnahmebereichs 15 und/oder in z-Richtung bewegbar gelagert.

Der Grundmagnet 12 der Magneteinheit 11 ist zu einem Erzeugen eines starken und insbesondere konstanten Grundmagnetfelds 20 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 21 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 20 einstellt, ausgebildet. Die Hochfrequenzantenneneinheit 14 wird von einer Hochfrequenzantennensteuereinheit 22 der Magnetresonanzvorrichtung 10 gesteuert und strahlt hochfrequente Magnetresonanzsequenzen in den Patientenaufnahmebereich 15 der Magnetresonanzvorrichtung 10 ein.

Zu einer Steuerung des Grundmagneten 12, der Gradientensteuereinheit 13 und zur Steuerung der Hochfrequenzantennensteuereinheit 14 weist die Magnetresonanzvorrichtung 10 eine Systemsteuereinheit 23 auf. Die Systemsteuereinheit 23 steuert zentral die Magnetresonanzvorrichtung 10, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle BS, die mit der Systemsteuereinheit 23 verbunden ist. Steuerinformationen wie beispielsweise Bildgebungsparameter, sowie rekonstruierte Magnetresonanzbilder können auf einer Darstellungseinheit 24, beispielsweise auf zumindest einem Monitor, der Benutzerschnittstelle BS für ein medizinisches Bedienpersonal angezeigt werden. Weiterhin weist die Benutzerschnittstelle BS eine Eingabeeinheit 25 auf, mittels der Informationen und/oder Parameter während eines Messvorgangs von einem medizinischen Bedienpersonal eingegeben werden können.

Zu einer Ausführung des Verfahrens zu einem Unterstützen und/oder Assistieren eines Benutzers mittels des Assistenzsystems AS bei einem Ausführen eines Messprogramms MP während einer Magnetresonanzdatenerfassung umfasst die Magnetresonanzvorrichtung das Assistenzsystem AS. Das Assistenzsystem AS ist dabei entsprechend der Beschreibung zu Fig. 2 oder zu Fig. 4 ausgebildet. Das Assistenzsystem AS ist im vorliegenden Ausführungsbeispiel getrennt zur Systemsteuereinheit 23 ausgebildet, jedoch mit dieser zu einem Datenaustausch verbunden. In einer alternativen Ausbildung des Assistenzsystems AS kann dieses auch in der Systemsteuereinheit 23 integriert angeordnet sein.

Die dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Computerimplementiertes Verfahren zu einem Unterstützen und/oder Assistieren eines Benutzers mittels eines Assistenzsystems bei einem Ausführen eines Messprogramms während einer Magnetresonanzdatenerfassung, umfassend die Verfahrensschritte:
- Auswählen eines Messprogramms zu einem Erfassen von Magnetresonanzdaten mit einer definierten diagnostischen Fragestellung,
- Ausführen des ausgewählten Messprogramms, wobei das Ausführen des Messprogramms ein Erfassen von Magnetresonanzdaten und/oder ein Rekonstruieren von Bilddaten aus den erfassten Magnetresonanzdaten umfasst, und Bereitstellen der Magnetresonanzdaten und/oder der rekonstruierten Bilddaten,
- Erfassen weiterer Messinformationen, wobei die weiteren Messinformationen vor einem Ausführen des ausgewählten Messprogramms oder während eines Ausführens des ausgewählten Messprogramms erfasst werden, und Bereitstellen der weiteren Messinformationen,
- Ermitteln zumindest einer Bewertungsinformation mittels zumindest eines Analysemoduls des Assistenzsystems in Abhängigkeit von den bereitgestellten Magnetresonanzdaten und/oder den bereitgestellten rekonstruierten Bilddaten und/oder den bereitgestellten weiteren Messinformationen, und Bereitstellen der zumindest einen Bewertungsinformation,
- Generieren einer Assistenzinformation, wobei die Assistenzinformation in Abhängigkeit von der zumindest einen Bewertungsinformation mittels des zumindest einen Analysemoduls generiert wird, und Bereitstellen der Assistenzinformation und
- Ausgabe der Assistenzinformation über eine Benutzerschnittstelle an einen Benutzer.

2. Verfahren nach Anspruch 1,
**dadurch gekennzeichnet, dass** die Assistenzinformation eine Statusinformation des Analysemoduls umfasst.

3. Verfahren nach einem der vorhergehenden Ansprüche, **dadurch gekennzeichnet, dass** das zumindest eine Analysemodul zumindest ein Qualitäts-Analysemodul und/oder zumindest ein klinisches Analysemodul und/oder zumindest ein technisches Analysemodul und/oder zumindest ein allgemeines Analysemodul umfasst.

4. Verfahren nach einem der vorhergehenden Ansprüche, **dadurch gekennzeichnet, dass** das zumindest eine Analysemodul die zumindest eine Bewertungsinformation mittels eines regelbasierten Algorithmus und/oder eines auf maschinellem Lernen basierten Algorithmus ermittelt.

5. Verfahren nach einem der vorhergehenden Ansprüche, **dadurch gekennzeichnet, dass** anhand der zumindest einen bereitgestellten Bewertungsinformation des zumindest einen Analysemoduls zumindest ein Vorschlag für zumindest einen Messschritt des Messprogramms mittels eines Empfehlungsmoduls des Assistenzsystems ermittelt wird und der zumindest eine Vorschlag für den zumindest einen Messschritt bereitgestellt wird.

6. Verfahren nach Anspruch 5,
**dadurch gekennzeichnet, dass** das Assistenzsystem zu einer Ausführung des zumindest einen Vorschlags für den zumindest einen Messschritt ausgebildet ist.

7. Verfahren nach einem der vorhergehenden Ansprüche, **dadurch gekennzeichnet, dass** das Messprogramm mehrere Messchritte umfasst und für zumindest einen der mehreren Messschritte eine Unterstützung mittels des Assistenzsystems für einen Benutzer auswählbar ist.

8. Verfahren nach einem der vorhergehenden Ansprüche, **dadurch gekennzeichnet, dass** das Messprogramm mehrere Messschritte umfasst und die mehreren Messschritte des Messprogramms nacheinander ausgeführt werden, wobei für zumindest einen Messschritt eine Unterstützung mittels des Assistenzsystems ausgewählt wurde, wobei ein dem zumindest einem Messschritt, für den die Unterstützung ausgewählt wurde, nachfolgender Messschritt erst gestartet wird, wenn bei dem zumindest einen Messschritt, für den die Unterstützung ausgewählt wurde, die Unterstützung mittels des Assistenzsystems abgeschlossen ist.

9. Verfahren nach einem der vorhergehenden Ansprüche, **dadurch gekennzeichnet, dass** das Messprogramm mehrere Messschritte umfasst und für jeden Messschritt des Messprogramms, für den eine Unterstützung mittels des Assistenzsystems ausgewählt wurde, die Unterstützung mittels des Assistenzsystems in einem Quasi-Echtzeitmodus oder in einem Echtzeitmodus durchgeführt wird.

10. Verfahren nach einem der vorhergehenden Ansprüche, **dadurch gekennzeichnet, dass** ein Austausch und/oder das Bereitstellen der Magnetresonanzdaten und/oder der Bilddaten und/oder der weiteren Messinformationen und/oder der zumindest einen Bewertungsinformation und/oder der zumindest einen Assistenzinformation und/oder zumindest einer erweiterte Assistenzinformation eines Empfehlungsmoduls mittels eines Transaktionsmanagers des Assistenzsystems erfolgt.

11. Verfahren nach einem der vorhergehenden Ansprüche, **dadurch gekennzeichnet, dass** das Assistenzsystem ein Konfigurations-Userinterface umfasst, wobei das Konfigurations-Userinterface dazu ausgebildet ist, eine Auswahl zumindest eines Analysemoduls und/oder zumindest eines Empfehlungsmoduls für zumindest einen Messschritt zu konfigurieren.

12. Verfahren nach einem der vorhergehenden Ansprüche, **dadurch gekennzeichnet, dass** das zumindest ein Analysemodul und/oder das zumindest eine Empfehlungsmodul Ausgangsdaten generiert, wobei die Ausgangsdaten ein definiertes Ausgangsdatenformat umfasst.

13. Assistenzsystem, das zu einer Durchführung des Verfahrens zu einem Unterstützen und/oder Assistieren eines Benutzers bei einem Ausführen eines Messprogramms während einer Magnetresonanzdatenerfassung nach einem der vorhergehenden Ansprüche ausgebildet ist, wobei das Assistenzsystem einen Transaktionsmanager und zumindest ein Analysemodul umfasst.

14. Magnetresonanzvorrichtung mit einem Assistenzsystem nach Anspruch 13.

15. Computerprogrammprodukt, welches ein Programm umfasst und direkt in einen Speicher einer programmierbaren Steuerungseinheit ladbar ist, mit Programmmitteln, um ein Verfahren zu einem Unterstützen und/oder Assistieren eines Benutzers mittels eines Assistenzsystems bei einem Ausführen eines Messprogramms zur einer Magnetresonanzdatenerfassung nach einem der Ansprüche 1 bis 12 auszuführen, wenn das Programm in der Steuerungseinheit ausgeführt wird.
